# EUROPEAN PATENT APPLICATION

(11) **EP 1 541 694 A1**
(43) Date of publication of application: **15.06.2005**
(21) Application number: 03028713.0
(22) Date of filing: 12.12.2003
(51) Int. Cl.: C12Q 1/48, G01N 33/50

(54) **Methods of identifying, selecting and/or characterizing compounds which modulate the activity of a Src family kinase**

(71) Applicant: Sirenade Pharmaceuticals AG, 82152 Martinsried (DE)
(72) Inventor: Obermeier, Axel, 81379 München (DE); Bieger, Boris, 81379 München (DE)
(74) Representative: Graf von Stosch, Andreas, Dr. rer. nat.

(57) **Abstract**

The invention relates to a method of identifying, selecting and/or characterizing a compound which modulates the activity of at least one Src family kinase. It further relates to compounds identified by said method, pharmaceutical compositions and the use of those compounds and pharmaceutical compositions in the treatment of diseases, which are at least in part caused by a Src family kinase.

SEQUENCE LISTING

<110> SiREEN AG

<120> Methods of identifying, selecting and/or characterizing
      compounds which modulate the activity of a Src family
      kinase

<130> SI07P001EP

<140> 03028713.0
<141> 2003-12-12

<160> 15

<170> PatentIn Ver. 2.1

<210> 1
<211> 536
<212> PRT
<213> Homo sapiens

<220>
<223> Src kinase (Fig. 18)

<220>
<221> SITE
<222> (298)
<223> Constant amino acid K in domain SH2

<220>
<221> SITE
<222> (341)
<223> Constant amino acid T in domain SH2

<220>
<221> SITE
<222> (530)
<223> Constant amino acid Y in domain SH1

<400> 1


<210> 2
<211> 543
<212> PRT
<213> Homo sapiens

<220>
<223> Yes kinase (Fig. 18)

<220>
<221> SITE
<222> (305)
<223> Constant amino acid K in domain SH2

<220>
<221> SITE
<222> (348)
<223> Constant amino acid T in domain SH2

<220>
<221> SITE
<222> (537)
<223> Constant amino acid Y in domain SH1

<400> 2
Met Gly Cys Ile Lys Ser Lys Glu Asn Lys Ser Pro Ala Ile Lys Tyr
1               5                   10                  15

Arg Pro Glu Asn Thr Pro Glu Pro Val Ser Thr Ser Val Ser His Tyr
            20                  25                  30

Gly Ala Glu Pro Thr Thr Val Ser Pro Cys Pro Ser Ser Ser Ala Lys
        35                  40                  45

Gly Thr Ala Val Asn Phe Ser Ser Leu Ser Met Thr Pro Phe Gly Gly
    50                  55                  60

Ser Ser Gly Val Thr Pro Phe Gly Gly Ala Ser Ser Ser Phe Ser Val
65                  70                  75                  80

Val Pro Ser Ser Tyr Pro Ala Gly Leu Thr Gly Gly Val Thr Ile Phe
                85                  90                  95

Val Ala Leu Tyr Asp Tyr Glu Ala Arg Thr Thr Glu Asp Leu Ser Phe
            100                 105                 110

Lys Lys Gly Glu Arg Phe Gln Ile Ile Asn Asn Thr Glu Gly Asp Trp
        115                 120                 125

Trp Glu Ala Arg Ser Ile Ala Thr Gly Lys Asn Gly Tyr Ile Pro Ser
    130                 135                 140

Asn Tyr Val Ala Pro Ala Asp Ser Ile Gln Ala Glu Glu Trp Tyr Phe
145                 150                 155                 160

Gly Lys Met Gly Arg Lys Asp Ala Glu Arg Leu Leu Leu Asn Pro Gly
                165                 170                 175

Asn Gln Arg Gly Ile Phe Leu Val Arg Glu Ser Glu Thr Thr Lys Gly
            180                 185                 190

Ala Tyr Ser Leu Ser Ile Arg Asp Trp Asp Glu Ile Arg Gly Asp Asn
        195                 200                 205

Val Lys His Tyr Lys Ile Arg Lys Leu Asp Asn Gly Gly Tyr Tyr Ile
    210                 215                 220

Thr Thr Arg Ala Gln Phe Asp Thr Leu Gln Lys Leu Val Lys His Tyr
225                 230                 235                 240

Thr Glu His Ala Asp Gly Leu Cys His Lys Leu Thr Thr Val Cys Pro
                245                 250                 255

Thr Val Lys Pro Gln Thr Gln Gly Leu Ala Lys Asp Ala Trp Glu Ile
            260                 265                 270

Pro Arg Glu Ser Leu Arg Leu Glu Val Lys Leu Gly Gln Gly Cys Phe
        275                 280                 285

Gly Glu Val Trp Met Gly Thr Trp Asn Gly Thr Thr Lys Val Ala Ile
    290                 295                 300

Lys Thr Leu Lys Pro Gly Thr Met Met Pro Glu Ala Phe Leu Gln Glu
305                 310                 315                 320

Ala Gln Ile Met Lys Lys Leu Arg His Asp Lys Leu Val Pro Leu Tyr
                325                 330                 335

Ala Val Val Ser Glu Glu Pro Ile Tyr Ile Val Thr Glu Phe Met Ser
            340                 345                 350

Lys Gly Ser Leu Leu Asp Phe Leu Lys Glu Gly Asp Gly Lys Tyr Leu
        355                 360                 365

Lys Leu Pro Gln Leu Val Asp Met Ala Ala Gln Ile Ala Asp Gly Met
    370                 375                 380

Ala Tyr Ile Glu Arg Met Asn Tyr Ile His Arg Asp Leu Arg Ala Ala
385                 390                 395                 400

Asn Ile Leu Val Gly Glu Asn Leu Val Cys Lys Ile Ala Asp Phe Gly
                405                 410                 415

Leu Ala Arg Leu Ile Glu Asp Asn Glu Tyr Thr Ala Arg Gln Gly Ala
            420                 425                 430

Lys Phe Pro Ile Lys Trp Thr Ala Pro Glu Ala Ala Leu Tyr Gly Arg
        435                 440                 445

Phe Thr Ile Lys Ser Asp Val Trp Ser Phe Gly Ile Leu Gln Thr Glu
    450                 455                 460

Leu Val Thr Lys Gly Arg Val Pro Tyr Pro Gly Met Val Asn Arg Glu
465                 470                 475                 480

Val Leu Glu Gln Val Glu Arg Gly Tyr Arg Met Pro Cys Pro Gln Gly
                485                 490                 495

Cys Pro Glu Ser Leu His Glu Leu Met Asn Leu Cys Trp Lys Lys Asp
            500                 505                 510

Pro Asp Glu Arg Pro Thr Phe Glu Tyr Ile Gln Ser Phe Leu Glu Asp
        515                 520                 525

Tyr Phe Thr Ala Thr Glu Pro Gln Tyr Gln Pro Gly Glu Asn Leu
    530                 535                 540


<210> 3
<211> 537
<212> PRT
<213> Homo sapiens

<220>
<223> Fyn kinase (Fig. 18)

<220>
<221> SITE
<222> (299)
<223> Constant amino acid K in domain SH2

<220>
<221> SITE
<222> (342)
<223> Constant amino acid T in domain SH2

<220>
<221> SITE
<222> (531)
<223> Constant amino acid Y in domain SH1

<400> 3
Met Gly Cys Val Gln Cys Lys Asp Lys Glu Ala Thr Lys Leu Thr Glu
  1               5                  10                  15

Glu Arg Asp Gly Ser Leu Asn Gln Ser Ser Gly Tyr Arg Tyr Gly Thr
             20                  25                  30

Asp Pro Thr Pro Gln His Tyr Pro Ser Phe Gly Val Thr Ser Ile Pro
         35                  40                  45

Asn Tyr Asn Asn Phe His Ala Ala Gly Gly Gln Gly Leu Thr Val Phe
     50                  55                  60

Gly Gly Val Asn Ser Ser Ser His Thr Gly Thr Leu Arg Thr Arg Gly
 65                  70                  75                  80

Gly Thr Gly Val Thr Leu Phe Val Ala Leu Tyr Asp Tyr Glu Ala Arg
                 85                  90                  95

Thr Glu Asp Asp Leu Ser Phe His Lys Gly Glu Lys Phe Gln Ile Leu
            100                 105                 110

Asn Ser Ser Glu Gly Asp Trp Trp Glu Ala Arg Ser Leu Thr Thr Gly
        115                 120                 125

Glu Thr Gly Tyr Ile Pro Ser Asn Tyr Val Ala Pro Val Asp Ser Ile
    130                 135                 140

Gln Ala Glu Glu Trp Tyr Phe Gly Lys Leu Gly Arg Lys Asp Ala Glu
145                 150                 155                 160

Arg Gln Leu Leu Ser Phe Gly Asn Pro Arg Gly Thr Phe Leu Ile Arg
                165                 170                 175

Glu Ser Glu Thr Thr Lys Gly Ala Tyr Ser Leu Ser Ile Arg Asp Trp
            180                 185                 190

Asp Asp Met Lys Gly Asp His Val Lys His Tyr Lys Ile Arg Lys Leu
        195                 200                 205

Asp Asn Gly Gly Tyr Tyr Ile Thr Thr Arg Ala Gln Phe Glu Thr Leu
    210                 215                 220

Gln Gln Leu Val Gln His Tyr Ser Glu Arg Ala Ala Gly Leu Cys Cys
225                 230                 235                 240

Arg Leu Val Val Pro Cys His Lys Gly Met Pro Arg Leu Thr Asp Leu
                245                 250                 255

Ser Val Lys Thr Lys Asp Val Trp Glu Ile Pro Arg Glu Ser Leu Gln
            260                 265                 270

Leu Ile Lys Arg Leu Gly Asn Gly Gln Phe Gly Glu Val Trp Met Gly
        275                 280                 285

Thr Trp Asn Gly Asn Thr Lys Val Ala Ile Lys Thr Leu Lys Pro Gly
    290                 295                 300

Thr Met Ser Pro Glu Ser Phe Leu Glu Glu Ala Gln Ile Met Lys Lys
305                 310                 315                 320

Leu Lys His Asp Lys Leu Val Gln Leu Tyr Ala Val Val Ser Glu Glu
                325                 330                 335

Pro Ile Tyr Ile Val Thr Glu Tyr Met Asn Lys Gly Ser Leu Leu Asp
            340                 345                 350

Phe Leu Lys Asp Gly Glu Gly Arg Ala Leu Lys Leu Pro Asn Leu Val
        355                 360                 365

Asp Met Ala Ala Gln Val Ala Ala Gly Met Ala Tyr Ile Glu Arg Met
    370                 375                 380

Asn Tyr Ile His Arg Asp Leu Arg Ser Ala Asn Ile Leu Val Gly Asn
385                 390                 395                 400

Gly Leu Ile Cys Lys Ile Ala Asp Phe Gly Leu Ala Arg Leu Ile Glu
                405                 410                 415

Asp Asn Glu Tyr Thr Ala Arg Gln Gly Ala Lys Phe Pro Ile Lys Trp
            420                 425                 430

Thr Ala Pro Glu Ala Ala Leu Tyr Gly Arg Phe Thr Ile Lys Ser Asp
        435                 440                 445

Val Trp Ser Phe Gly Ile Leu Leu Thr Glu Leu Val Thr Lys Gly Arg
    450                 455                 460

Val Pro Tyr Pro Gly Met Asn Asn Arg Glu Val Leu Glu Gln Val Glu
465                 470                 475                 480

Arg Gly Tyr Arg Met Pro Cys Pro Gln Asp Cys Pro Ile Ser Leu His
                485                 490                 495

Glu Leu Met Ile His Cys Trp Lys Lys Asp Pro Glu Glu Arg Pro Thr
            500                 505                 510

Phe Glu Tyr Leu Gln Ser Phe Leu Glu Asp Tyr Phe Thr Ala Thr Glu
        515                 520                 525

Pro Gln Tyr Gln Pro Gly Glu Asn Leu
    530                 535


<210> 4
<211> 536
<212> PRT
<213> Chicken

<220>
<223> Yrk kinase (Fig. 18)

<220>
<221> SITE
<222> (298)
<223> Constant amino acid K in domain SH2

<220>
<221> SITE
<222> (341)
<223> Constant amino acid T in domain SH2

<220>
<221> SITE
<222> (530)
<223> Constant amino acid Y in domain SH1

<400> 4
Met Gly Cys Val His Cys Lys Glu Lys Ile Ser Gly Lys Gly Gln Gly
  1               5                  10                  15

Gly Ser Gly Thr Gly Thr Pro Ala His Pro Pro Ser Gln Tyr Asp Pro
             20                  25                  30

Asp Pro Thr Gln Leu Ser Gly Ala Phe Thr His Ile Pro Asp Phe Asn
         35                  40                  45

Asn Phe His Ala Ala Ala Val Ser Pro Pro Val Pro Phe Ser Gly Pro
     50                  55                  60

Gly Phe Tyr Pro Cys Asn Thr Leu Gln Ala His Ser Ser Ile Thr Gly
 65                  70                  75                  80

Gly Gly Val Thr Leu Phe Ile Ala Leu Tyr Asp Tyr Glu Ala Arg Thr
                 85                  90                  95

Glu Asp Asp Leu Ser Phe Gln Lys Gly Glu Lys Phe His Ile Ile Asn

            100                 105                 110

Asn Thr Glu Gly Asp Trp Trp Glu Ala Arg Ser Leu Ser Ser Gly Ala
        115                 120                 125

Thr Gly Tyr Ile Pro Ser Asn Tyr Val Ala Pro Val Asp Ser Ile Gln
    130                 135                 140

Ala Glu Glu Trp Tyr Phe Gly Lys Ile Gly Arg Lys Asp Ala Glu Arg
145                 150                 155                 160

Gln Leu Leu Cys His Gly Asn Cys Arg Gly Thr Phe Leu Ile Arg Glu
                165                 170                 175

Ser Glu Thr Thr Lys Gly Ala Tyr Ser Leu Ser Ile Arg Asp Trp Asp
            180                 185                 190

Glu Ala Lys Gly Asp His Val Lys His Tyr Lys Ile Arg Lys Leu Asp
        195                 200                 205

Ser Gly Gly Tyr Tyr Ile Thr Thr Arg Ala Gln Phe Asp Thr Ile Gln
    210                 215                 220

Gln Leu Val Gln His Tyr Ile Glu Arg Ala Ala Gly Leu Cys Cys Arg
225                 230                 235                 240

Leu Ala Val Pro Cys Pro Lys Gly Thr Pro Lys Leu Ala Asp Leu Ser
                245                 250                 255

Val Lys Thr Lys Asp Val Trp Glu Ile Pro Arg Glu Ser Leu Gln Leu
            260                 265                 270

Leu Gln Lys Leu Gly Asn Gly Gln Phe Gly Glu Val Trp Met Gly Thr
        275                 280                 285

Trp Asn Gly Thr Thr Lys Val Ala Val Lys Thr Leu Lys Pro Gly Thr
    290                 295                 300

Met Ser Pro Glu Ala Phe Leu Glu Glu Ala Gln Ile Met Lys Arg Leu
305                 310                 315                 320

Arg His Asp Lys Leu Val Gln Leu Tyr Ala Val Val Ser Glu Glu Pro
                325                 330                 335

Ile Tyr Ile Val Thr Glu Phe Met Ser Gln Gly Ser Leu Leu Asp Phe
            340                 345                 350

Leu Lys Asp Gly Asp Gly Arg Tyr Leu Lys Leu Pro Gln Leu Val Asp
        355                 360                 365

Met Ala Ala Gln Ile Ala Ala Gly Met Ala Tyr Ile Glu Arg Met Asn
    370                 375                 380

Tyr Ile His Arg Asp Leu Arg Ala Ala Asn Ile Leu Val Gly Asp Asn
385                 390                 395                 400

Leu Val Cys Lys Ile Ala Asp Phe Gly Leu Ala Arg Leu Ile Glu Asp
                405                 410                 415

Asn Glu Tyr Thr Ala Arg Gln Gly Ala Lys Phe Pro Ile Lys Trp Thr
            420                 425                 430

Ala Pro Glu Ala Ala Leu Phe Gly Lys Phe Thr Ile Lys Ser Asp Val

435                 440                 445

Trp Ser Phe Gly Ile Leu Leu Thr Glu Leu Val Thr Lys Gly Arg Val
    450                 455                 460

Pro Tyr Pro Gly Met Asn Asn Arg Glu Val Leu Glu Gln Val Glu Arg
465                 470                 475                 480

Gly Tyr Arg Met Gln Cys Pro Gly Gly Cys Pro Pro Ser Leu His Asp
                485                 490                 495

Val Met Val Gln Cys Trp Lys Arg Glu Pro Glu Glu Arg Pro Thr Phe
            500                 505                 510

Glu Tyr Leu Gln Ser Phe Leu Glu Asp Tyr Phe Thr Ala Thr Glu Pro
        515                 520                 525

Gln Tyr Gln Pro Gly Asp Asn Gln
    530                 535


<210> 5
<211> 529
<212> PRT
<213> Homo sapiens

<220>
<223> Fgr kinase (Fig. 18)

<220>
<221> SITE
<222> (291)
<223> Constant amino acid K in domain SH2

<220>
<221> SITE
<222> (334)
<223> Constant amino acid T in domain SH2

<220>
<221> SITE
<222> (523)
<223> Constant amino acid Y in domain SH1

<400> 5
Met Gly Cys Val Phe Cys Lys Lys Leu Glu Pro Val Ala Thr Ala Lys
1               5                   10                  15

Glu Asp Ala Gly Leu Glu Gly Asp Phe Arg Ser Tyr Gly Ala Ala Asp
            20                  25                  30

His Tyr Gly Pro Asp Pro Thr Lys Ala Arg Pro Ala Ser Ser Phe Ala
        35                  40                  45

His Ile Pro Asn Tyr Ser Asn Phe Ser Ser Gln Ala Ile Asn Pro Gly
    50                  55                  60

Phe Leu Asp Ser Gly Thr Ile Arg Gly Val Ser Gly Ile Gly Val Thr
65                  70                  75                  80

Leu Phe Ile Ala Leu Tyr Asp Tyr Glu Ala Arg Thr Glu Asp Asp Leu
                85                  90                  95

Thr Phe Thr Lys Gly Glu Lys Phe His Ile Leu Asn Asn Thr Glu Gly
            100                 105                 110

Asp Trp Trp Glu Ala Arg Ser Leu Ser Ser Gly Lys Thr Gly Cys Ile
        115                 120                 125

Pro Ser Asn Tyr Val Ala Pro Val Asp Ser Ile Gln Ala Glu Glu Trp
    130                 135                 140

Tyr Phe Gly Lys Ile Gly Arg Lys Asp Ala Glu Arg Gln Leu Leu Ser
145                 150                 155                 160

Pro Gly Asn Pro Gln Gly Ala Phe Leu Ile Arg Glu Ser Glu Thr Thr
                165                 170                 175

Lys Gly Ala Tyr Ser Leu Ser Ile Arg Asp Trp Asp Gln Thr Arg Gly
            180                 185                 190

Asp His Val Lys His Tyr Lys Ile Arg Lys Leu Asp Met Gly Gly Tyr
        195                 200                 205

Tyr Ile Thr Thr Arg Val Gln Phe Asn Ser Val Gln Glu Leu Val Gln
    210                 215                 220

His Tyr Met Glu Val Asn Asp Gly Leu Cys Asn Leu Leu Ile Ala Pro
225                 230                 235                 240

Cys Thr Ile Met Lys Pro Gln Thr Leu Gly Leu Ala Lys Asp Ala Trp
                245                 250                 255

Glu Ile Ser Arg Ser Ser Ile Thr Leu Glu Arg Arg Leu Gly Thr Gly
            260                 265                 270

Cys Phe Gly Asp Val Trp Leu Gly Thr Trp Asn Gly Ser Thr Lys Val
        275                 280                 285

Ala Val Lys Thr Leu Lys Pro Gly Thr Met Ser Pro Lys Ala Phe Leu
    290                 295                 300

Glu Glu Ala Gln Val Met Lys Leu Leu Arg His Asp Lys Leu Val Gln
305                 310                 315                 320

Leu Tyr Ala Val Val Ser Glu Glu Pro Ile Tyr Ile Val Thr Glu Phe
                325                 330                 335

Met Cys His Gly Ser Leu Leu Asp Phe Leu Lys Asn Pro Glu Gly Gln
            340                 345                 350

Asp Leu Arg Leu Pro Gln Leu Val Asp Met Ala Ala Gln Val Ala Glu
        355                 360                 365

Gly Met Ala Tyr Met Glu Arg Met Asn Tyr Ile His Arg Asp Leu Arg
    370                 375                 380

Ala Ala Asn Ile Leu Val Gly Glu Arg Leu Ala Cys Lys Ile Ala Asp
385                 390                 395                 400

Phe Gly Leu Ala Arg Leu Ile Lys Asp Asp Glu Tyr Asn Pro Cys Gln
                405                 410                 415

Gly Ser Lys Phe Pro Ile Lys Trp Thr Ala Pro Glu Ala Ala Leu Phe
            420                 425                 430

Gly Arg Phe Thr Ile Lys Ser Asp Val Trp Ser Phe Gly Ile Leu Leu
        435                 440                 445

Thr Glu Leu Ile Thr Lys Gly Arg Ile Pro Tyr Pro Gly Met Asn Lys
    450                 455                 460

Arg Glu Val Leu Glu Gln Val Glu Gln Gly Tyr His Met Pro Cys Pro
465                 470                 475                 480

Pro Gly Cys Pro Ala Ser Leu Tyr Glu Ala Met Glu Gln Thr Trp Arg
                485                 490                 495

Leu Asp Pro Glu Glu Arg Pro Thr Phe Glu Tyr Leu Gln Ser Phe Leu
            500                 505                 510

Glu Asp Tyr Phe Thr Ser Ala Glu Pro Gln Tyr Gln Pro Gly Asp Gln
        515                 520                 525

Thr

<210> 6
<211> 525
<212> PRT
<213> Homo sapiens

<220>
<223> Hck kinase (Fig. 18)

<220>
<221> SITE
<222> (289)
<223> Constant amino acid K in domain SH2

<220>
<221> SITE
<222> (332)
<223> Constant amino acid T in domain SH2

<220>
<221> SITE
<222> (525)
<223> Constant amino acid Y in domain SH1

<400> 6
Gly Gly Arg Ser Ser Cys Glu Asp Pro Gly Cys Pro Arg Asp Glu Glu
  1               5                  10                  15

Arg Ala Pro Arg Met Gly Cys Met Lys Ser Lys Phe Leu Gln Val Gly
             20                  25                  30

Gly Asn Thr Phe Ser Lys Thr Glu Thr Ser Ala Ser Pro His Cys Pro
         35                  40                  45

Val Tyr Val Pro Asp Pro Thr Ser Thr Ile Lys Pro Gly Pro Asn Ser
     50                  55                  60

His Asn Ser Asn Thr Pro Gly Ile Arg Glu Ala Gly Ser Glu Asp Ile
 65                  70                  75                  80

Ile Val Val Ala Leu Tyr Asp Tyr Glu Ala Ile His His Glu Asp Leu
                 85                  90                  95

Ser Phe Gln Lys Gly Asp Gln Met Val Val Leu Glu Glu Ser Gly Glu
            100                 105                 110

Trp Trp Lys Ala Arg Ser Leu Ala Thr Arg Lys Glu Gly Tyr Ile Pro
        115                 120                 125

Ser Asn Tyr Val Ala Arg Val Asp Ser Leu Glu Thr Glu Glu Trp Phe
    130                 135                 140

Phe Lys Gly Ile Ser Arg Lys Asp Ala Glu Arg Gln Leu Leu Ala Pro
145                 150                 155                 160

Gly Asn Met Leu Gly Ser Phe Met Ile Arg Asp Ser Glu Thr Thr Lys
                165                 170                 175

Gly Ser Tyr Ser Leu Ser Val Arg Asp Tyr Asp Pro Arg Gln Gly Asp
            180                 185                 190

Thr Val Lys His Tyr Lys Ile Arg Thr Leu Asp Asn Gly Gly Phe Tyr
        195                 200                 205

Ile Ser Pro Arg Ser Thr Phe Ser Thr Leu Gln Glu Leu Val Asp His
    210                 215                 220

Tyr Lys Lys Gly Asn Asp Gly Leu Cys Gln Lys Leu Ser Val Pro Cys
225                 230                 235                 240

Met Ser Ser Lys Pro Gln Lys Pro Trp Glu Lys Asp Ala Trp Glu Ile
                245                 250                 255

Pro Arg Glu Ser Leu Lys Leu Glu Lys Lys Leu Gly Ala Gly Gln Phe
            260                 265                 270

Gly Glu Val Trp Met Ala Thr Tyr Asn Lys His Thr Lys Val Ala Val
        275                 280                 285

Lys Thr Met Lys Pro Gly Ser Met Ser Val Glu Ala Phe Leu Ala Glu
    290                 295                 300

Ala Asn Val Met Lys Thr Leu Gln His Asp Lys Leu Val Lys Leu His
305                 310                 315                 320

Ala Val Val Thr Lys Glu Pro Ile Tyr Ile Ile Thr Glu Phe Met Ala
                325                 330                 335

Lys Gly Ser Leu Leu Asp Phe Leu Lys Ser Asp Glu Gly Ser Lys Gln
            340                 345                 350

Pro Leu Pro Lys Leu Ile Asp Phe Ser Ala Gln Ile Ala Glu Gly Met
        355                 360                 365

Ala Phe Ile Glu Gln Arg Asn Tyr Ile His Arg Asp Leu Arg Ala Ala
    370                 375                 380

Asn Ile Leu Val Ser Ala Ser Leu Val Cys Lys Ile Ala Asp Phe Gly
385                 390                 395                 400

Leu Ala Arg Val Ile Glu Asp Asn Glu Tyr Thr Ala Arg Glu Gly Ala
                405                 410                 415

Lys Phe Pro Ile Lys Trp Thr Ala Pro Glu Ala Ile Asn Phe Gly Ser
            420                 425                 430

Phe Thr Ile Lys Ser Asp Val Trp Ser Phe Gly Ile Leu Leu Met Glu
        435                 440                 445

Ile Val Thr Tyr Gly Arg Ile Pro Tyr Pro Gly Met Ser Asn Pro Glu
    450                 455                 460

Val Ile Arg Ala Leu Glu Arg Gly Tyr Arg Met Pro Arg Pro Glu Asn
465                 470                 475                 480

Cys Pro Glu Glu Leu Tyr Asn Ile Met Met Arg Cys Trp Lys Asn Arg
                485                 490                 495

Pro Glu Glu Arg Pro Thr Phe Glu Tyr Ile Gln Ser Val Leu Asp Asp
            500                 505                 510

Phe Tyr Thr Ala Thr Glu Ser Gln Tyr Gln Gln Gln Pro
        515                 520                 525


<210> 7
<211> 512
<212> PRT
<213> Homo sapiens

<220>
<223> Lyn kinase (Fig. 18)

<220>
<221> SITE
<222> (275)
<223> Constant amino acid K in domain SH2

<220>
<221> SITE
<222> (319)
<223> Constant amino acid T in domain SH2

<220>
<221> SITE
<222> (508)
<223> Constant amino acid Y in domain SH1

<400> 7
Met Gly Cys Ile Lys Ser Lys Gly Lys Asp Ser Leu Ser Asp Asp Gly
  1               5                  10                  15

Val Asp Leu Lys Thr Gln Pro Val Arg Asn Thr Glu Arg Thr Ile Tyr
             20                  25                  30

Val Arg Asp Pro Thr Ser Asn Lys Gln Gln Arg Pro Val Pro Glu Ser
         35                  40                  45

Gln Leu Leu Pro Gly Gln Arg Phe Gln Thr Lys Asp Pro Glu Glu Gln
     50                  55                  60

Gly Asp Ile Val Val Ala Leu Tyr Pro Tyr Asp Gly Ile His Pro Asp
 65                  70                  75                  80

Asp Leu Ser Phe Lys Lys Gly Glu Lys Met Lys Val Leu Glu Glu His
                 85                  90                  95

Gly Glu Trp Trp Lys Ala Lys Ser Leu Leu Thr Lys Lys Glu Gly Phe

            100                 105                 110

Ile Pro Ser Asn Tyr Val Ala Lys Leu Asn Thr Leu Glu Thr Glu Glu
        115                 120                 125

Trp Phe Phe Lys Asp Ile Thr Arg Lys Asp Ala Glu Arg Gln Leu Leu
    130                 135                 140

Ala Pro Gly Asn Ser Ala Gly Ala Phe Leu Ile Arg Glu Ser Glu Thr
145                 150                 155                 160

Leu Lys Gly Ser Phe Ser Leu Ser Val Arg Asp Phe Asp Pro Val His
                165                 170                 175

Gly Asp Val Ile Lys His Tyr Lys Ile Arg Ser Leu Asp Asn Gly Gly
            180                 185                 190

Tyr Tyr Ile Ser Pro Arg Ile Thr Phe Pro Cys Ile Ser Asp Met Ile
        195                 200                 205

Lys His Tyr Gln Lys Gln Ala Asp Gly Leu Cys Arg Arg Leu Glu Lys
    210                 215                 220

Ala Cys Ile Ser Pro Lys Pro Gln Lys Pro Trp Asp Lys Asp Ala Trp
225                 230                 235                 240

Glu Ile Pro Arg Glu Ser Ile Lys Leu Val Lys Arg Leu Gly Ala Gly
                245                 250                 255

Gln Phe Gly Glu Val Trp Met Gly Tyr Tyr Asn Asn Ser Thr Lys Val
            260                 265                 270

Ala Val Lys Thr Leu Lys Pro Gly Thr Met Ser Val Gln Ala Phe Leu
        275                 280                 285

Glu Glu Ala Asn Leu Met Lys Thr Leu Gln His Asp Lys Leu Val Arg
    290                 295                 300

Leu Tyr Ala Val Val Thr Arg Glu Glu Pro Ile Tyr Ile Ile Thr Glu
305                 310                 315                 320

Tyr Met Ala Lys Gly Ser Leu Leu Asp Phe Leu Lys Ser Asp Glu Gly
                325                 330                 335

Gly Lys Val Leu Leu Pro Lys Leu Ile Asp Phe Ser Ala Gln Ile Ala
            340                 345                 350

Glu Gly Met Ala Tyr Ile Glu Arg Lys Asn Tyr Ile His Arg Asp Leu
        355                 360                 365

Arg Ala Ala Asn Val Leu Val Ser Glu Ser Leu Met Cys Lys Ile Ala
    370                 375                 380

Asp Phe Gly Leu Ala Arg Val Ile Glu Asp Asn Glu Tyr Thr Ala Arg
385                 390                 395                 400

Glu Gly Ala Lys Phe Pro Ile Lys Trp Thr Ala Pro Glu Ala Ile Asn
                405                 410                 415

Phe Gly Cys Phe Thr Ile Lys Ser Asp Val Trp Ser Phe Gly Ile Leu
            420                 425                 430

Leu Tyr Glu Ile Val Thr Tyr Gly Lys Ile Pro Tyr Pro Gly Arg Thr

435                 440                 445

Asn Ala Asp Val Met Thr Ala Leu Ser Gln Gly Tyr Arg Met Pro Arg
    450                 455                 460

Val Glu Asn Cys Pro Asp Glu Leu Tyr Asp Ile Met Lys Met Cys Trp
465                 470                 475                 480

Lys Glu Lys Ala Glu Glu Arg Pro Thr Phe Asp Tyr Leu Gln Ser Val
                485                 490                 495

Leu Asp Asp Phe Tyr Thr Ala Thr Glu Gly Gln Tyr Gln Gln Gln Pro
            500                 505                 510

<210> 8
<211> 509
<212> PRT
<213> Homo sapiens

<220>
<223> Lck kinase (Fig. 18)

<220>
<221> SITE
<222> (273)
<223> Constant amino acid K in domain SH2

<220>
<221> SITE
<222> (316)
<223> Constant amino acid T in domain SH2

<220>
<221> SITE
<222> (505)
<223> Constant amino acid Y in domain SH1

<400> 8
Met Gly Cys Gly Cys Ser Ser His Pro Glu Asp Asp Trp Met Glu Asn
  1               5                  10                  15

Ile Asp Val Cys Glu Asn Cys His Tyr Pro Ile Val Pro Leu Asp Gly
             20                  25                  30

Lys Gly Thr Leu Leu Ile Arg Asn Gly Ser Glu Val Arg Asp Pro Leu
         35                  40                  45

Val Thr Tyr Glu Gly Ser Asn Pro Pro Ala Ser Pro Leu Gln Asp Asn
     50                  55                  60

Leu Val Ile Ala Leu His Ser Tyr Glu Pro Ser His Asp Gly Asp Leu
 65                  70                  75                  80

Gly Phe Glu Lys Gly Glu Pro Leu Arg Ile Leu Glu Gln Ser Gly Glu
                 85                  90                  95

Trp Trp Lys Ala Gln Ser Leu Thr Thr Gly Gln Glu Gly Phe Ile Pro
            100                 105                 110

Phe Asn Phe Val Ala Lys Ala Asn Ser Leu Glu Pro Glu Pro Trp Phe
        115                 120                 125

Phe Lys Asn Leu Ser Arg Lys Asp Ala Glu Arg Gln Leu Leu Ala Pro
    130                 135                 140

Gly Asn Thr His Gly Ser Phe Leu Ile Arg Glu Ser Glu Ser Thr Ala
145                 150                 155                 160

Gly Ser Phe Ser Leu Ser Val Arg Asp Phe Asp Gln Asn Gln Gly Glu
                165                 170                 175

Val Val Lys His Tyr Lys Ile Arg Asn Leu Asp Asn Gly Gly Phe Tyr
            180                 185                 190

Ile Ser Pro Arg Ile Thr Phe Pro Gly Leu His Glu Leu Val Arg His
        195                 200                 205

Tyr Thr Asn Ala Ser Asp Gly Leu Cys Thr Arg Leu Ser Arg Pro Cys
    210                 215                 220

Gln Thr Gln Lys Pro Gln Lys Pro Trp Trp Glu Asp Glu Trp Glu Val
225                 230                 235                 240

Pro Arg Glu Thr Leu Lys Leu Val Glu Arg Leu Gly Ala Gly Gln Phe
                245                 250                 255

Gly Glu Val Trp Met Gly Tyr Tyr Asn Gly His Thr Lys Val Ala Val
            260                 265                 270

Lys Ser Leu Lys Gln Gly Ser Met Ser Pro Asp Ala Phe Leu Ala Glu
        275                 280                 285

Ala Asn Leu Met Lys Gln Leu Gln His Gln Arg Leu Val Arg Leu Tyr
    290                 295                 300

Ala Val Val Thr Gln Glu Pro Ile Tyr Ile Ile Thr Glu Tyr Met Glu
305                 310                 315                 320

Asn Gly Ser Leu Val Asp Phe Leu Lys Thr Pro Ser Gly Ile Lys Leu
                325                 330                 335

Thr Ile Asn Lys Leu Leu Asp Met Ala Ala Gln Ile Ala Glu Gly Met
            340                 345                 350

Ala Phe Ile Glu Glu Arg Asn Tyr Ile His Arg Asp Leu Arg Ala Ala
        355                 360                 365

Asn Ile Leu Val Ser Asp Thr Leu Ser Cys Lys Ile Ala Asp Phe Gly
    370                 375                 380

Leu Ala Arg Leu Ile Glu Asp Asn Glu Tyr Thr Ala Arg Glu Gly Ala
385                 390                 395                 400

Lys Phe Pro Ile Lys Trp Thr Ala Pro Glu Ala Ile Asn Tyr Gly Thr
                405                 410                 415

Phe Thr Ile Lys Ser Asp Val Trp Ser Phe Gly Ile Leu Leu Thr Glu
            420                 425                 430

Ile Val Thr His Gly Arg Ile Pro Tyr Pro Gly Met Thr Asn Pro Glu
        435                 440                 445

Val Ile Gln Asn Leu Glu Arg Gly Tyr Arg Met Val Arg Pro Asp Asn
    450                 455                 460

Cys Pro Glu Glu Leu Tyr Gln Leu Met Arg Leu Cys Trp Lys Glu Arg
465                 470                 475                 480

Pro Glu Asp Arg Pro Thr Phe Asp Tyr Leu Arg Ser Val Leu Glu Asp
                485                 490                 495

Phe Phe Thr Ala Thr Glu Gly Gln Tyr Gln Pro Gln Pro
            500                 505


<210> 9
<211> 505
<212> PRT
<213> Homo sapiens

<220>
<223> Blk kinase (Fig. 18)

<220>
<221> SITE
<222> (269)
<223> Constant amino acid K in domain SH2

<220>
<221> SITE
<222> (312)
<223> Constant amino acid T in domain SH2

<220>
<221> SITE
<222> (501)
<223> Constant amino acid Y in domain SH1

<400> 9
Met Gly Leu Val Ser Ser Lys Lys Pro Asp Lys Glu Lys Pro Ile Lys
  1               5                  10                  15

Glu Lys Asp Lys Gly Gln Trp Ser Pro Leu Lys Val Ser Ala Gln Asp
             20                  25                  30

Lys Asp Ala Pro Pro Leu Pro Pro Leu Val Val Phe Asn His Leu Thr
         35                  40                  45

Pro Pro Pro Pro Asp Glu His Leu Asp Glu Asp Lys His Phe Val Val
     50                  55                  60

Ala Leu Tyr Asp Tyr Thr Ala Met Asn Asp Arg Asp Leu Gln Met Leu
 65                  70                  75                  80

Lys Gly Glu Lys Leu Gln Val Leu Lys Gly Thr Gly Asp Trp Trp Leu
                 85                  90                  95

Ala Arg Ser Leu Val Thr Gly Arg Glu Gly Tyr Val Pro Ser Asn Phe
            100                 105                 110

Val Ala Arg Val Glu Ser Leu Glu Met Glu Arg Trp Phe Phe Arg Ser
        115                 120                 125

Gln Gly Arg Lys Glu Ala Glu Arg Gln Leu Leu Ala Pro Ile Asn Lys
    130                 135                 140

Ala Gly Ser Phe Leu Ile Arg Glu Ser Glu Thr Asn Lys Gly Ala Phe
145                 150                 155                 160

Ser Leu Ser Val Lys Asp Val Thr Thr Gln Gly Glu Leu Ile Lys His
                165                 170                 175

Tyr Lys Ile Arg Cys Leu Asp Glu Gly Gly Tyr Tyr Ile Ser Pro Arg
            180                 185                 190

Ile Thr Phe Pro Ser Leu Gln Ala Leu Val Gln His Tyr Ser Lys Lys
        195                 200                 205

Gly Asp Gly Leu Cys Gln Arg Leu Thr Leu Pro Cys Val Arg Pro Ala
    210                 215                 220

Pro Gln Asn Pro Trp Ala Gln Asp Glu Trp Glu Ile Pro Arg Gln Ser
225                 230                 235                 240

Leu Arg Leu Val Arg Lys Leu Gly Ser Gly Gln Phe Gly Glu Val Trp
                245                 250                 255

Met Gly Tyr Tyr Lys Asn Asn Met Lys Val Ala Ile Lys Thr Leu Lys
            260                 265                 270

Glu Gly Thr Met Ser Pro Glu Ala Phe Leu Gly Glu Ala Asn Met Met
        275                 280                 285

Lys Ala Leu Gln His Glu Arg Leu Val Arg Leu Tyr Ala Val Val Thr
    290                 295                 300

Lys Glu Pro Ile Tyr Ile Val Thr Glu Tyr Met Ala Arg Gly Cys Leu
305                 310                 315                 320

Leu Asp Phe Leu Lys Thr Asp Glu Gly Ser Arg Leu Ser Leu Pro Arg
                325                 330                 335

Leu Ile Asp Met Ser Ala Gln Ile Ala Glu Gly Met Ala Tyr Ile Glu
            340                 345                 350

Arg Met Asn Ser Ile His Arg Asp Leu Arg Ala Ala Asn Ile Leu Val
        355                 360                 365

Ser Glu Ala Leu Cys Cys Lys Ile Ala Asp Phe Gly Leu Ala Arg Ile
    370                 375                 380

Ile Asp Ser Glu Tyr Thr Ala Gln Glu Gly Ala Lys Phe Pro Ile Lys
385                 390                 395                 400

Trp Thr Ala Pro Glu Ala Ile His Phe Gly Val Phe Thr Ile Lys Ala
                405                 410                 415

Asp Val Trp Ser Phe Gly Val Leu Leu Met Glu Val Val Thr Tyr Gly
            420                 425                 430

Arg Val Pro Tyr Pro Gly Met Ser Asn Pro Glu Val Ile Arg Asn Leu
        435                 440                 445

Glu Arg Gly Tyr Arg Met Pro Arg Pro Asp Thr Cys Pro Pro Glu Leu
    450                 455                 460

Tyr Arg Gly Val Ile Ala Glu Cys Trp Arg Ser Arg Pro Glu Glu Arg
465                 470                 475                 480

Pro Thr Phe Glu Phe Leu Gln Ser Val Leu Glu Asp Phe Tyr Thr Ala
                485                 490                 495

Thr Glu Arg Gln Tyr Glu Leu Gln Pro
            500                 505

<210> 10
<211> 34
<212> DNA
<213> Artificial Sequence

<220>
<223> Description of Artificial Sequence:
      Oligonucleotide (sense) used for the generation of
      Src-K298A (page 35 of description)

<400> 10
ccagggtggc catcgccacc ctgaagcctg gcac                                34

<210> 11
<211> 34
<212> DNA
<213> Artificial Sequence

<220>
<223> Description of Artificial Sequence:
      Oligonucleotide (antisense) used for the
      generation of Src-K298A (page 35 of description)

<400> 11
gtgccaggct tcagggtggc gatggccacc ctgg                                34

<210> 12
<211> 32
<212> DNA
<213> Artificial Sequence

<220>
<223> Description of Artificial Sequence:
      Oligonucleotide (sense) used for the generation of
      Src-T341Q (page 35 of description)

<400> 12
ccatttacat cgtccaggag tacatgagca ag                                  32

<210> 13
<211> 32
<212> DNA
<213> Artificial Sequence

<220>
<223> Description of Artificial Sequence:
      Oligonucleotide (antisense) used for the
      generation of Src-T341Q (page 35 of description)

<400> 13
cttgctcatg tactcctgga cgatgtaaat gg                                  32

<210> 14
<211> 29
<212> DNA
<213> Artificial Sequence

<220>
<223> Description of Artificial Sequence:
      Oligonucleotide (sense) used for the generation of
      Src-Y530F (page 35 of description)

<400> 14
ccaccgagcc ccagttccag cccggggag                                   29


<210> 15
<211> 29
<212> DNA
<213> Artificial Sequence

<220>
<223> Description of Artificial Sequence:
      Oligonucleotide (antisense) used for the
      generation of Src-Y530F (page 35 of description)

<400> 15
ctccccgggc tggaactggg gctcggtgg                                   29

## Description

The present invention relates to methods of identifying, selecting and/or characterizing compounds which modulate the activity of a Src family kinase and their use in the treatment of diseases, which are at least in part caused by at least one Src family kinase.

Various methods of selecting, identifying and/or characterizing compounds are known in the art. These screening methods are used to identify and/or characterize compounds for their potency to bind to target proteins and/or to modulate the function of target proteins. Methods of this kind are available for numerous protein targets which are inter alia involved in the etiology of certain pathological disorders.

The most common screening systems are based on measurements of the binding constants of the compound to be tested for their binding properties to certain·target proteins. However, it is preferred to use cell based assays to screen for potentially interesting compounds. In general, these cell based assays make use of an overexpression of the target protein to permit large-scale screening. Nagy et al. describe an inducible expression system for NMDA receptors which may be used for drug screening purposes (Neurochem. Int. 2003; 43(1), 19-29)). A related method is disclosed by Uchino et al. (Brain Res. Mol. Brain Res., 1997; 44(1), 1-11), namely a cell-based, inducible drug screening system for compounds acting on an NMDA receptor channel. Vanhoenacker et al. describe a high level expression system of human serotonin receptors in L929 cells using an inducible expression system (Receptor Channels; 1997; 5(3-4), 125-137). This system is established for characterization purposes of a potentially interesting substance.

One protein target of interest amongst many others, for which various methods of identifying or characterising compounds (with activity to modulate the target protein function) are available, is e.g. Hsp 90 (Heat shock protein 90). The Hsp90 family of proteins is a group of highly conserved stress proteins that are expressed in all eukaryotic cells. It plays a key role in the cellular stress response by interacting with many proteins after their native conformation has been altered by environmental stresses, such as heat shock, ensuring adequate protein folding and preventing non-specific aggregation (Smith et al. (1998) Pharmacological Review, (50) 493-513). Beside that Hsp90 has an important regulatory role by maintaining the conformational stability and maturation of several key client proteins.

For example, WO 03/067262 discloses a method of identifying compounds which modulate the interaction between the Hsp90 and the co-factor Aha-1. The method is based on a cell based assay. Upon addition of a test compound an effect on the cells may be observed, e.g. determination of the morphological status of the cells. Assay methods disclosed therein may be used to determine the ability of test compounds to inhibit cellular proliferation. WO 03/050295 relates to a cell-based method for identifying compounds modulating a high affinity form of Hsp90, which occurs in certain tumor cells. WO00/686693 discloses a method of screening and identifying a molecule that alters the centrosomal location of Hsp90 in a cell. The modulating effect of a test compound on Hsp90 may also affect the correct folding of proteins interacting with Hsp90. In such a case, the compound can act as an indirect inhibitor of the protein interacting with Hsp90.

In other words, methods known in the art for the identification/characterization of compounds with modulating effects on target proteins, e.g. Hsp90 amongst numerous other target proteins, may be based on a variety of mechanisms depending upon the specific cellular function of the respective target protein and may be determined by direct or indirect modes of action.

Other cellular protein targets, which play a key role for cellular processes, are the members of the Src kinase family. These kinases are implicated in e.g. cancer, immune system dysfunction and bone remodelling diseases. For general reviews, see Thomas and Brugge, Annu. Rev. Cell Dev. Biol. (1997) 13, 513; Lawrence and Niu, Pharmacol. Ther. (1998) 77, 81; Tatosyan and Mizenina, Biochemistry (Moscow) (2000) 65, 49; Boschelli et al., Drugs of the Future 2000, 25(7), 717, (2000).

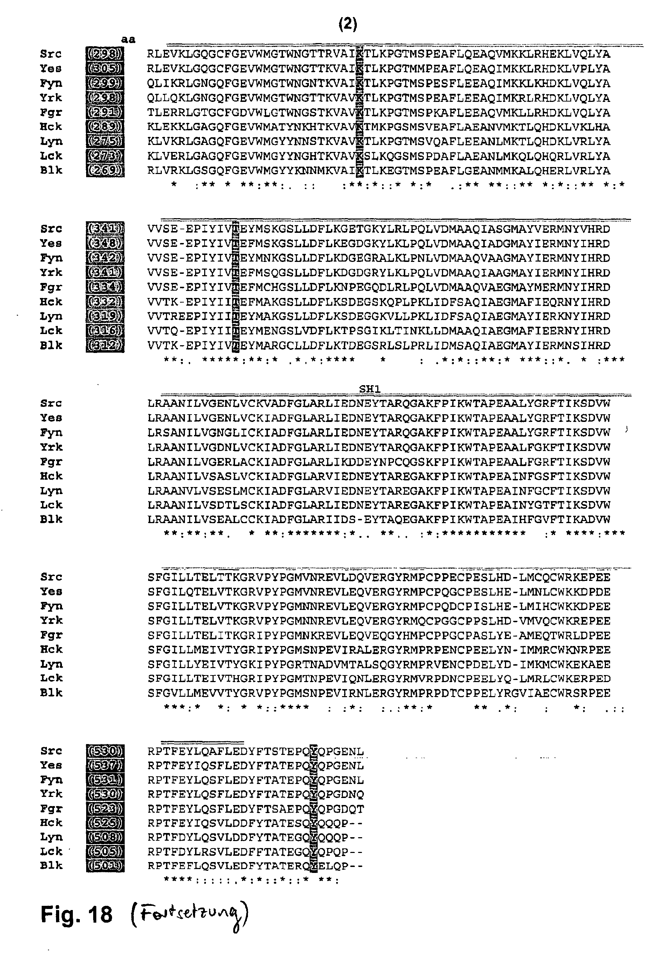

Members of the Src family include the following nine kinases in mammals: Src, Fyn, Yes, Fgr, Lyn, Hck, Lck, Blk and Yrk. These are non receptor protein kinases that range in molecular mass from 52 to 62 kD. All are characterized by a common structural organization that is comprised of six distinct functional domains: Src homology domain 4 (SH4), a unique domain, SH3 domain, SH2 domain, a catalytic domain (SH1), and a C-terminal regulatory region (Tatosyan et al. Biochemistry (Moscow) 65, 49-58 (2000)). An alignment of the sequences of said members of the Src family is given in Figure 18. All sequences are human, except Yrk which is from chicken.

Src (a member of the Src kinase family) was the first Src family member identified. Its identification was due to the discovery of an oncogenic variant, "v-Src", in the genome of Rous sarcoma virus which was considered to be responsible for the rapid eruption of tumors in animals infected with this avian retrovirus (Jove and Hanafusa, 1987. Cell transformation by the viral src oncogene. Annu. Rev. Cell Biol. 3, 31-56.). Its cellular homolog, c-Src or Src, is involved in cell division (probably through interaction with different growth factor receptors), as well as cell shape changes, adhesion and motility through regulation of integrin signalling and cytoskeletal architecture (Roche et al., 1995, Science 269, 1567-9; Mao et al., 1997, Oncogene 15, 3083-90; Parsons and Parsons, 1997, Curr. Opin. Cell Biol. 9, 187-92; Abu-Ghazaleh et al., 2001, Biochem. J. 360, 255-64; Belsches-Jablonski et al., 2001, Oncogene 20, 1465-75; Avizienyte et al., 2002, Nat. Cell Biol. 4, 632-638; Frame, 2002, BBA 1602, 114-30; Kitagawa et al., 2002, J. Biol. Chem. 277, 366-71).

Furthermore, Src is involved in the replication of hepatitis B virus. The virally encoded transcription factor HBx activates Src in a step required for propagation of the virus (Klein et al., EMBO J., 18, 5019, (1999) and Klein et al., Mol.Cell. Biol., 17, 6427 (1997)).

Because of its functions, a number of studies have implicated Src in the generation and/or progression of several types of cancer, including breast, hepatic, pancreatic and ovarian cancer and in particular several kinds of leukemia and lymphomas as well as colon cancer (Cartwright et al., 1990, Proc. Natl. Acad. Sci. USA 87, 558-62; Lynch et al., 1993, Leukemia 7, 1416-22; Pories et al., 1998, Gastroenterology 114, 1287-95; Frame, 2002, BBA 1602, 114-30; Talamonti et al., J. Clin. Invest., 91, 53 (1993); Lutz et al., Biochem. Biophys. Res. 243, 503 (1998); Rosen et al., J. Biol. Chem., 261, 13754 (1986); Bolen et al., Proc. Natl. Acad. Sci. USA, 84, 2251 (1987); Masaki et al., Hepatology, 27, 1257 (1998); Biscardi et al., Adv. Cancer Res., 76, 61 (1999); Lynch et al., Leukemia, 7, 1416 (1993)) and other disorders.

An unambiguous correlation between Src kinase activity and advancing tumor stage in colon cancer has been demonstrated (Talamonti et al., J. Clin. Invest., 91, 53 (1993)). It was shown that there is a strong positive correlation between Src activity and tumor progression from non-malignant polyps towards invasive cancers and metastatic foci. Therefore, activation of Src kinase in primary colorectal carcinoma entails a poor clinical prognosis (Aligayer et al., 2002, Cancer 94, 344-51). A reduced activation of Src in human colon and ovarian cancer cells was shown to reduce their tumorigenicity, suggesting that Src inhibitors could have a potential as anti-cancer drugs (Wiener et al., Clin. Cancer Res., 5, 2164 (1999); Staley et al., Cell Growth Diff., 8, 269 (1997)). Colon cancer is a common disease which leads to death in ~50% of the cases as a consequence of metastasis. Thus, therapeutic substances are highly desired for more successful treatment strategies of colon cancer and other cancers as well.

Since Src and other Src family members (which are involved in similar signal transduction pathways as Src and reflect, therefore, a spectrum of functions within the cell which is quite similar to Src's physiological function) are involved in various pathologic disorders, there is a long lasting need in the art for methods that allow to identify and characterise compounds modulating the activity of Src family members. While there are methods known in the art that merely allow to screen for compounds with binding affinity to Src family members, at this time no system exists which allows to a) detect/identify compounds, which modulate the activity of members of the Src kinase family and b) distinguish between different modes of action of identified compounds, in particular between direct and indirect effects on a member of the Src family, different binding modes or the characteristic specificity of the identified test compound(s).

Nevertheless, such a system is highly required to obtain specific conclusions about the effectiveness, mode of action and specificity of a test compound. Furthermore, experimental results giving these conclusions are of utmost importance for further research activities directed to the use of a compound as a medicament for the prevention or treatment of a disease or disorder state, which is at least partially caused by a Src family member.

Consequently, it is the object of the present invention to provide methods for the identification of compounds (modulating the function of Src kinase family members), by which discriminating clues are given for the effectiveness, the mode of action and the specificity of the test compounds used.

According to the invention a cellular assay is provided, by which (i) an inducible expression of Src family kinases or Src family kinase mutants is carried out and (ii) a determination of the effectiveness, mode of action and the specificity of a test compound towards Src family kinases is based on phenotype changes of the cells upon contact with the test compound(s). The extent of suppression of phenotype changes by the test compound is used as a read-out of the method according to the invention. These phenotype changes can be qualitatively observed with a microscope and/or can be quantified employing various assay formats.

The present invention relates in its first embodiment to a method of identifying, selecting and/or characterizing a compound, which modulates the activity of at least one Src family kinase, comprising the steps of:
(a) cultivating at least one cell (or at least one cell line) containing at least one nucleic acid coding for a Src family kinase or a mutated Src family kinase under suitable conditions,
(b) expressing the nucleic acid(s) in the cell(s) of step (a) under suitable conditions,
(c) contacting the cell(s) of step (b) with at least one test compound and
(d) determining whether the phenotype of the cell(s) of step (c) is changed as compared with the phenotype of the cell(s) of step (b) whereby a difference in the phenotype indicates that said test compound modulates the activity of at least one Src family kinase.

"Src family kinase" according to the invention means a protein tyrosine kinase form the Src family and relates to any member of the Src family, including but not limited to Src, Yes, Fgr, Fyn, Lck, Hck, Lyn, Blk and Yrk. Preferably, the Src family kinases are vertebrate, in particular mammalian kinases (e.g. horse, pig, chicken, rat, mouse. Human Src family kinase sequences are most preferred.

"Mutated Src family kinase" or "Src family kinase mutant" according to the invention means a Src family kinase which shows a change in at least one amino acid. Such a change includes any possible alteration in the amino acid sequence (caused e.g. by addition, deletion, substitution etc.) and modification of amino acids.

"Src" or "Src kinase" according to the invention relates to the aforementioned member Src of the Src family. This applies correspondingly to "mutated Src" or "mutated Src kinase".

"Compounds" according to the invention relates to any molecules which may or may not modulate the activity of at least one Src family kinase or Src family kinase mutant, in particular they can either directly or indirectly modulate at least one Src family kinase or Src family kinase mutant (direct or indirect Src family kinase inhibitors). "Compound" may be a small (organic) chemical entity, peptide antibody or intrabody molecule that effect the function of at least one member of the Src family. Suitable compounds may be selected from compound collections or designed compounds, for example using combinatorial chemistry. Preferably, the compound has a molecular weight of less than 3000, more preferably of less than 1500 Da. "Compound" may be a peptide or a polypeptide, a modified peptide, e.g. a cyclic peptide or modified at the terminal end groups, e.g. with the introduction of a chloromethyl ketone, aldehyde, or boronic acid group at the C-Terminus or blocked at the N-terminus with a carbobenzyl group, a naturally occurring substance or a conjugate substance comprising at least two components.

"Contacting" according to the invention means - in its broadest sense - any interaction between the test compound with the Src family kinases or mutated Src family kinases of the invention, whereby any of the two components may be independently of each other in a liquid phase, for example in solution, or in suspension or can be bound to a solid phase, for example, in the form of an essentially planar surface or in the form of particles, pearls or the like.

The term "contacting" more specifically comprises any possibility of interaction between a test compound and a cell or cell line of the invention. A test compound may be added to a cell or cell line or a cell or cell line may be added to a test compound, e.g. in solution.

The change of "phenotype" according to the invention relates to any kind of observable change, e.g. change in/of cell viability, cell death (apoptosis, necrosis), differentiation, morphology, motility, metabolism, plasticity, cellular proliferation and/or features of cell growth, e.g. growth in one, two, three etc. layers, aggregation/clustering of cells, distribution of cells over a surface or a culture dish.

In a preferred embodiment the cell(s) or the cell line of step (a) of the method according to the invention exhibits the ability to inducibly display a characteristic phenotype reflecting the, preferably inducible, expression of at least one Src family kinase and/or at least one mutated Src family kinase, preferably a hyperactivated form of a Src family kinase and/or a mutated Src family kinase. Thereby, the inventive method allows to observe the phenotype of a cell being induced to (over)express at least one Src kinase and/or mutated Src kinase as a direct read-out.

In another preferred embodiment the cell of step (a) of the method according to the invention exhibits the ability to identify a compound which modulate the expression or activity of a Src family kinase or a mutated Src family kinase in the context of a living cell. Upon addition of a test compound the "Src-like" phenotype (as a result of the (over)expression of the Src family kinase) develops (no effect of the test compound on the induced cell) or the phenotype shows upon addition of the test compound various degrees of deviations from the "Src-like" phenotype (test compound induces an effect on the cell).

In a further preferred embodiment the method of the invention gives a clue to the mode of action of test compounds. Their mode of action may be direct or indirect, since the test compound may exert its impact on the cell e.g. via direct binding to a Src kinase or via indirect effects, e.g. by interfering with the Hsp90 chaperone system, on the activity of which proper folding of Src kinases depend. By using kinase dead Src family kinases it becomes possible to determine whether these compounds exert their effect via direct inhibition of Src kinase activity or by interaction with non catalytic domains of a Src kinase or - by indirect effects - affecting Src folding and expression.

In a further preferred embodiment the cell of step (a) of the method according to the invention exhibits the ability to determine the selectivity and/or specificity of a compound.

According to the invention it is preferred, that at least one, preferably two, more preferably three and most preferably four of the properties ((i) "Src-like" phenotype, (ii) detectability of the compound effect, (iii) conclusion about the mode of action, (iv) selectivity and specificity of the compound) are fulfilled by a method according to the invention. The number of the Src family kinase(s) and/or mutants of these Src family kinase(s) to be used in step (a) of the method of the invention in order to get information about the aforementioned properties depends on their characteristics. E.g. a wild type Src family kinase will generally provide information pertaining to the properties (i) and (ii). A mutant of a Src kinase family member may even additionally open access by its own to the mode of action of a test compound (iii) (in other words direct or indirect effects of the test compound) and/or (iv) to the specificity/selectivity of a test compound. However, according to the invention it may be necessary to use (at least) two different Src family kinases and/or typically mutants of Src family kinases to retrieve information about the properties (iii) and (iv), respectively, of the compounds tested.

In a preferred embodiment of the method according to the present invention a wildtype Src family kinase is used and at least one further mutant Src family kinase. The mutant Src family kinase(s) used for the assay according to the invention may show mutations which lead to hyperactivated forms of a Src family kinase member inducing a very strong phenotype, to a kinase dead mutant Src family kinase member (without any kinase activity) and/or to a mutant Src family kinase member which allows to determine the specificity of active compounds for specific Src family kinases. Additional mutants may be generated and used. Preferably single amino acid substitutions are used for generating these Src family kinase mutants. However, the mutants may also be based on insertions or deletions.

"Kinase dead" Src family kinase mutants are typically based on mutations in the ATP binding site, preferably in at least one consensus amino acid, of a member of the Src family kinase resulting e.g. in an abolishment of the binding of ATP to the ATP binding site ("kinase dead mutant type"). Such a mutation leads to an inactive Src family kinase. Nevertheless, any Src family kinase mutant with any other mutation of a Src family kinase exhibiting loss of ATP binding may be suitable for use of a method according to the invention. These "dead end" Src family kinase mutants are preferably used as a control for the mode of action of a test compound. Such a control "kinase-dead" Src family kinase mutant can be used to distinguish whether a test compound inhibits the Src family kinase activity, interacts with non-catalytic domains and functions or affects the folding and/or expression of the Src family kinase.

Another preferred embodiment of the method according to the invention employs at least one Src family kinase mutant which can be used to determine the specificity and binding mode of a test compound ("specificity determining mutant type"). This Src family kinase mutant according to the invention is encoded by a nucleic acid (coding for a Src family kinase) containing at least one mutation, e.g. in the ATP binding site, preferably by substitution of at least one amino acid, which acts as a specificity determinant of the encoded Src family kinase member or the Src kinase family as such. Preferably the mutant has a mutation within the ATP binding site, e.g. a deletion, substitution or modification. It is preferred that the mutation relates to at least one amino acid which is characteristic for Src family kinases (and eventually some other related tyrosine kinases) in contrast to other tyrosine kinases, e.g. the mutant relates to a substitution, modification or deletion of a threonine in the ATP binding site, particularly T341 of Src or the analogous residues of the other Src family member. However, any mutant of a Src family kinase member with any other mutation exhibiting the described property is also usable for carrying out a method according to invention. Such a Src family kinase mutant at a specificity determining residue affects the following: A test compound binding to the ATP binding site and exhibiting a significant degree of specificity for a Src family kinase (wildtype) will not bind with high affinity to said Src family kinase mutant. Consequently, the test compound will not inhibit this Src family kinase mutant with a potency comparable to a Src family kinase (wildtype). However, this Src family kinase mutant shows wildtype-like activity (wildtype or hyperactive kinase activity) and therefore, both are indistinguishable otherwise. Thus, this Src family kinase mutant allows to determine the binding mode and specificity of a test compound.

A further preferred embodiment of the method according to the invention employs at least one Src family kinase mutant which causes an hyperactive form of a Src family kinase ("hyperactive mutant type"). Such a mutation e.g. renders phoshorylation of an amino acid of the regulatory residue impossible, e.g. at the (tyrosine) phosphorylation site. The phosphorylation of this amino acid is necessary for switching a Src family kinase from an active into the inactive form. Since the ratio of phosphorylation/dephosphorylation regulates the inactive/active conformation of a Src family kinase, a mutation, which does not allow the Src family kinase to be phosphorylated, leads to a permanent and constitutive activation and thus, to a hyperactive form of said Src family kinase. Thus, the binding of a test compound to this hyperactive Src family kinase mutant leads to a characteristic phenotype. In general, an assay according to the invention employs preferably a Src family kinase mutant, which cannot be phosphorylated at the regulatory site. Such a Src family kinase mutant according to the invention can be encoded by a nucleic acid (coding for a Src family kinase) exhibiting a mutation, e.g. at the regulatory phosphorylation site, particularly concerning or involving the regulatory tyrosin residue involved (which may be substituted for another amino acid or deleted or modified). This phosphorylation site is located in the carboxy-terminal tail of a Src family kinase which controls the kinase activity. Nevertheless, any other mutation of a Src family kinase leading to the described hyperactive form is employable for a method according to the present invention.

Another preferred embodiment of the method according to the invention employs at least one Src family kinase mutant which leads (i) to a hyperactive form of a Src family kinase and, moreover, (ii) exhibits a mutation which allows to determine the specificity and binding mode of a test compound ("double mutant type"). Typically such a Src family kinase mutant has at least two mutations, preferably a mutation leading to a hyperactive form and mutation of at least one specificity determining residue (mutations as outlined above). Consequently, this Src family kinase mutant according to the invention can be encoded by a nucleic acid (coding for a Src family kinase) which combines these mutations. More precisely, a nucleic acid comprising (i) at least one mutation at the regulatory (tyrosine) phosphorylation site in the carboxy-terminal tail, regulating the ratio of inactive/active form of a Src family kinase, as described above, and (ii) at least one other mutation, e.g. in the ATP binding site, preferably by a substitution, modification or deletion of a threonine residue in the ATP binding site, which represent(s) a specificity determinant as described above.

This type of Src kinase family member "double" mutant may be used for a method according to the invention in combination with at least one other type of the aforementioned types of Src family kinase mutants (e.g. hyperactive mutant, kinase dead mutant and/or a specificity determining mutant). In general, at least one, preferably two, more preferably three and most preferably four mutant types of the group of mutant types consisting of "kinase dead" mutant type, hyperactive mutant type, specificity determining mutant type and double mutant type, as disclosed above, are used for method according to the invention. Preferably, the method according to the invention is carried out with at least the "kinase dead" mutant type (in other words cells expressing this mutant type) and the hyperactive mutant type, more preferably combined with cell expressing the specificity determining mutant type. In a further preferred embodiment of the method of the present invention the method uses wt Src family kinase (cells expressing a wt Src family kinase) as a control. Another control may be cells not being induced by an inductor, e.g. tetracycline.

One preferable feature of the method according to the invention is the comparison of the phenotype change of the afore-mentioned Src family kinase mutant types upon addition of a test compound.

In a preferred embodiment at least one, preferably two, three or four of the aforementioned characteristics are detectable by selecting at least one, preferably two, three or four or even more nucleic acids coding for a mutated Src family kinase from the group consisting of SEQ ID NOs 1 to 4 (Src sequences), SEQ ID NOs 5 to 8 (Yes sequences), SEQ ID NOs 9 to 12 (Fgr sequences), SEQ ID NOs 13 to 16 (Fyn sequences), SEQ ID NOs 17 to 20 (Lck sequences), SEQ ID NOs 21 to 24 (Hck sequences), SEQ ID NOs 25 to 28 (Lyn sequences), SEQ ID NOs 29 to 32 (Blk sequences), and SEQ ID NOs 33 to 36 (Yrk sequences). Thereby, the method of the invention provides results for at least one of the abilities disclosed above. The method of the invention can be carried out with any of the sequences listed above. Preferably the method of the invention is performed with all or a subset of sequences for one of the members of Src kinase family. However, any test compound can be tested for its properties against at least two members of a subset of a Src family kinase (of the aforementioned Src kinase sequences and its mutants) in order to identify and characterise potentially effective test compounds by the method of the invention.

Consequently, in a preferred embodiment the active test compound modulates the activity of a Src family kinase or mutated Src family kinase directly or indirectly. Preferably, this compound inhibits the activity of effect mediated by the Src family kinase or mutated Src family kinase.

"Modulate" the activity and/or the expression of a Src family kinase or a mutated Src family kinase according to the invention means to change, e.g. decrease or increase, inhibit or activate, partially or completely their activity and/or expression.

Change or modulation of the "activity" of a Src family kinase or a mutated Src family relates to any mode of alteration resulting in a change of the phenotype of cells and - according to the invention - must not be understood as a limitation to the "kinase activity" of a Src family kinase or a mutated Src family kinase.

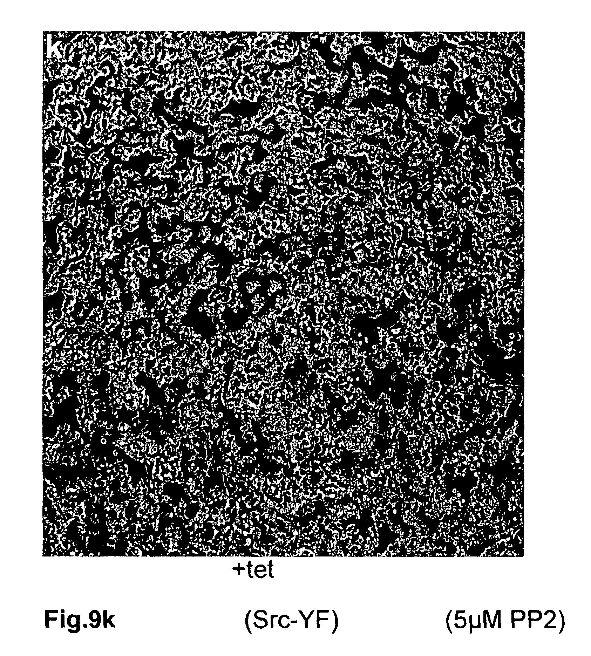

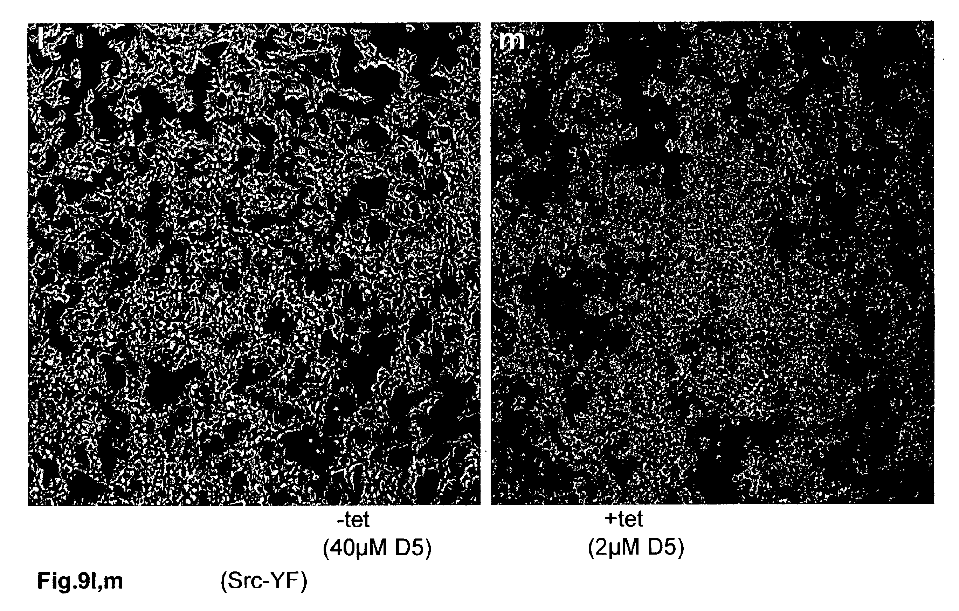

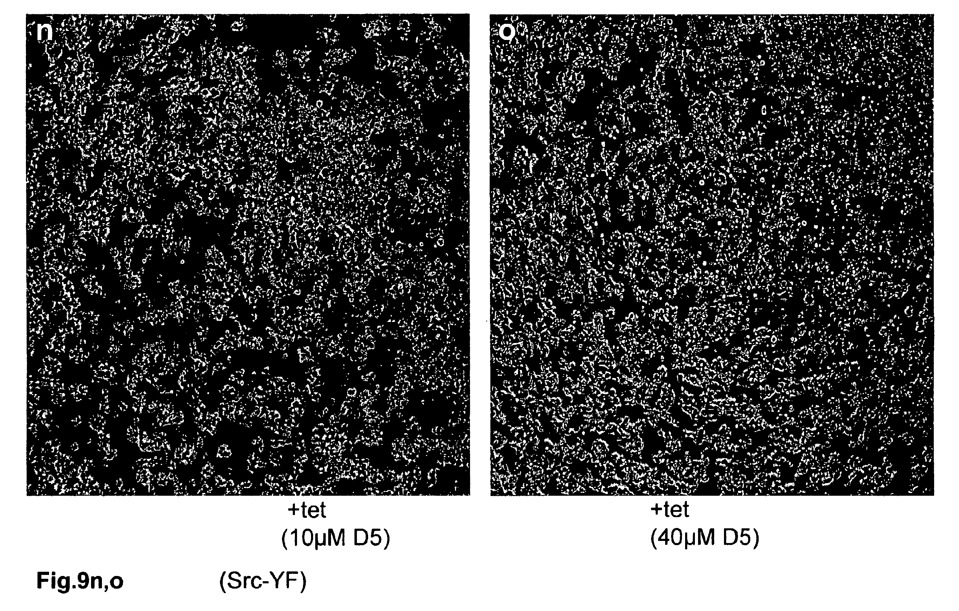

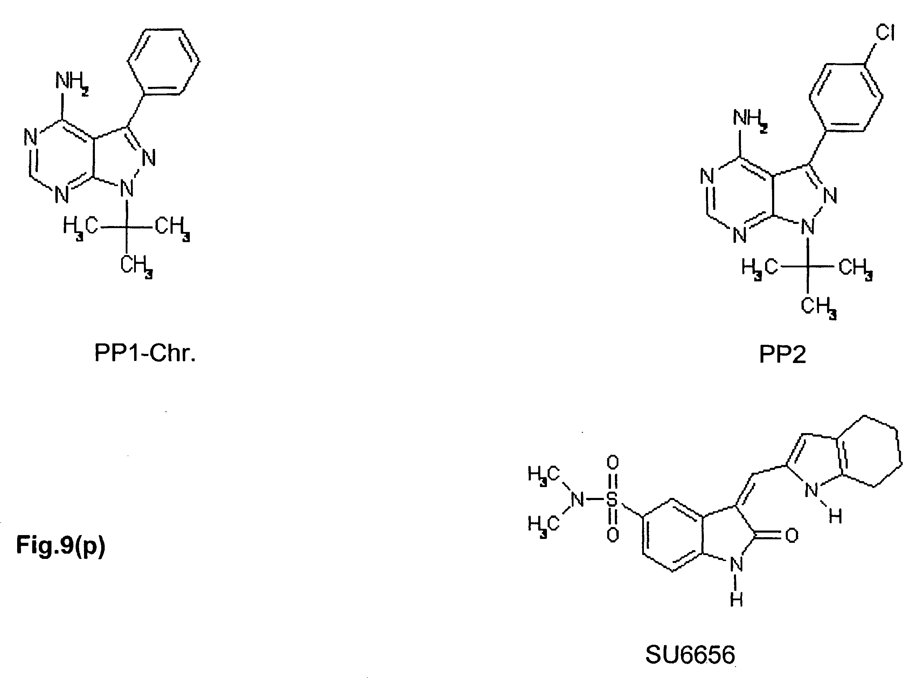

"Direct inhibitors" directly bind to a domain of a Src family kinase or Src family kinase mutant. Those direct inhibitors will most likely bind to the kinase domain, especially in the ATP binding pocket or the substrate binding site. Nevertheless, effects through binding of direct inhibitors to other sites or domains are also possible, since they may exert structural changes on e.g. the substrate binding site or ATP binding site or the entry of the ATP binding site. Direct inhibitors of the Src family kinase family are e.g. PP1-Chr., PP2 and SU6656. These direct inhibitors were also used in several experiments according to the invention. Their chemical structure is shown in Fig. 9p.

"Indirect inhibitors" do not inhibit the Src family kinase or mutated Src family kinase, itself but other components, in particular proteins, involved in the signal transductions pathway or folding or formation of a Src family kinase. For example they inhibit the Hsp90/Hsp70 chaperone system (Stebbins et al., 1997, Cell 89, 239-50; Young et al., 2001, J. Cell Biol. 154, 267-73; Neckers, 2002, Trends Molec. Med. 8, S55-S61), whose function is required for a correct folding of Src family kinase (Whitesell et al., 1994, Proc. Natl. Acad. Sci. USA 91, 8324-8; Xu et al., 1999, Proc. Natl. Acad. Sci. USA 96, 109-14). If a Src family kinase is misfolded due to inhibition of this chaperone machinery, it is quickly destined to proteasomal degradation resulting in a functional knock-down of Src family kinase. Indirect inhibitors for example are the compounds geldanamycin, 17-AAG and radicicol, known to inhibit the (ATPase of) Hsp90, whose chaperone activity is required for a proper folding of Src family kinases (among other Hsp90 clients).

For the method of the invention expression constructs can be generated to enable stable and, if necessary, inducible expression of Src family kinase, and mutated Src family kinases, for example, the Src family kinase mutants Src-KA, Src-TQ, Src-YF and Src-TQ/YF (abbreviations denote single letter amino acid-code, for further information see below) in cells, preferred in mammalian cells.

Therefore, in a preferred embodiment of the invention the nucleic acid which codes for a mutated Src family kinase is selected from the group consisting of SEQ ID NOs 1 to 4.

These sequences relate to mutant derivatives of Src (member of the Src family, see above). More precisely, they relate to the mutant derivatives Src-KA, Src-TQ, Src-YF and Src-TQ/YF of the Src wildtype expression construct that can be generated according to the invention.

In Src-KA lysine298 (K298) (or its analogues of other Src family kinases) of human Src has been mutated to alanine (A). K298 is one of the critical "consensus" amino acids in the ATP binding site of the tyrosine kinase domain of Src. When K298 is mutated to A, ATP binding is abolished and the kinase is completely inactive. The kinase-inactive Src-KA mutant is used in one embodiment of this invention as a control for compound-mode-of-action. Compounds, which have an effect on Src, Src-TQ, Src-YF or Src-TQ/YF expressing cells due to direct inhibition of the kinase activity of Src shall not produce any effect in Src-KA expressing cells. Src-KA or its analogues for other members of the Src family kinases leads to "kinase-dead Src" and makes it - in almost all cases - possible to differentiate between compounds which exert their effect through i) inhibition of Src kinase activity or ii) interaction with Src's non-catalytic domains and functions or, indirectly, affecting Src folding and expression.

Any other mutation of a Src or other members of the Src kinase family, which produces the same effect as mutant Src-KA may be used instead of Src-KA in order to get the same conclusion as with Src-KA. E.g. the method of the invention may be performed with another Src kinase family mutant which forms a "kinase-dead" Src (e.g. another mutation in the ATP binding site) as well.

In Src-TQ the threonine341 (T341) (or its analogues of other Src family kinases) of human Src is mutated to glutamine (Q). T341 is an amino acid at the ATP binding site, which distinguishes Src family kinases and a few other related tyrosine kinases from the remainder of the tyrosine kinases. Therefore, Src-TQ allows to determine the specificity of active, kinase-inhibitory compounds for Src and Src-like kinases. The 3D-structure of Src family kinases and related tyrosine kinases, like Abl, shows that this threonine residue is a specificity determinant of these kinases for the binding of classical, specific inhibitors and is important for inhibitor-kinase-interactions. For instance, the Abl tyrosine kinase inhibitor drug Gleevec® (STI571; Novartis, Basel, Switzerland) does not inhibit Abl-TQ, and the classical Src family kinase inhibitors PP1 and PP2 do not inhibit Src family kinases when they carry the respective TQ mutations. Compounds that bind to the ATP binding site and which exhibit a significant degree of specificity for Src or Src family-related tyrosine kinases will not bind with high affinity to Src-TQ and will consequently not inhibit its kinase with a potency comparable to (wildtype) Src. However, importantly, the Src-TQ mutation (or its analogues for other members of the Src kinase family) does not change the activity of a Src wildtype or hyperactive kinase activity (Src-YF) and, therefore, Src-TQ and Src (as well as the pair Src-YF and Src-TQ/YF) are indistinguishable otherwise and do display the same activity and produce the same phenotype. Thus, the Src-TQ mutant or its analogues for other members of the Src family kinases makes it possible to differentiate between candidate compounds with respect to their binding mode and specificity.

Other mutants (mutations at other position of the wildtype) of members of the Src family may have the same properties and functional characteristics as Src-TQ and may be used according to the invention as well.

In Src-YF the tyrosine530 (Y530) (or its analogues of other Src family kinases), which is located in the carboxy-terminal tail of Src, is mutated to phenylalanine (F) resulting in a hyperactive form of Src. For a better understanding of this effect, it is necessary to know that Src family kinases consist of different domains, as above-mentioned. These domains work together to generate a tightly regulated protein tyrosine kinase: Src is composed of an N-terminal sequence responsible for membrane-attachment followed by a unique region, a kinase domain (Src homology domain 1 (SH1)), connected by a linker sequence to a domain that binds phosphotyrosine-containing sequences (SH2), followed by a domain that binds proline-rich motifs (SH3) and finally a carboxy-terminal tail containing a regulatory tyrosine phosphorylation site (Y530). The kinase activity of Src is mainly controlled by this C-terminal tyrosine: When it is phosphorylated (by another kinase, called Csk) pY530 binds to the Src-SH2 domain leading to a "closed", inactive conformation of Src which is further stabilized by the intramolecular interaction of the SH3 domain with a sequence in the kinase-SH2-linker. In a non-transformed intact cell there is usually a balance between the active and the inactive conformation of Src (which is determined by other kinases and phosphatases responsible for phosphorylation and dephosphorylation of Y530) with the majority of the wildtype Src protein normally existing in the inactive form. When Y530 is mutated to F (as in Y530F), Src-YF cannot be phosphorylated at this position resulting in a constitutively active (hyperactive) Src mutant. Besides displaying a higher kinase activity level, Src-YF is qualitatively comparable with Src with regard to localization and protein-protein interaction, because it does not contain any additional mutations, like v-Src.

Src-YF or its analogues for other members of the Src family kinases leads to a hyperactive Src kinase and its expression induces a characteristic phenotype. Other mutants (mutations at other position of the wildtype) of members of the Src family may have the same properties and functional characteristics as Src-YF and may be used according to the invention as well.

Src-TQ/YF (or its analogues for other members of the Src family kinases) shows hyperactive (Src-YF)-like activity and has a mutation at the ATP-binding site (see also Src-TQ as explained above) in addition to the YF-mutation. In a direct comparison with Src-YF, Src-TQ/YF or its analogues for other members of the Src family kinases allows to determine the specificity of active, kinase-inhibitory compounds for Src and Src-like kinases. Furthermore, it allows to exclude an indirect mode of action of a compound brought about by an inhibitor of a downstream signalling mediator of Src. Other mutants (mutations at other position of the wildtype) of members of the Src kinase family may have the same properties and functional characteristics as Src-TQ/YF and may be used according to the invention as well.

In conclusion, cellular assays according to the invention have several major advantages over in vitro assays based on a purified protein, which are more commonly used for screening purposes, since a test compound will only be effective, if it (i) is able to pass the plasma membrane of cells, which is a prerequisite for a drug (not testable in in vitro assays), (ii) is not chemically modified or degraded by any of the enzymes in the intracellular or extracellular milieu, (iii) is selective enough to specifically bind to the target protein, which represents just one out of >10⁵ different proteins present in a typical human cell and/or if it (iv) produces an unambiguous biological effect, which results from its specific effect on the target. Whereas features (i) - (iii) are features of cell-based assays in general, (iv) is provided for by the negative, positive and specificity-related controls included in the special integrated set-up of the cell-based assay platform of the invention. Furthermore, any compound-induced effect, which is not related to a (direct or indirect) inhibition of Src family kinases, like general non-target specific toxicity or any other striking side effect on a living cell, preferably a living human cell, of this invention can be distinguished from target-specific effects mediated via (direct or indirect) inhibition of Src family kinases by virtue of the inducibility of the target expression in the cells or cell lines of this invention.

Therefore, two levels of the specificity of a compound according to the invention can be distinguished: (a) a specificity with regard to general target-independent side effects and toxicity and (b) a specificity with regard to selectivity in binding to Src family and closely related kinases as opposed to non-selective binding. Particularly, the method according to the invention using mutated Src family kinases allows to distinguish between different modes of action of compounds, including direct/indirect effects on Src family kinases as well as binding mode and specificity of direct Src family kinase inhibitors.

The nucleic acid coding for a kinase of the Src family or a mutant thereof according to the invention can be contained in a vector.

A "vector" according to the invention is an entity which is capable of being introduced or of introducing the proteins and/or nucleic acid into a cell. It is preferred that the Src family kinases or mutated Src family kinases encoded by the introduced nucleic acid of the invention are expressed within the cell upon introduction of the vector. The vector can exist separately in the cell or can be integrated into the genome of the cell according to the invention.

In a preferred embodiment the vector of the present invention comprises plasmids, phagemids, phages, cosmids, artificial mammalian chromosomes, knock-out or knock-in constructs, viruses, in particular adenovirus, vaccinia virus, lentivirus (Chang, L.J. and Gay, E.E. (20001) Curr. Gene Therap. 1:237-251), Herpes simplex virus (HSV-1, Carlezon, W.A. et al. (2000) Crit. Rev. Neurobiol.), baculovirus, retrovirus, adeno-associated-virus (AAV, Carter, P.J. and Samulski, R.J. (2000) J. Mol. Med. 6:17-27), rhinovirus, human immune deficiency virus (HIV), filovirus and engineered versions thereof (see, for example, Cobinger G. P. et al (2001) Nat. Biotechnol. 19:225-30), virosomes, "naked" DNA liposomes, and nucleic acid coated particles, in particular gold spheres. Particularly preferred are viral vectors like adenoviral vectors or retroviral vectors (Lindemann et al. (1997) Mol. Med. 3:466-76 and Springer et al. (1998) Mol. Cell. 2:549-58). Liposomes are usually small unilamellar or multilamellar vesicles made of neutral cationic and/or anionic lipids, for example, by ultrasound treatment of liposomal suspensions. The DNA can, for example, be ionically bound to the surface of the liposomes or internally enclosed in the liposome. Suitable lipid mixtures are known in the art and comprise, for example, cholesterol, phospholipide like, for example, phosphatidylcholin (PC), phosphatidylserin (PS) and the like, DOTMA (1, 2-Dioleyloxpropyl-3-trimethylammoniumbromid) and DPOE (Dioleoylphosphatidylethanolamin) which both have been used on a variety of cell lines.

A large number of specialized prokaryotic vectors have been described (e.g., plasmids such as those capable of replication in E. coli such as, for example, pBR322, ColE1, pSC101, pACYC 184, piVX). Such plasmids are, for example, disclosed by Sambrook, J. et al. (In: Molecular Cloning, A Laboratory Manual, 2nd Ed., Cold Spring Harbor Press, Cold Spring Harbor, N.Y. (1989)). Sambrook, J. et al., herein incorporated by reference, provide a review of the characteristics of mammalian vectors.

For expression in mammalian cells, eukaryotic genes are typically cloned first into a bacterial vector and then subcloned into a vector suitable for eukaryotic expression or, preferably, expressed by a one step process. Thus, although many vectors have been described that are capable of replicating in both prokaryotic and eukaryotic cells, such vectors are designed to express a particular inserted polynucleotide in only one class of cell. Such vectors are illustrated, e.g. by U.S. Pat. No. 4,970,155 (Ikasinski, G. F.), which describes a prokaryotic plasmid that contains eukaryotic transcription and replication elements, such that an inserted polynucleotide can be expressed only in a eukaryotic cell. Similarly, U.S. Pat. No. 5,266,490 (Davis, S. et al.) describes an expression vector that contains an SV40 origin of replication, a eukaryotic transcription unit of the early immediate human cytomegalovirus (CMV) promoter region; and a generic polylinker and an SV40 splice/polyadenylation site. The vector also contains a pBR322 origin of replication and an antibiotic resistance gene under control of a prokaryotic promoter.

Also known are vectors that are capable of expressing an inserted gene in both prokaryotic and eukaryotic cells without any requirement to modify the vector or reclone the inserted gene. He, B. et al. (Gene 164: 75-79 (1995) described expression vectors that direct the synthesis of proteins from a common set of signals in both prokaryotic and eukaryotic cells. To allow transcription from a common promoter the vectors rely upon a phage RNA polymerase. To direct initiation of translation to the same start codon the vectors utilize an internal ribosome entry site from encephalomyocarditis virus that has been modified to include a prokaryotic ribosome-binding site at an appropriate distance upstream from the desired start codon. Mole, S. E. et al. (Nucl. Acids Res. 15: 9090 (1987) describe pSEMCatR1, a prokaryotic-eukaryotic shuttle vector compatible with pUR and lambda gt11 expression systems. Kaehler, R. et al. (DD 206791) discuss a hybrid expression vector which contains prokaryotic and eukaryotic control units directly connected to the sequence being expressed. Alting-Mees, M. A. (Strategies 5:58-61 (1992) describes the Stratagene(R) vector pBK-CMV, which is intended to be suitable for gene expression in both prokaryotes and eukaryotes. However, genes cloned into pBK-CMV are expressed only inefficiently in eukaryotic cells.

"Expression vectors" within the meaning of the invention are vectors that are capable of mediating the expression of a nucleic acid. Preferred expression vectors according to the invention are e.g. pcDNA4/TO, PcDNA3-derivatives, pcDNA5/TO. Further examples are pGene/V5-His, pVg RXR and pIND-based inducible expression vectors.

For the expression constructs according to the invention, the pcDNA4/TO (Invitrogen, Carlsbad CA, USA) can be chosen as a preferred expression vector. This vector carries a tet-operator sequence enabling tetracycline (tet)-inducible expression in cells, preferably mammalian cells, of the desired transgene, whose nucleic acid, cDNA, has been cloned into its polylinker. The vector also contains a zeocin resistance gene which allows selection for stably transfected cells with the antibiotic zeocin (zeo). In various experiments of the invention, the human Src cDNA was cloned (directed) into the EcoRI restriction site of the vector's polylinker.In a preferred embodiment the expression of the nucleic acid of the expression construct is induced by adding tetracycline. Preferably, the induced expression causes an overexpression of the nucleic acid in the transfected cells.

In a preferred embodiment, the cell according to the invention is a cell line.

In another preferred embodiment the cell is an eukaryotic cell, preferably a vertebrate cell, more preferably a mammalian cell, most preferably a human cell.

In a further preferred embodiment the cell or cell line to be used in an assay system according to the invention is (a) an immortalised cell/cell line or (b) a tumor cell/cell line or (c) a lymphoid cell/cell line. For example, a cell or cell line to be used is a 32 Dar BAF cell (lymphoid); HL-60, K-562 or other leukaemia cells/cell lines; HCT116, HCT-15, HT-29 or other colon carcinoma cells/cell lines; SKOV-3, OVCAR-3, OVCAR-4, OVCAR-5, OVCAR-8 or other ovarian carcinoma cells/cell lines or epithelial cell/cell lines like HeLa, HEK 293.

In one particularly preferred embodiment the expression constructs were transfected in the "293/TREx" cell line. This cell line is derived from the human embryonic kidney epithelial cell line "HEK293", which is widely used in many laboratories. Its epithelial nature, which it shares with most (solid) cancers makes it an appropriate and relevant cellular system in the area of oncology. 293/TREx cells are preferred, because they are cells which stably express a so-called tet-repressor protein, which is able to block expression from a tet-operator-containing expression plasmid, e.g. pcDNA4/TO. When tet is added to the cells, it binds to the repressor, which changes the repressor conformation resulting in its release from the tet-operator and onset of expression, typically overexpression. Those cells can be cultivated in the presence of the antibiotic blasticidin in order to keep the selection pressure on repressor expression.

In general according to the invention, any system which allows to express or more preferably to overexpress the target protein(s), e.g kinases of the Src family or their mutants, preferably in an inducible form, is preferably employed to perform the method according to the invention. As disclosed above the induction of the system is generally achieved by promoter/repressor combinations of the expression vector. While there are a number of mechanisms known in the art to switch on gene expression by changing the environment conditions (e.g. heat shock induction), which may be employed for the present invention, it is preferred to induce the system by addition of substances which, e.g. deactivate the repression of gene transcription or induce expression by other mechanisms, like tetracycline, mifepristone, ponasteroneA or muristeroneA.

According to another preferred embodiment the phenotype change is determined by qualitative and/or quantitative analysis.

In a particularly preferred embodiment the phenotype change is determined by assessing one or more parameters of the cellular phenotype, e.g. the viability, cell death rate, growth properties, proliferation, upon contacting a compound to be tested with the cells or cell lines according to a method of the invention. The phenotype parameter(s) are measured and compared with the phenotype (e.g. viability or any other parameter) of said cell before addition of the compound, whereby a difference in the phenotype (e.g. viability or any other other parameter) of said cell indicates that the compound to be tested modulates the activity of a Src family kinase.

A qualitative analysis as well as the determination of changes in the cellular phenotype, e.g. viability, according to the invention can be carried out by visual inspection (eventually using a microscope) of the cell. Thereby, particularly the cell morphology, growth characteristics and/or distribution of the cells with and without addition of the test compound are scrutinized. This allows to get qualitative and semi-quantitative information about the effects of test compounds based on a biological readout (phenotype), which results from the activity (or e.g. suppression of activity induced by a test compound) of Src family kinase and mutated Src family kinase in a transfected cell (as compared to the phenotype without addition of the test compound). Control experiments using transfected cells, which are not triggered to overexpress the Src kinases or their mutants, are preferably employed (either with addition of the test compound or without the addition of a test compound) to avoid any artefacts.

A quantitative analysis as well as the determination of changes in the phenotype according to the invention can be measured by several methods and various parameters. The complex phenotypes that can be observed upon Src family kinase/ Src family kinase mutant induction according to the invention, may include changes in e.g. 1) viability, 2) proliferation, 3) cell death and 4) morphology/ growth properties (evenly distributed, aggregating). These changes can be quantitatively determined by using reference measurements which allow to calculate reference values.

A number of assays or systems for the measurement of these parameters are known in the art. Some of these measurements are specified by the web pages www.promega.com/tbs/cellprolif.htm or www.biochem.roche.com. In general, the final readouts can be colorimetric- (i.e. absorbance-), luminescence- or fluorescence-based.

Some of these known methods are mentioned in the following:

### Cell Viability methods

Most viability assays are based on the measurement of either
a) Metabolic activity or
b) Cell membrane integrity.

a) Metabolic activity is usually detected in cells via incubation with tetrazolium salts, e.g. MTT, XTT, WST-1, MTS, which are cleaved into a water-soluble, colored formazan product by metabolically active cells. Kits to perform such assays are available form companies like Roche (Basel, Switzerland), Promega (Madison, WI, USA) and many others. As an example, in the "CellTiter96 Aqueous One Solution Cell Proliferation Assay (MTS)" from Promega, the colored formazan product of the bioreduced tetrazolium compound MTS (3-(4,5-dimethylthiazol-2-yl)-5-(3-carboxymethoxyphenyl)-2-(4-sulfophenyl)-2H-tetrazolium) can be quantitatively detected at the wavelength of 490nm (absorbance) in a plate reader.
   Another method is based on the ability of viable cells to convert a redox dye (resazurin) into a fluorescent end product (resorufin). Viable cells retain the ability to reduce resazurin to resorufin, whereas nonviable/ dead cells rapidly loose metabolic capacity, do not reduce the indicator dye, and thus do not generate a fluorescent signal. This method is employed by the "CellTiter-Blue™ Assay" from Promega. Results obtained by the above described assays are further described in the examples.
   The ATP status of cells can also be analyzed by homogenous assay types and provides for a quantitative measure of cellular energy capacity and thus viability. Several companies including Perkin Elmer (Wellesley, MA, USA) or Promega provide kits for such assays. Results obtained by using the "CellTiter-Glo Luminescent Cell Viability Assay" (Promega) are discussed in the examples.
b) In healthy cells with uncompromised plasmamembrane integrity dyes such as trypan blue are excluded while dead or dying/damaged cells are stained and can be counted. Leaky cytoplasmic membranes as a measure for loss of cell viability can also be detected by measuring the release of molecules, like lactate dehydrogenase (LDH) or ⁵¹Cr from dying cells into the culture medium.

### Cell Proliferation Methods

While the cell viability methods mentioned under a) above can to some extent also be used to determine cell proliferation, the most prominent and accurate parameter for analyzing cell proliferation is the measurement of DNA synthesis as a specific marker for replication, which in turn is a prerequisite for cell proliferation. Labeled DNA precursors, like ³H-thymidine or 5-bromo-2'-deoxyridine (BrdU), are added to the cells and their incorporation into genomic DNA is quantified following incubation and sample preparation. The amount of incorporated label is proportional to the rate of cell division (proliferation). ³H-thymidine, BrdU and assay kits using these markers are available from many companies like Roche and BD Biosciences (Franklin Lanes, NJ, USA).

Indirect measurements of proliferation include Western-blot-or ELISA-based protein analysis of molecules -by quantity or activity- which regulate the cell cycle, like cyclin-dependent kinases.

### Cell Death

There are two major ways a cell can die: Necrosis or apoptosis (i.e. programmed cell death). For both processes, and to distinguish these two processes, there is a number of various assay types available, including DNA-fragmentation- (so-called "TUNEL"-) assays, westem-blot-or ELISA-based analysis of active caspases, which are the executioner proteases of the apoptosis pathways, or their cleavage products (like PARP), Annexin V staining of translocated, outer membrane-exposed phosphatidylserine, time-course-dependent ATP determinations, determination of the proteolytic activity of one or more caspases and intracellular staining with DNA-binding dyes. Many companies provide compounds or kits for such assays, including Roche (Basel, Switzerland), Alexis Corp. (Lausen, Switzerland), Limitech (Montreal, CA), Promega, Perkin Elmer, Molecular Probes and others.

### Cell morphology and growth characteristics

Those are difficult-to-quantify parameters. This aspect of the phenotype changes is obvious when examined by eye/microscope. Nevertheless, the evaluation of phenotype changes by eye can suffer from being i) inherently subjective, ii) qualitative or semi-quantitative instead of quantitative, iii) unsuitable for medium- or high throughput screening of compounds.

A quantitative evaluation can be realized by use of appropriate live cell image analysis software applications, as is e.g. available from Visitron System GmbH (Puchheim, Germany), which use special algorithms to detect and quantify parameters associated with and quantitatively reflecting individual cell morphology and overall growth property changes. To meet the requirements of a high throughput screening, a person skilled in the art can combine a suitable image analysis program with a fully motorized microscope; such a setup can automatically record and analyze images of each well of a microtiter plate transforming them into quantitative parameters. Special features, like an incubator-equipped microscope stage, which controls temperature and CO₂ level, allows the long-term and kinetic analysis of live cell phenotypic changes.

The invention also relates to a compound or a pharmaceutically acceptable derivative thereof identified, selected and/or characterized by a method of the invention. Preferably, this compound is obtained or obtainable by a method of the invention. Preferably, this compound is suitable for the treatment of diseases, which are at least in part caused by a Src family kinase.

A "pharmaceutically acceptable derivative" of a compound according to the invention relates to any non-toxic salt, ester, salt of an ester or other derivative of a compound of this invention that, upon administration to a recipient, is capable of providing, either directly or indirectly, a compound of this invention or an inhibitorily active metabolite or residue thereof. Pharmaceutically acceptable salts of the compounds of this invention include those derived from pharmaceutically acceptable inorganic and organic acids and bases.

"Diseases, which are at least in part caused by a Src family kinase" according to the invention mean any diseases or other deleterious condition in which at least one Src family kinase is known or will be known to play a role. Accordingly, the compounds of the invention are useful for treating diseases or conditions that are known to be affected by the activity of one or more Src-family kinases.

The invention also relates to the use of a compound or a pharmaceutically acceptable derivative thereof according to the invention for the production of a medicament for the treatment of diseases, which are at least in part caused by a Src family kinase, preferably cancer, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, Paget's disease, asthma, ischemic or reperfusion injury, allergic disease, atopic dermatitis or allergic rhinitis.

Furthermore, the invention also relates to a pharmaceutical composition for the treatment of diseases (or for manufacturing drugs for the treatment of diseases), which are at least in part caused by a Src family kinase comprising a compound or a pharmaceutically acceptable derivative thereof identified, selected and/or characterized by the method of the invention and a pharmaceutically acceptable carrier, adjuvant or vehicle. Preferably, these diseases include cancer, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, Paget's disease, asthma, ischemic or reperfusion injury, allergic disease, atopic dermatitis or allergic rhinitis.

Preferably, the compounds and/or pharmaceutical compositions according to the invention or identified by methods according to the invention are administered for the treatment of various forms of non-solid and solid cancers. Examples for cancers which can be treated by the compounds and/or pharmaceutical compositions according to the invention are Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer and/or skin, e.g. melanoma or non-melanoma skin cancer, including basal cell and squamous cell carcinomas.

A "pharmaceutically acceptable carrier, adjuvant, or vehicle" according to the invention refers to a non-toxic carrier, adjuvant or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of the present invention may be administered orally, parenterally, by inhalation spray, topically, rectally, nasally, buccally, vaginally or via an implanted reservoir.

The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutically acceptable compositions of this invention may be orally administered in any orally acceptable dosage form including, but not limited to, capsules, tablets, aqueous suspensions or solutions. In the case of tablets for oral use, carriers commonly used include lactose and com starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavouring or colouring agents may also be added.

Alternatively, the pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutically acceptable compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

The pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutically acceptable compositions of this invention are formulated for oral administration.

The amount of the compounds of the present invention that may be combined with the carrier materials to produce a composition in a single dosage form will vary depending upon the host treated, the particular mode of administration. Preferably, the compositions should be formulated so that a dosage of between 0.01-100 mg/kg body weight/day of the inhibitor can be administered to a patient receiving these compositions. Preferred dosages range from 0.1 - 5 mg/kg body weight/day, even further preferred dosages from 1 - 5 mg/kg body weight/day.

It has to be noted that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

The following figures and examples are thought to illustrate the invention and should not be construed to limit the scope of the invention thereon. All references cited by the disclosure of the present application are hereby incorporated in their entirety by reference.

### Figures

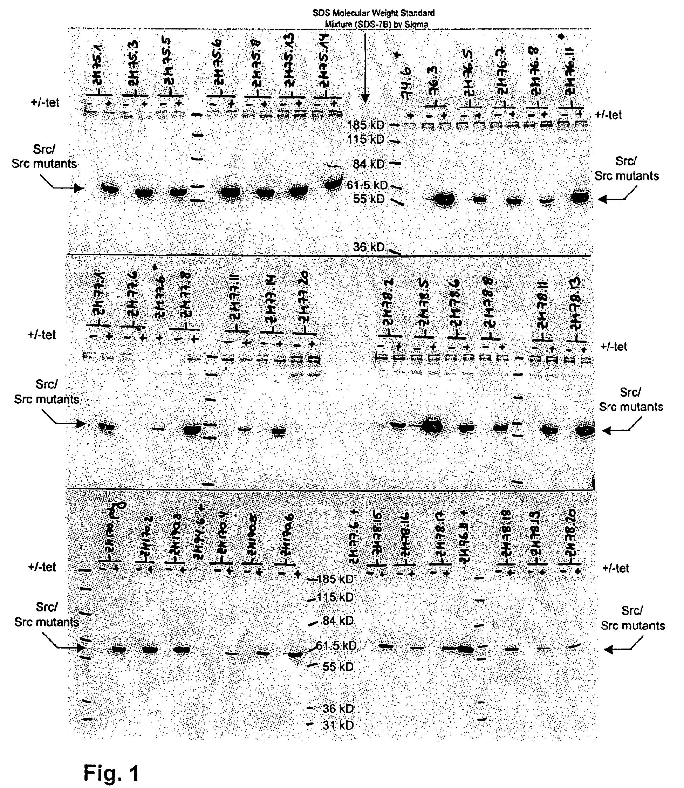

**Fig. 1** shows the inducible expression levels of different cell lines. More exact, it shows stable inducible expression of **Src (ZM75), Src-KA (ZM76), Src-TQ (ZM78), Src-YF (ZM77) and Src-TQ/YF (ZM170)** in some of the 293/TREx cell lines. (ZM74.6: control cell line, does not express any Src.). The cells were incubated with 2µg/ml tetracycline or not (+/-tet) for 24h prior to lysis. Lysates were subjected to SDS polyacrylamide gel electrophoresis and anti-Src Western blotting. The Src proteins are clearly expressed upon tet induction, whereas there is no or very little Src expression without tet (the little expression may be an artifact due to a minor proportion of +tet-lysates, unintentionally loaded into the neighbouring -tet lanes).
**Fig. 2 a-f** shows phenotypes of **Src** expressing **ZM75.poly** cell line incubated in serum-free medium for 1 day (a,b), 2 days (c, d) and 3 days (e, f) in the absence (a, c, e) or presence (b, d, f) of tet. Phenotype changes upon Src expression induction: a→b; c→d; e→f.
**Fig. 3 a, b** shows phenotypes of **Src** expressing **ZM75.7** cell line incubated in serum-free medium for 2 days without (a) or with (b) tet.
**Fig. 4 a-d** shows phenotypes of **Src-KA** expressing **ZM76.poly** cell line incubated in serum-containing medium incl. EGF (100ng/ml) for 1 day (a, b) and 2 days (c, d) without (a, c) or with tet (b, d). Phenotype changes upon Src expression induction: a→b; c→d.
**Fig. 5 a, b** shows phenotypes of **Src-KA** expressing **ZM76.3** cell line incubated in serum-containing medium incl. EGF (100ng/ml) for 2days without (a) or with (b) tet.
**Fig. 6 a, b** shows phenotypes of **Src-YF** expressing **ZM77.poly** cell line incubated in serum-containing medium for 1 day without (a) or with (b) tet.
**Fig. 7 a, b** shows phenotypes of **Src-TO** expressing **ZM78.poly** cell line incubated in serum-free medium for 2 days without (a) or with (b) tet.
**Fig. 8 a, b** shows phenotypes of **Src-TQ/YF** expressing **ZM170.21** cell line incubated in serum-containing medium for 10h without (a) or with (b) tet.
**Fig. 9 a-d, g-o** shows phenotypes of **Src-YF** expressing **ZM77.poly** cell line incubated in serum-containing medium for 1 day without (a, c, g, i, l) or with (b, d, h, j, k, m, n, o) tet and with the following drugs: 10µM PP1-Chr. (c, d), 5µM SU6656 (g, h), 1µM PP2 (j), 5µM PP2 (i, k), 2µM D5 (m), 10µM D5 (n), 40µM D5 (1, o) or vehicle (0.2% DMSO; "-" (a, b)) as indicated on the images.

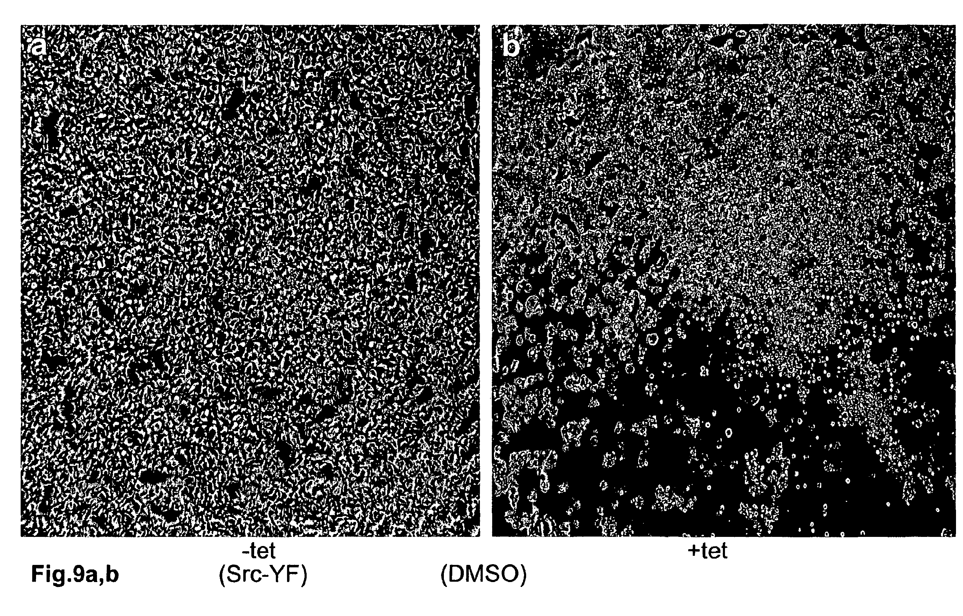

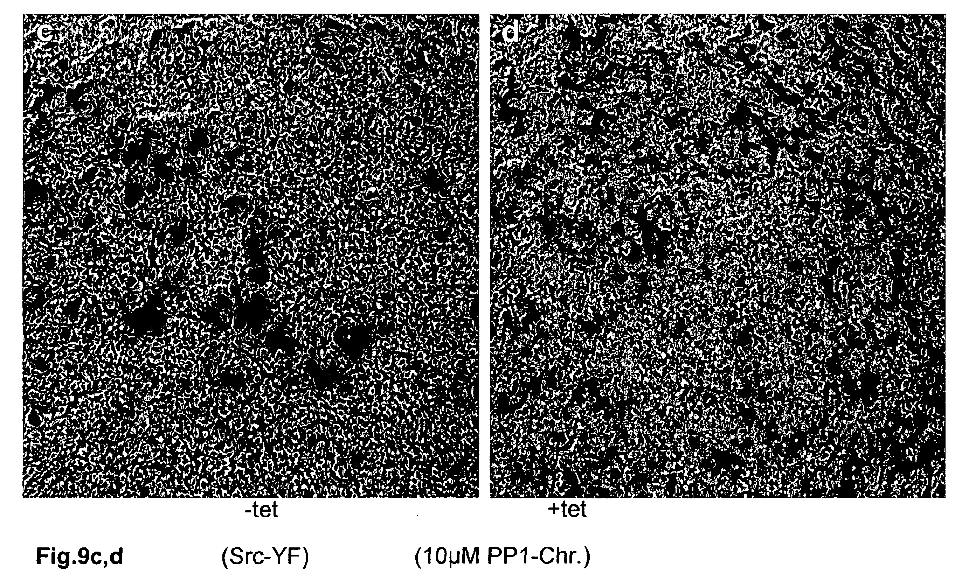

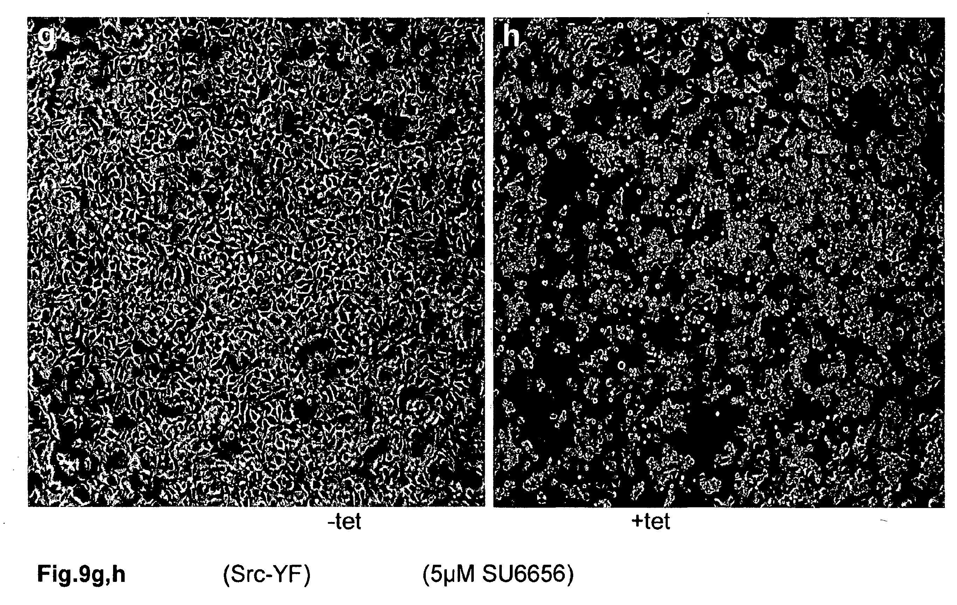

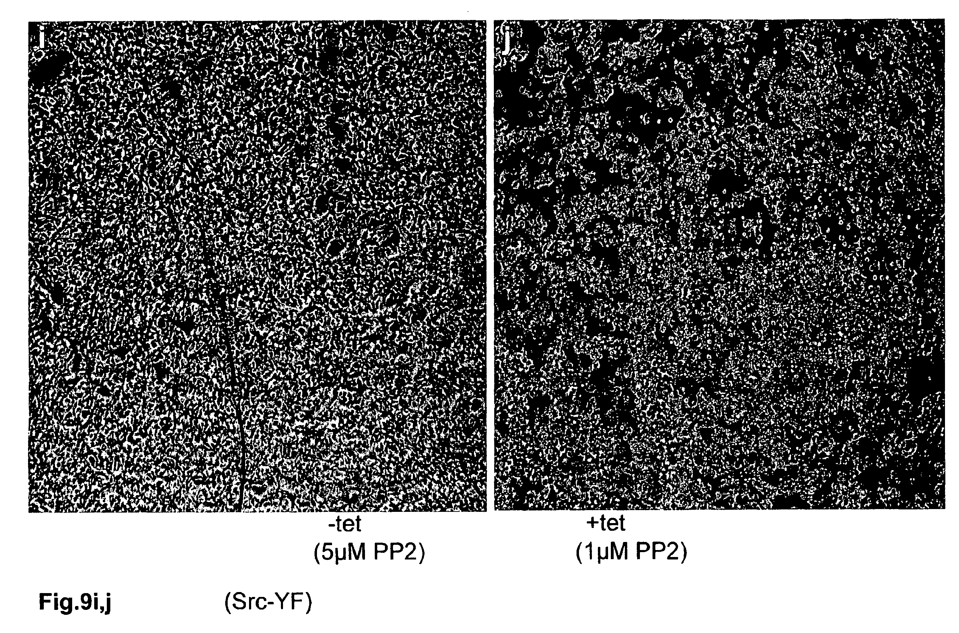

**Fig. 9 p** shows the structure of Src-specific inhibitors used for proof-of-principle experiments.

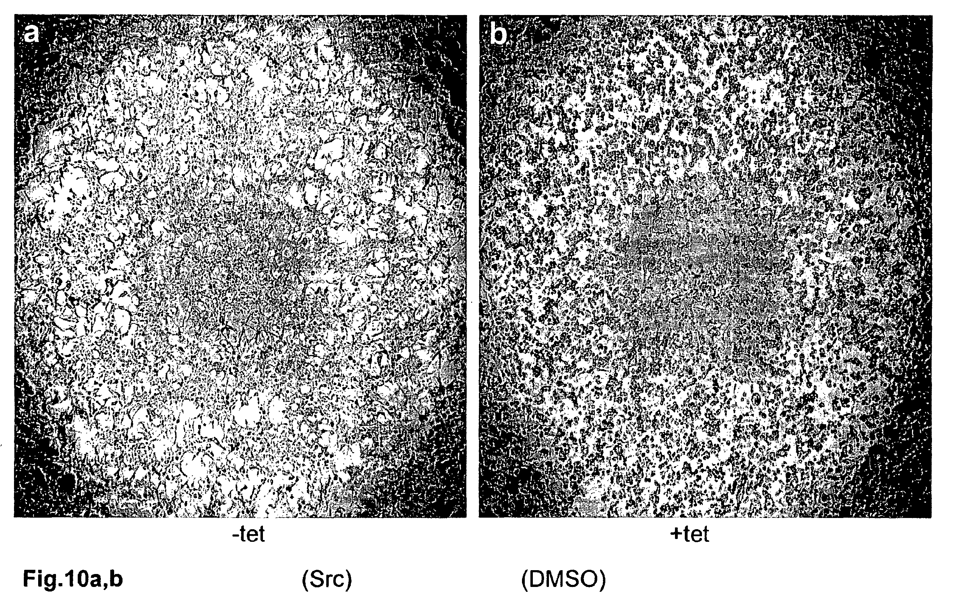

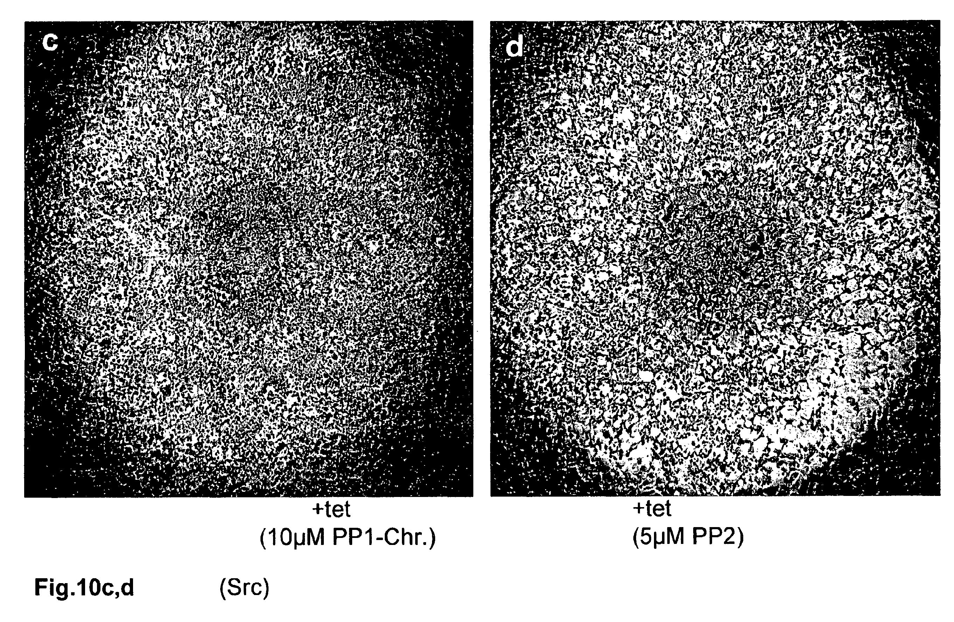

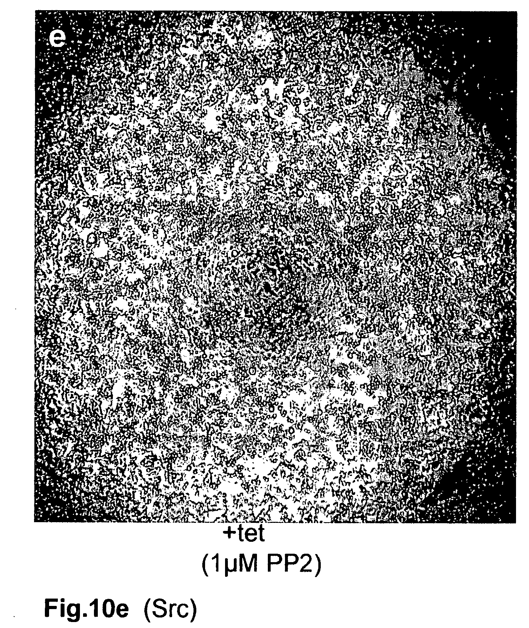

**Fig. 10 a-e** shows phenotypes of **Src** expressing **ZM75.7** cell line incubated in serum-free medium for 2 days without (a) or with (b-e) tet and with 10µM PP1-Chr. (c), 5µM PP2 (d), 1µm PP2 (e) or vehicle (0.2% DMSO; "-" (a, b)).

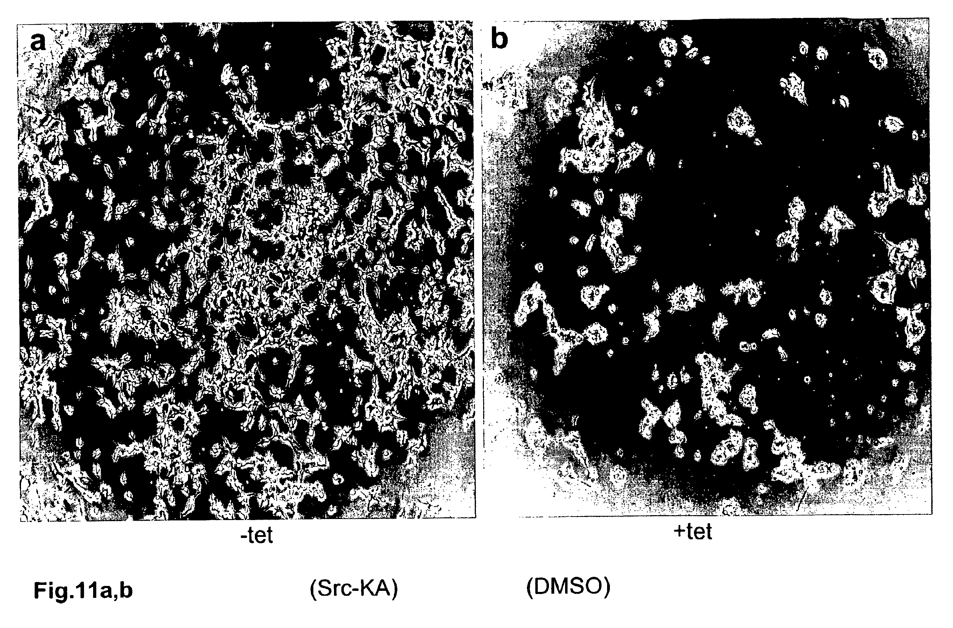

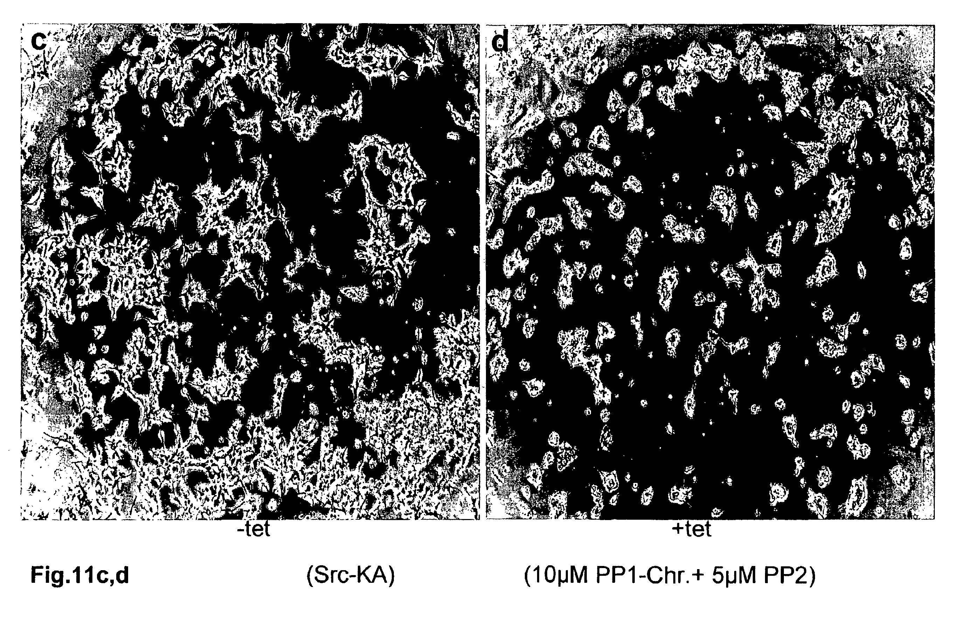

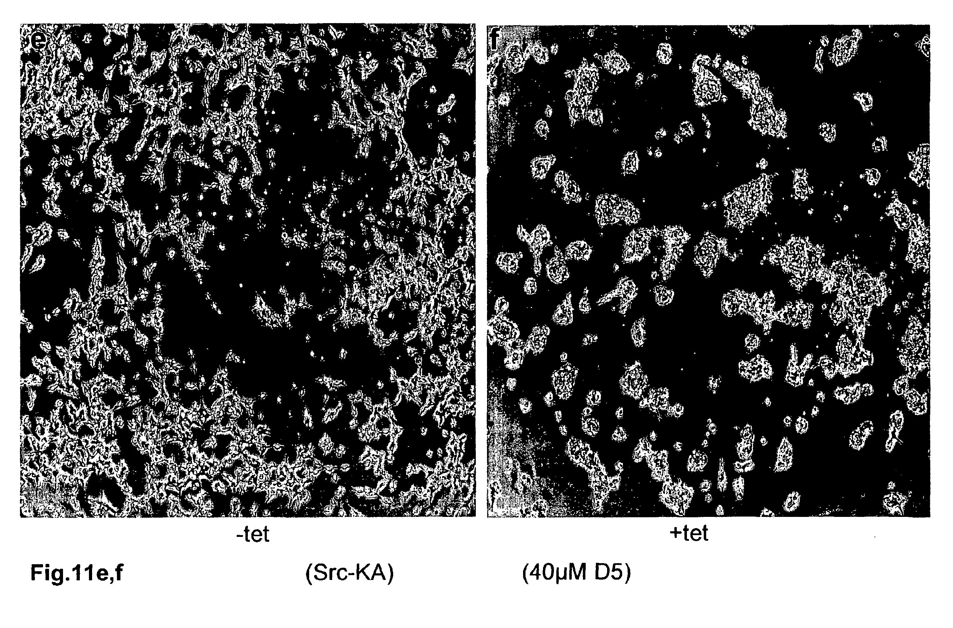

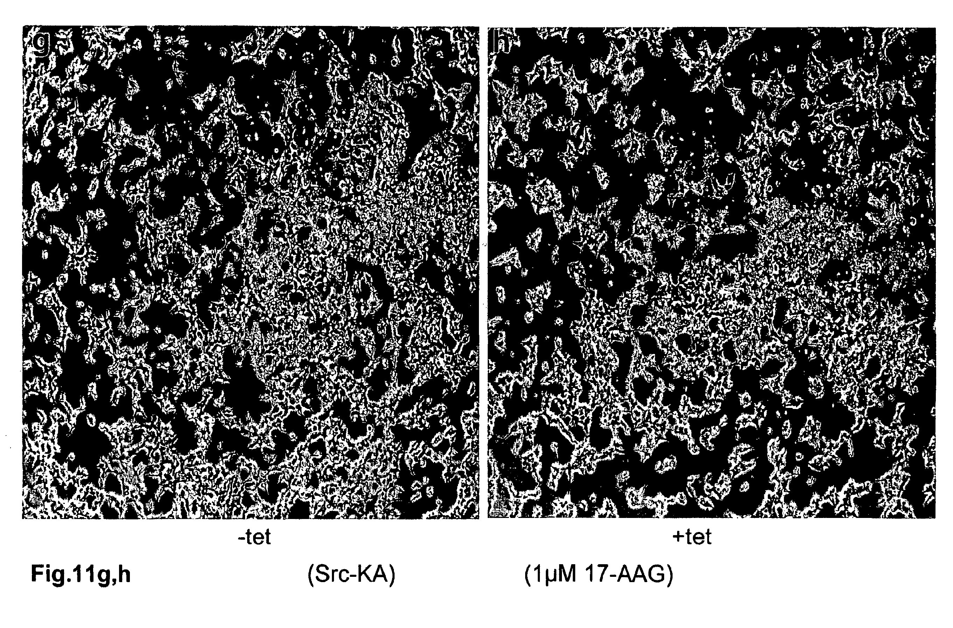

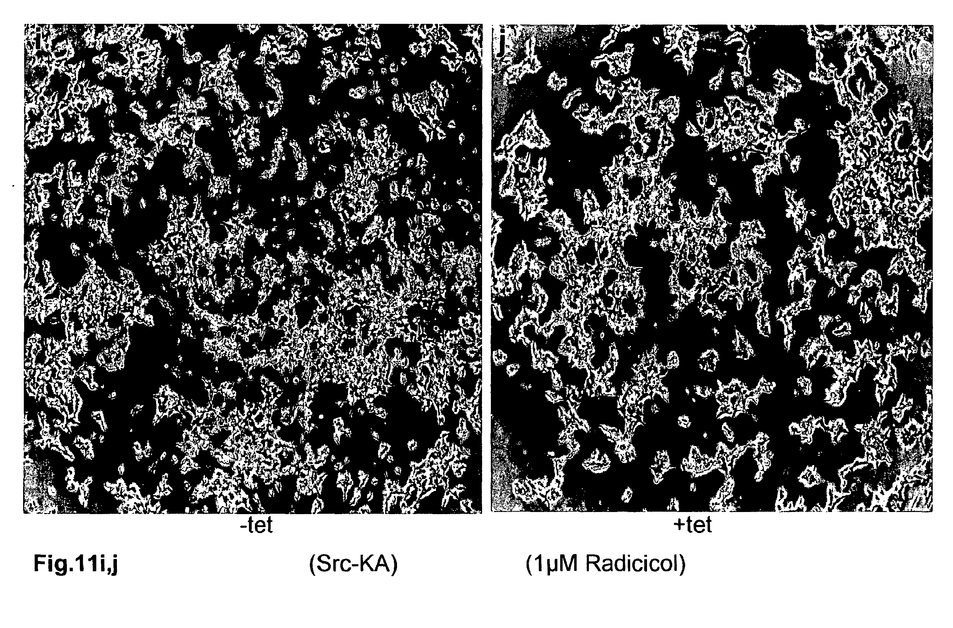

**Fig. 11 a-j** shows phenotypes of **Src-KA** expressing **ZM76.3** cell line incubated in serum-containing medium +EGF (100ng/ml) for 1.5 days without (a, c, e, g, i) or with (b, d, f, h, j) tet and with the following drugs: 10µM PP1-Chr.+ 5µM PP2 (c, d), 40µM D5 (e, f), 1µM 17-AAG (g, h), 1µM Radicicol (i, j) or vehicle (0.2% DMSO; "-" (a, b)).

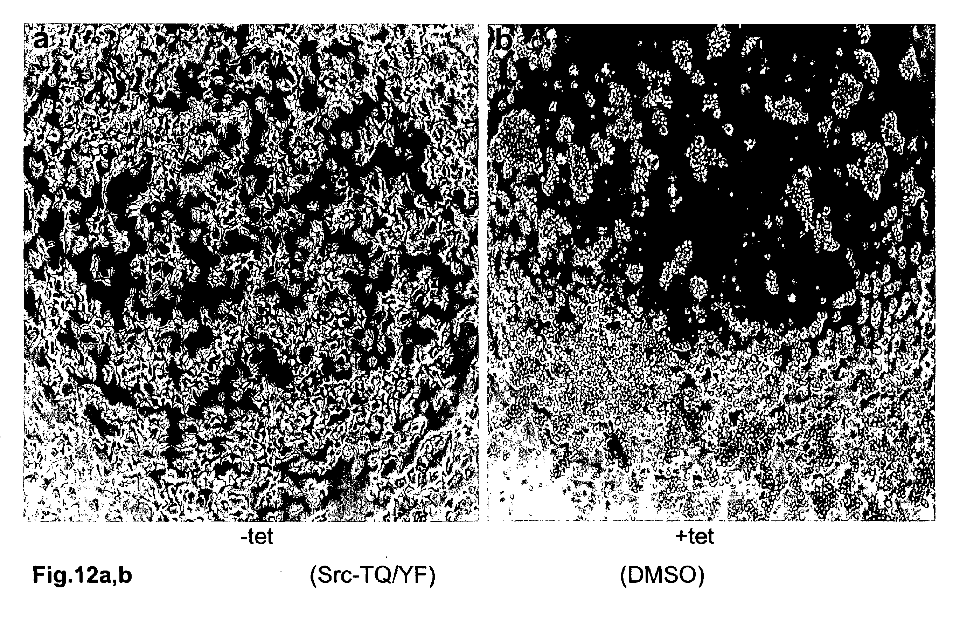

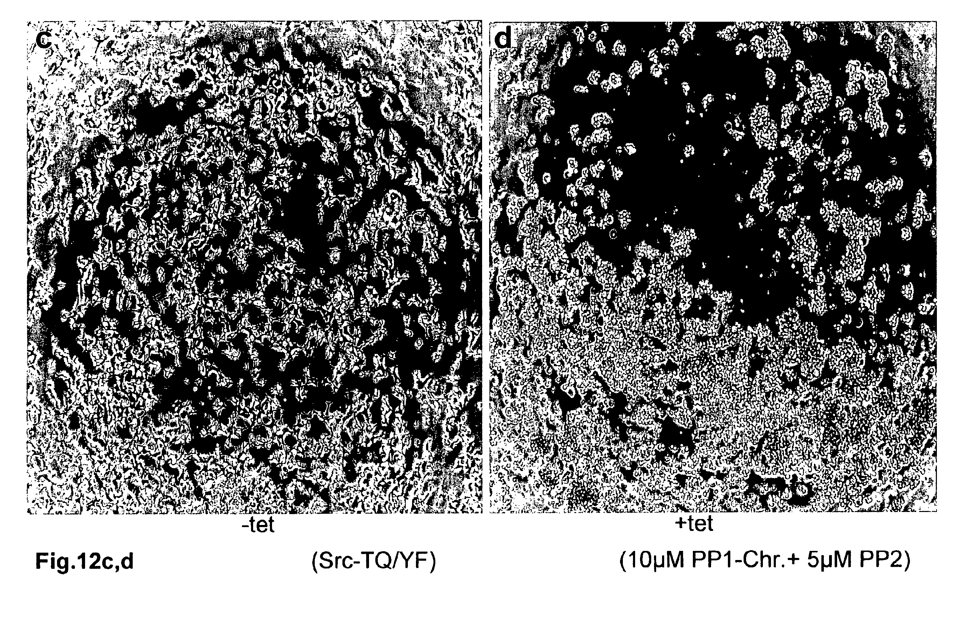

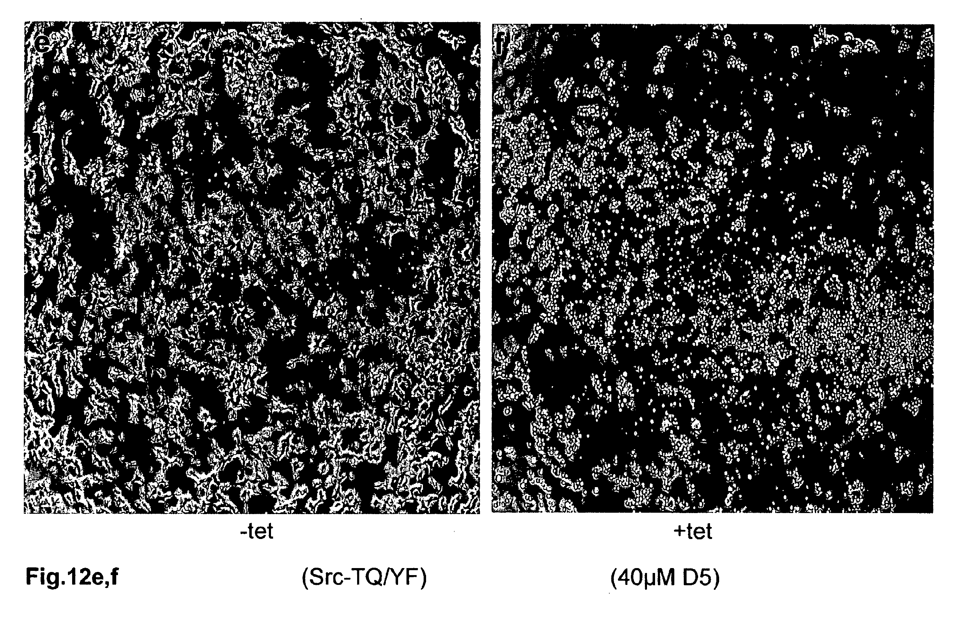

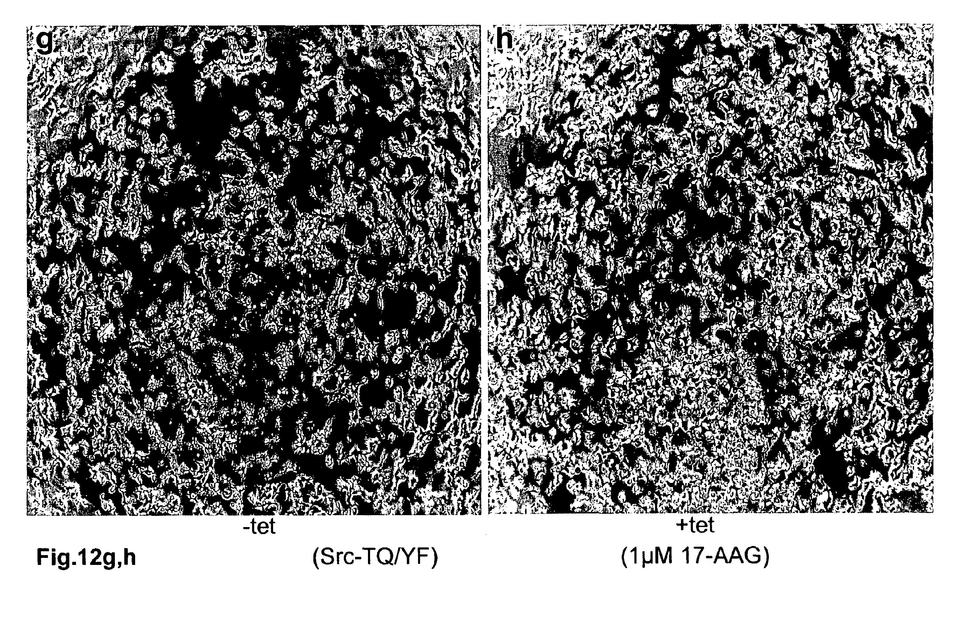

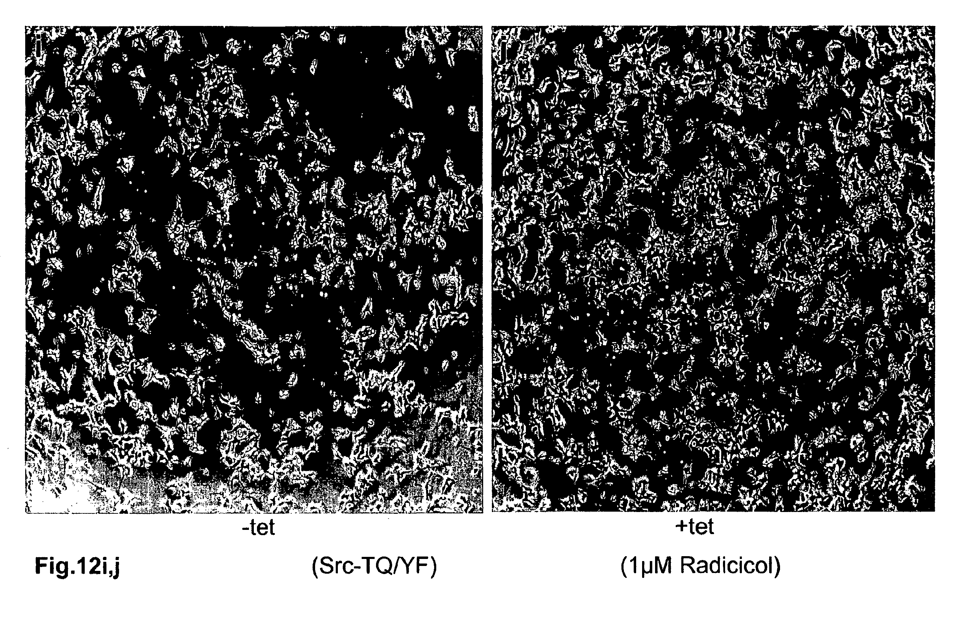

**Fig. 12 a-j** shows phenotypes of **Src-TO/YF** expressing **ZM170.21** cell line incubated in serum-containing medium for 10h without (a, c, e, g, i) or with (b, d, f, h, j) tet and with the following drugs: 10µM PP1-Chr.+ 5µM PP2 (c, d), 40µM D5 (e, f), 1µM 17-AAG (g, h), 1µM Radicicol (i, j) or vehicle (0.2% DMSO; "-" (a, b)).

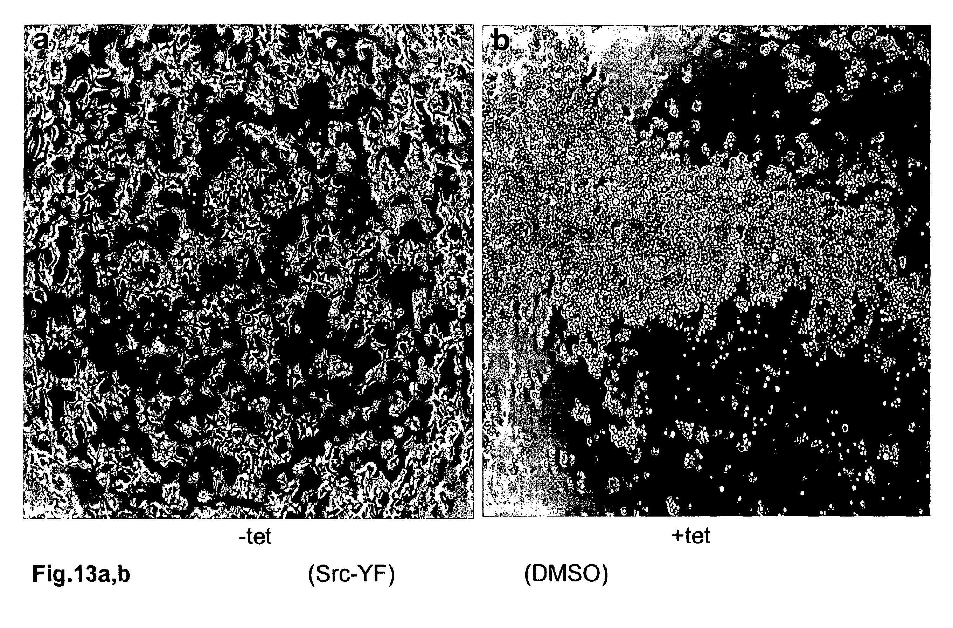

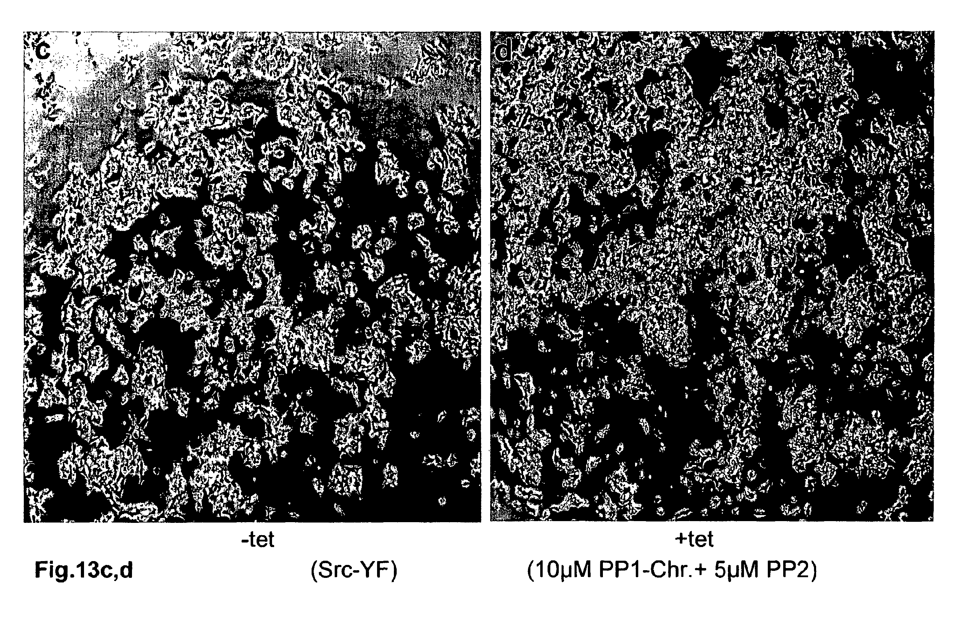

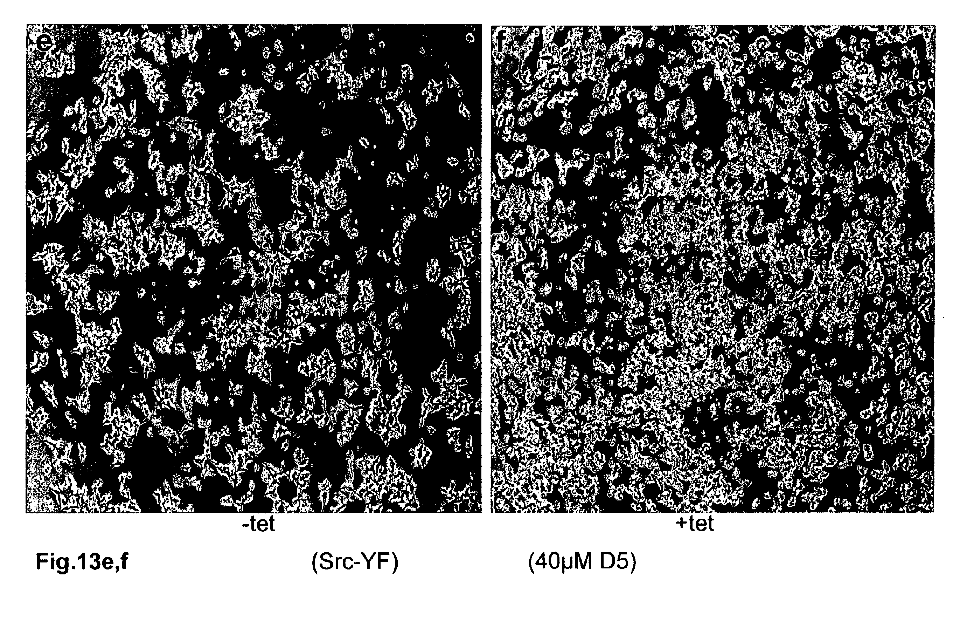

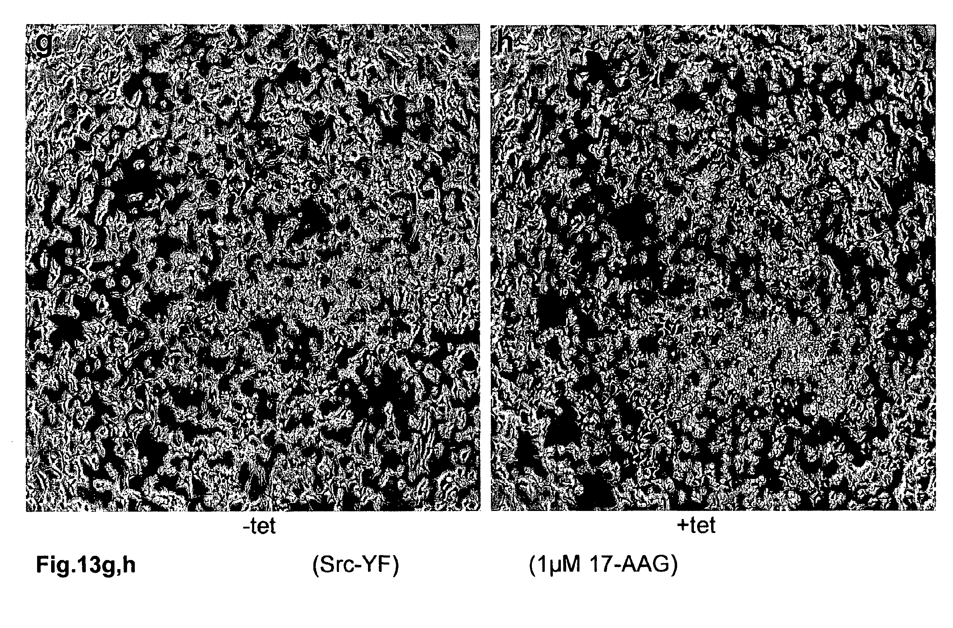

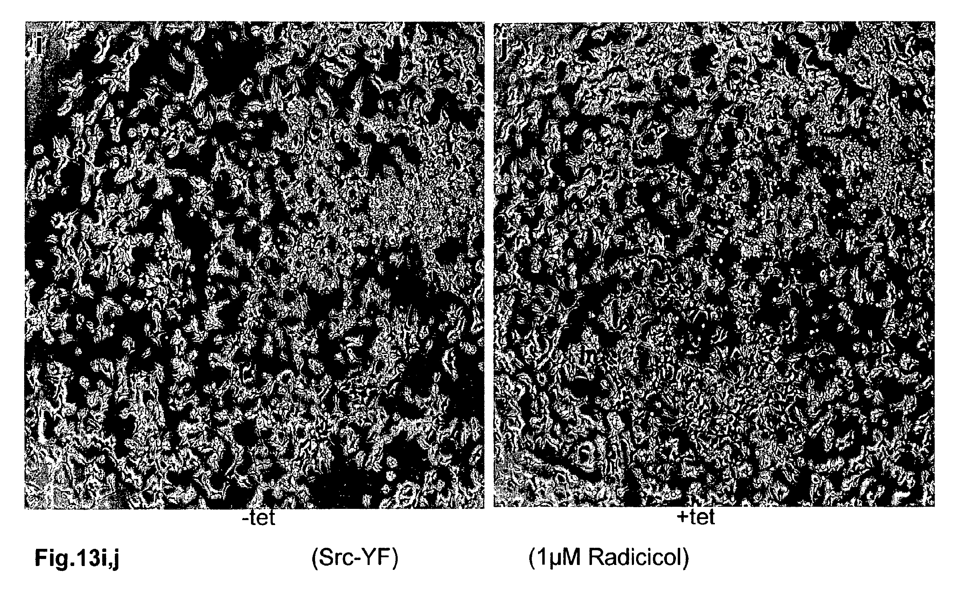

**Fig. 13 a-j** shows phenotypes of **Src-YF** expressing **ZM77.8** cell line incubated in serum-containing medium for 10h without (a, c, e, g, i) or with (b, d, f, h, j) tet and with the following drugs: 10µM PP1-Chr.+ 5µM PP2 (c, d), 40µM D5 (e, f), 1µM 17-AAG (g, h), 1µM Radicicol (i, j) or vehicle (0.2% DMSO; "-" (a, b)).

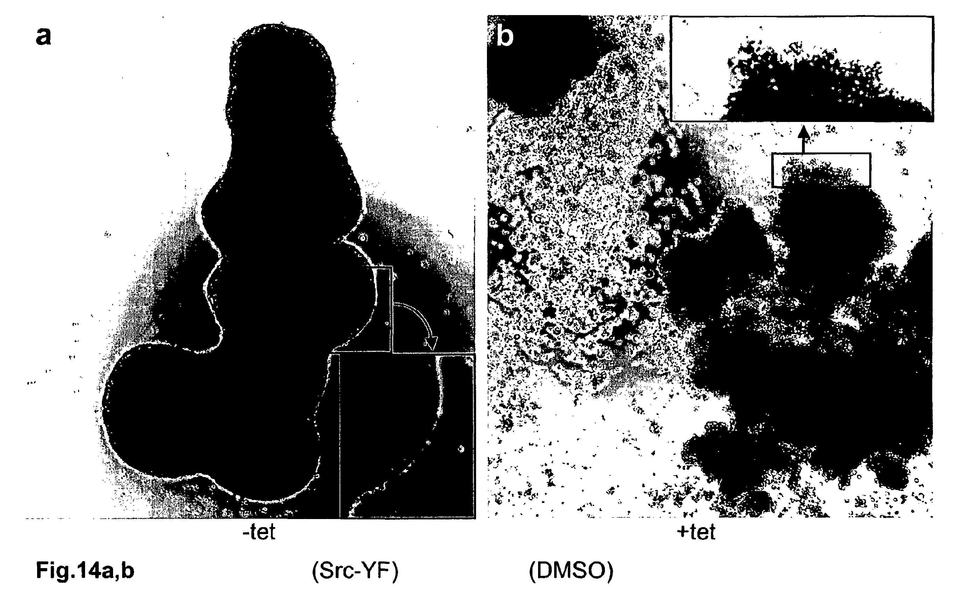

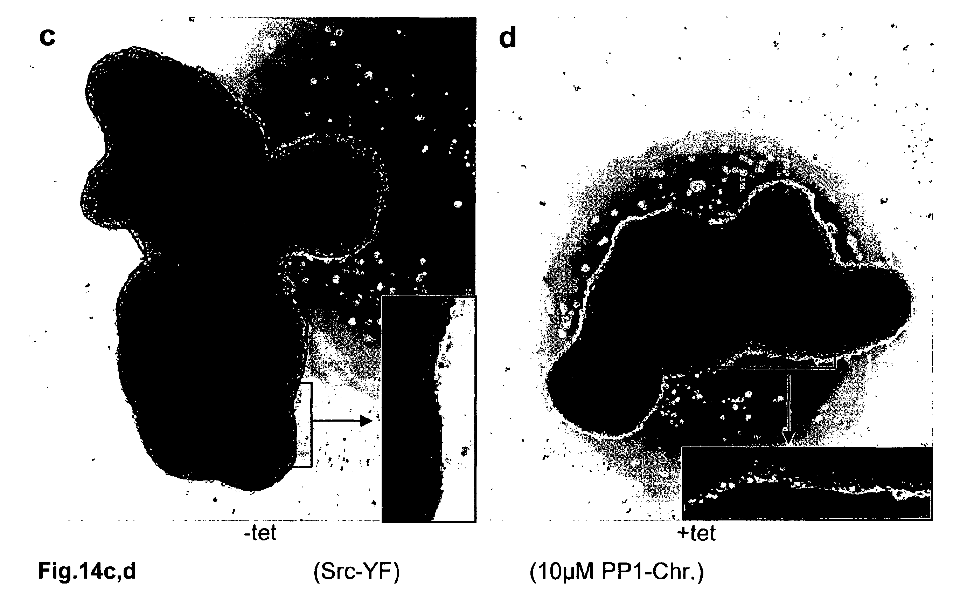

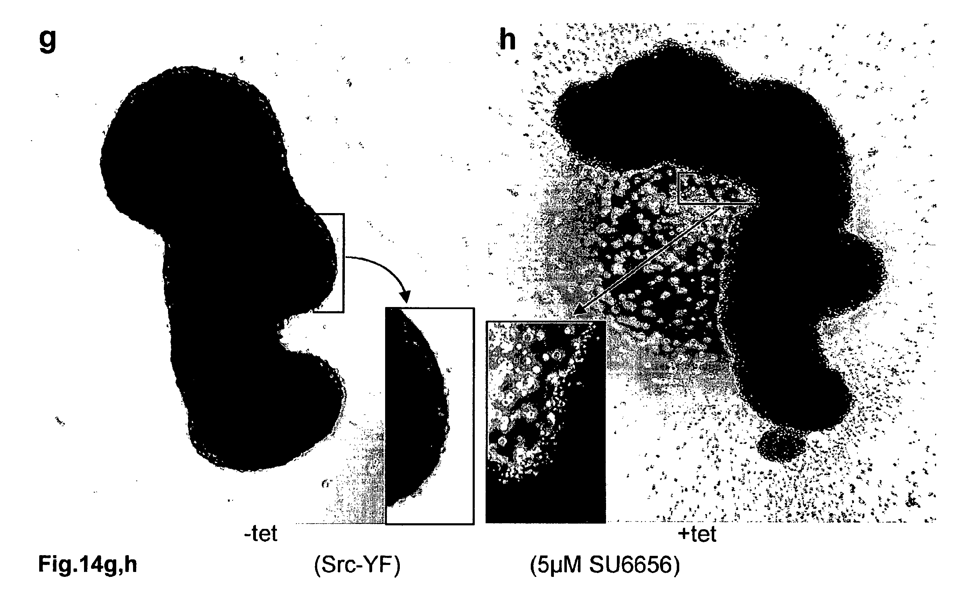

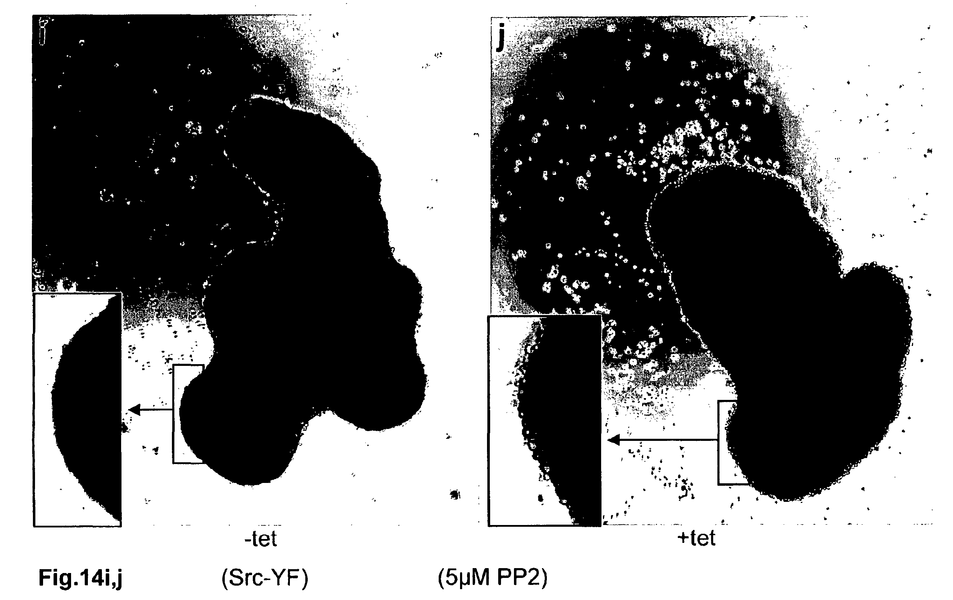

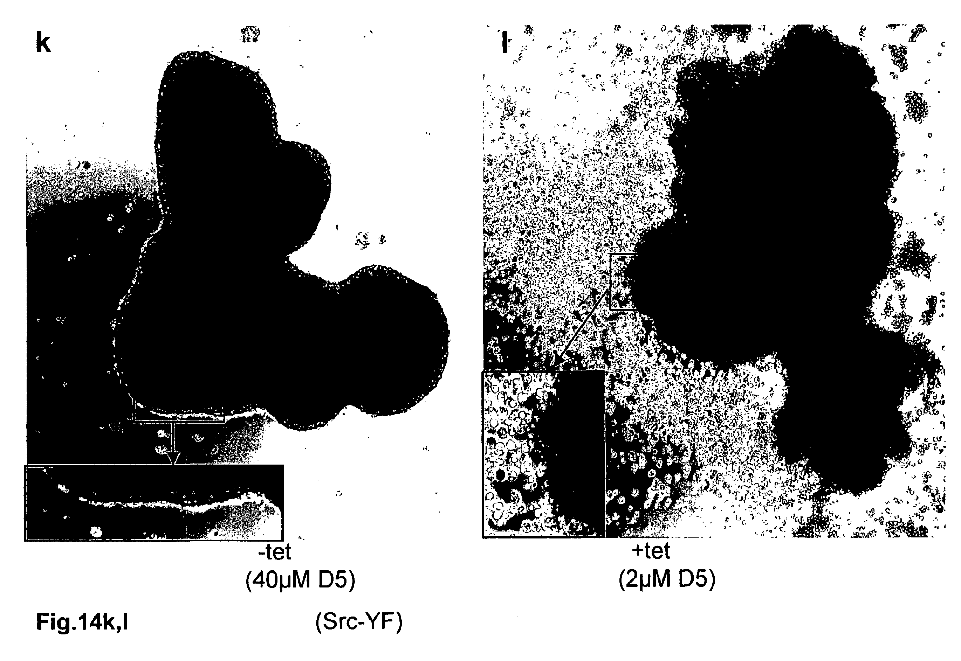

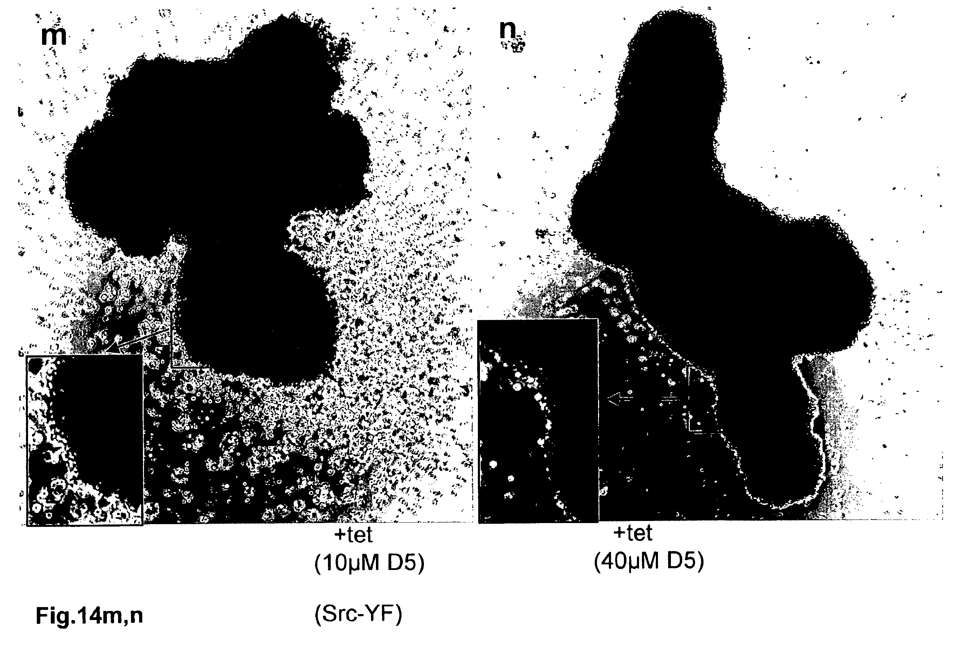

**Fig. 14 a-d, g-k** shows phenotypes of **Src-YF** expressing **ZM77.poly** cell line incubated in suspension (Ultralow Attachment Plates) in serum-containing medium for 2 days without (a, c, g, i, k) or with (b, d, h, j, 1, m, n) tet and the following drugs: 10µM PP1-Chr. (c, d); 5µM SU6656 (g, h); 5µM PP2 (i, j); 2µM D5 (1), 10µM D5 (m), 40µM D5 (k, n), or vehicle (0.2% DMSO;"-" (a, b)).
**Fig. 14 a, b** shows magnified insets of the cell aggregates show (a) the smooth surface characteristic for the "-tet-phenotype" and (b) the disintegration of the aggregate(s) into a loose mass of cells characteristic for the "+tet-phenotype", i.e. full Src activity
**Fig.14 c, d** shows magnified inset of the cell aggregate surface: (c) a smooth surface is characteristic for the "-tet-phenotype" (no Src activity); (d) increasingly "rough" surface correlates with increasing Src activity, "+tet-phenotype".
**Fig.14 g, h** shows magnified inset of the cell aggregate surface: (g) a smooth surface is characteristic for the "-tet-phenotype" (no Src activity); (h) increasingly "rough" surface correlates with increasing Src activity, "+tet-phenotype".
**Fig.14 i, j** shows magnified inset of the cell aggregate surface: (i) a smooth surface is characteristic for the "-tet-phenotype" (no Src activity); (j) increasingly "rough" surface correlates with increasing Src activity, "+tet-phenotype".
**Fig.14 k, 1** shows magnified inset of the cell aggregate surface: (k) a smooth surface is characteristic for the "-tet-phenotype" (no Src activity); (1) increasingly "rough" surface correlates with increasing Src activity, "+tet-phenotype".
**Fig.14 m** shows magnified inset of the cell aggregate surface: an increasingly "rough" surface correlates with increasing Src activity, "+tet-phenotype" (in comparison with Fig. 14 k).
**Fig.14 n** shows magnified inset of the cell aggregate surface: an increasingly "rough" surface correlates with increasing Src activity, "+tet-phenotype" (in comparison with Fig. 14 k).

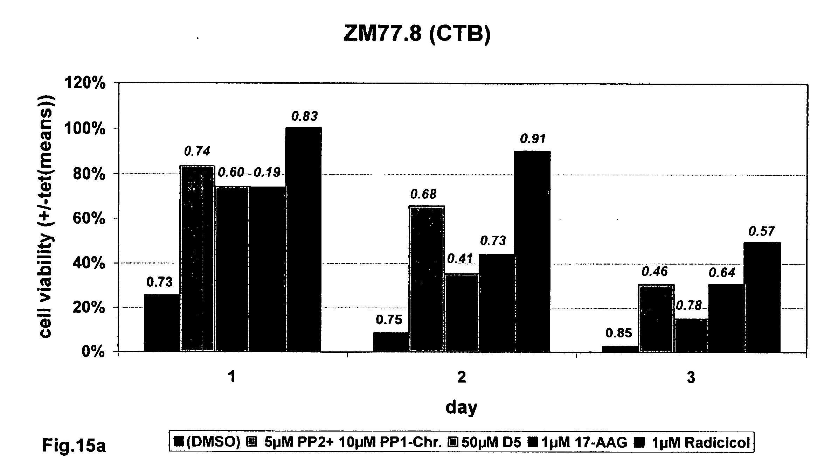

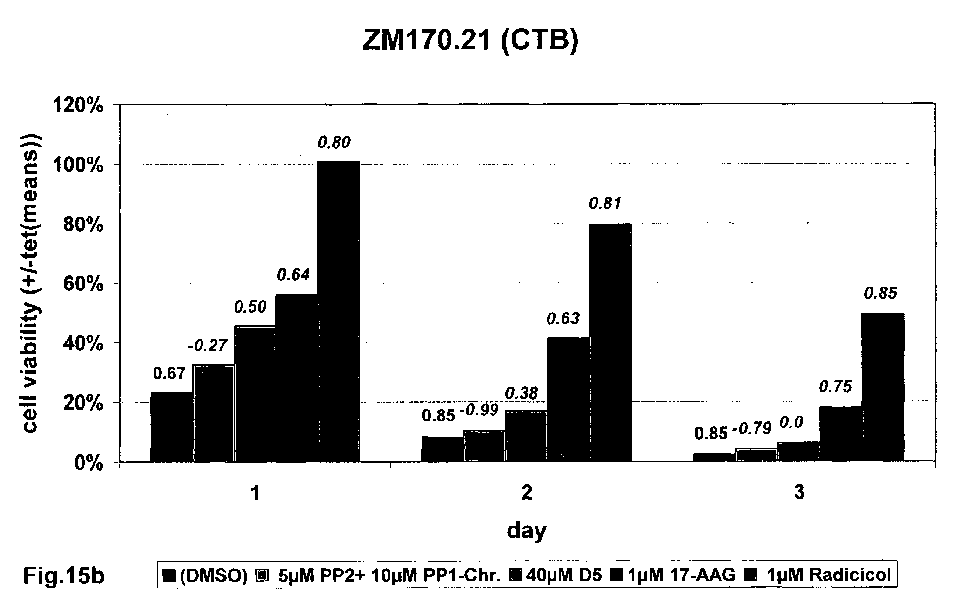

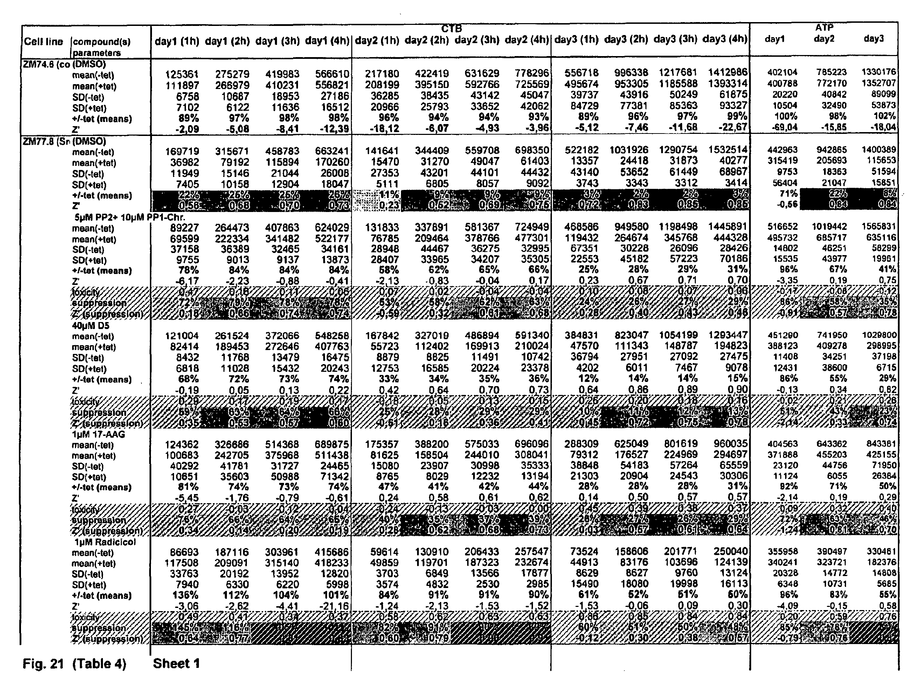

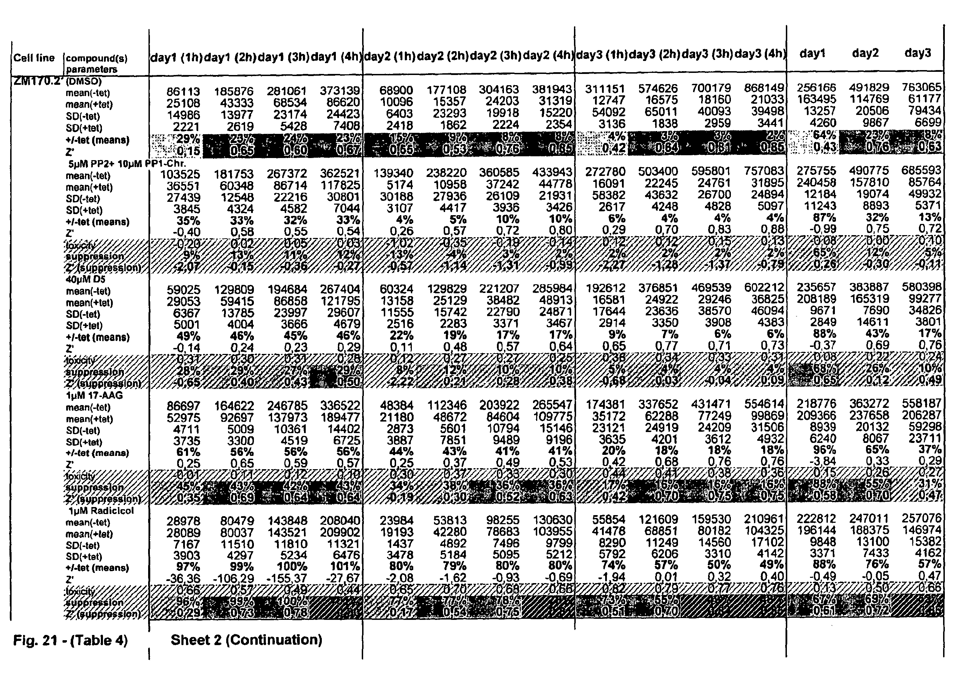

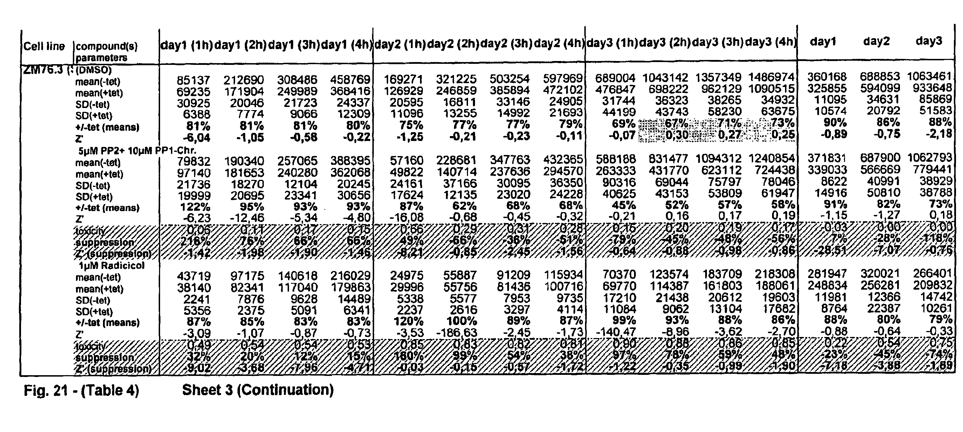

**Fig. 15 a, b** shows graphic display of the data from Tab.4 (Fig. 21) obtained for cell lines **ZM77.8 (Src-YF)** (Fig. 15 a) and **ZM170.21 (Src-TQ/YF)** (Fig. 15 b). The columns reflect the relative cell viability /metabolic activity values +/-tet(means) of Tab.4. The numbers on top of the respective first (left) columns (no compound) are the corresponding Z' values (concerning the numbers 0.73, 0.75 and 0.85 in Fig. 15 a; the numbers 0.67, 0.85 and 0.85 in Fig. 15 b). The italic numbers on top of the respective other columns (compounds) are the corresponding Z' (suppression) values.
   The cells were incubated with the following compounds: first (left) columns, respectively: no compound (vehicle DSMO); second columns, respectively. 5µM PP2 + 10µMPP1-Chr.; third columns, respectively: 40µM D5; fourth columns, respectively: 1µM 17-AAG; fifth columns, respectively: 1µM Radicol.

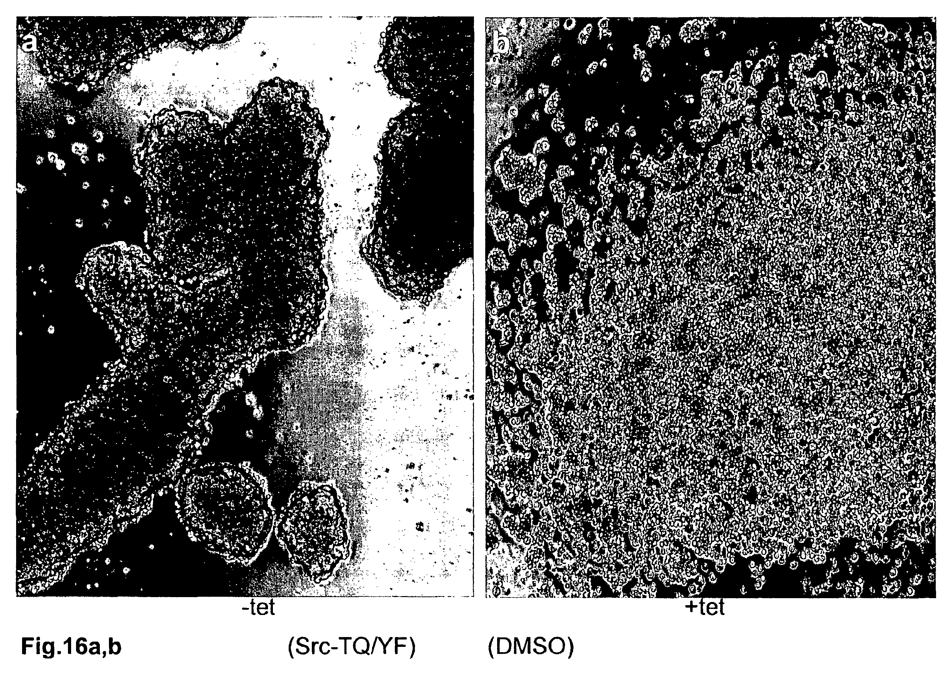

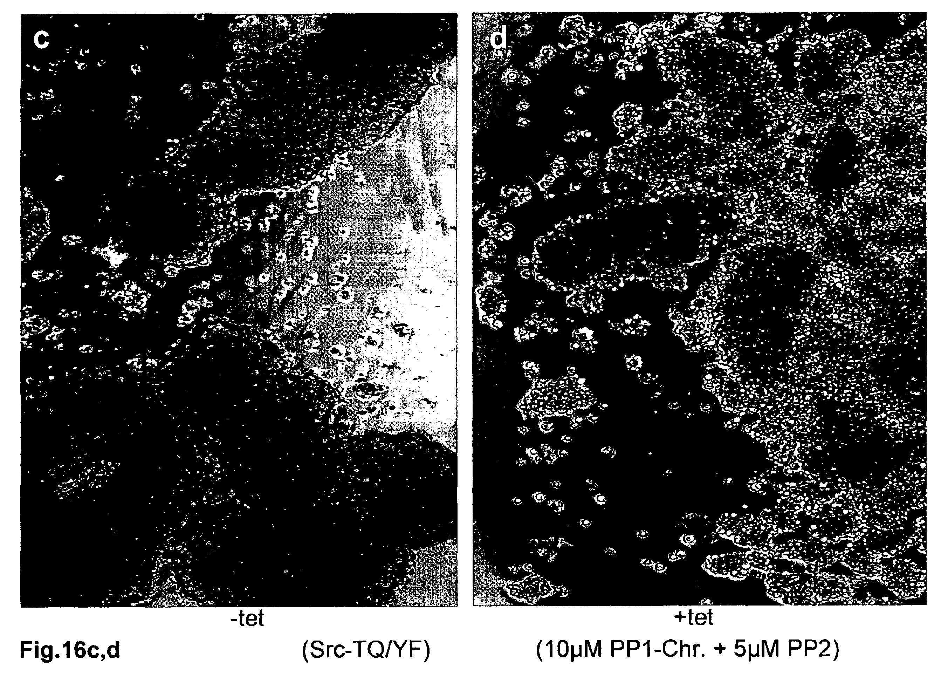

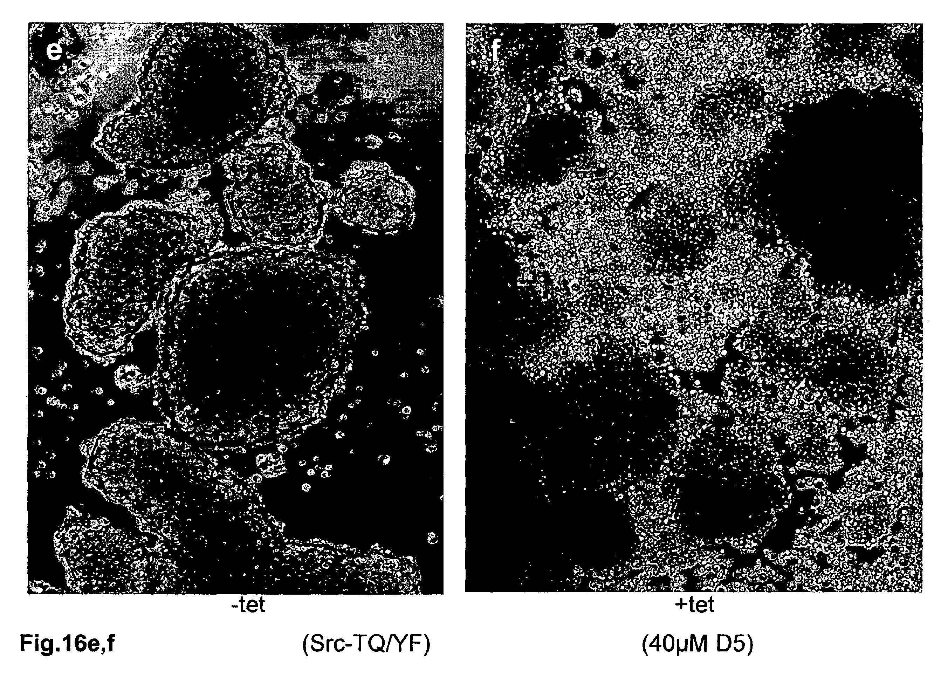

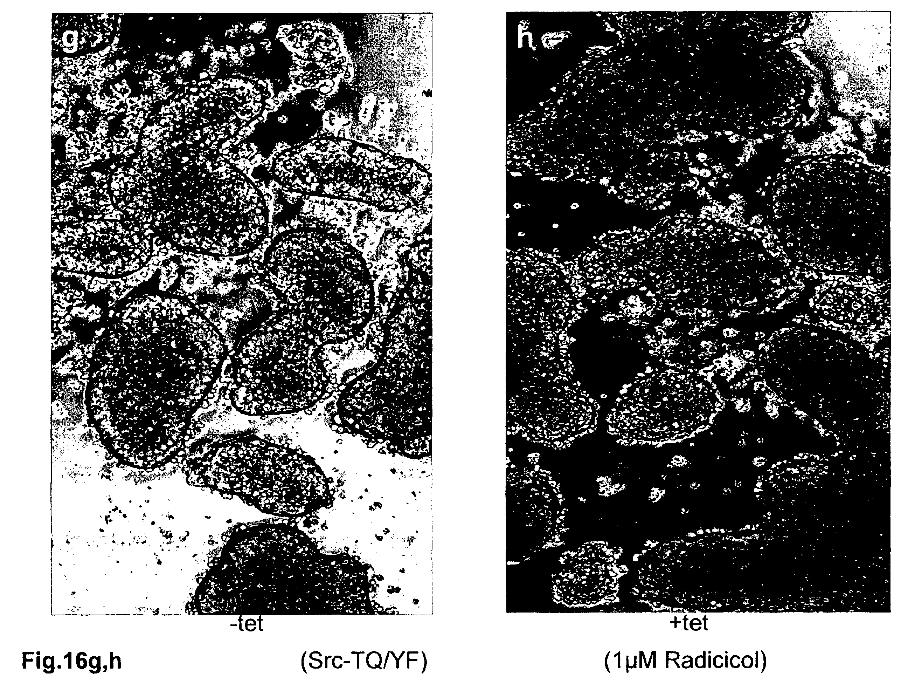

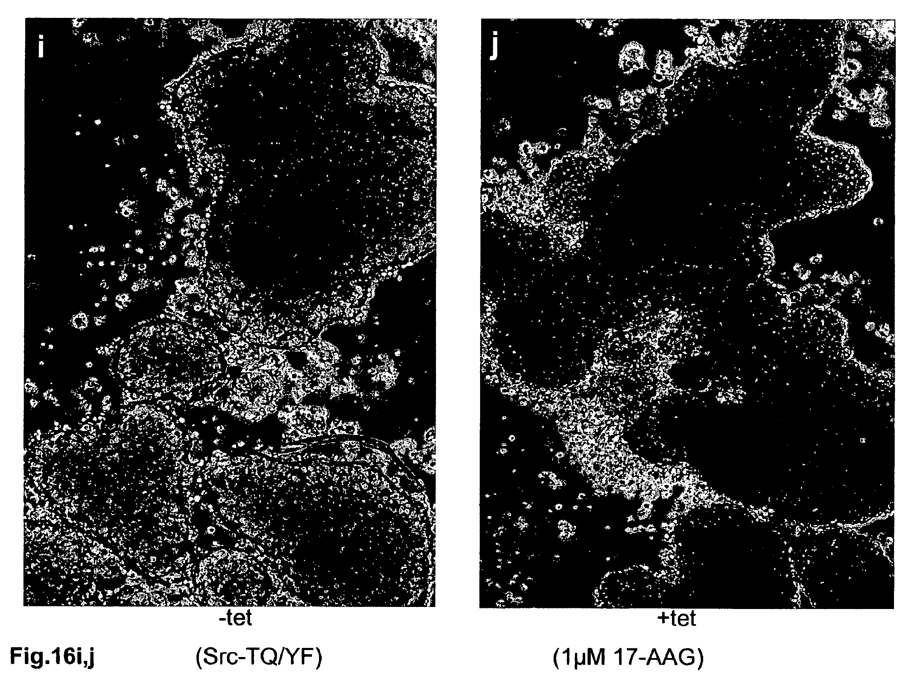

**Fig. 16 a-j** shows phenotypes of **Src-TQ/YF** expressing **ZM170.21** cell line incubated in suspension (Ultralow Attachment Plates) in serum-containing medium for 1 day without tet (a,c, e, g, i) or with tet (b, d, f, h, j) and the following drugs: 10µM PP1-Chr. + 5µM PP2 (c, d); 40µM D5 (e, f); 1µM Radicicol (g, h); 1µM 17-AAG (i, j) or vehicle (0.2% DMSO; "-" (a, b)).

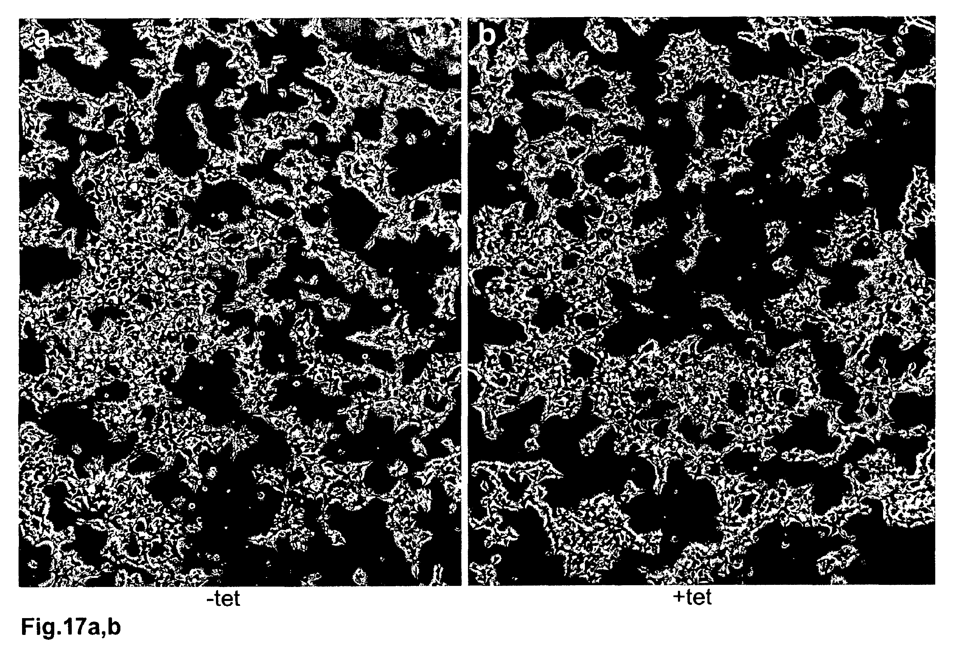

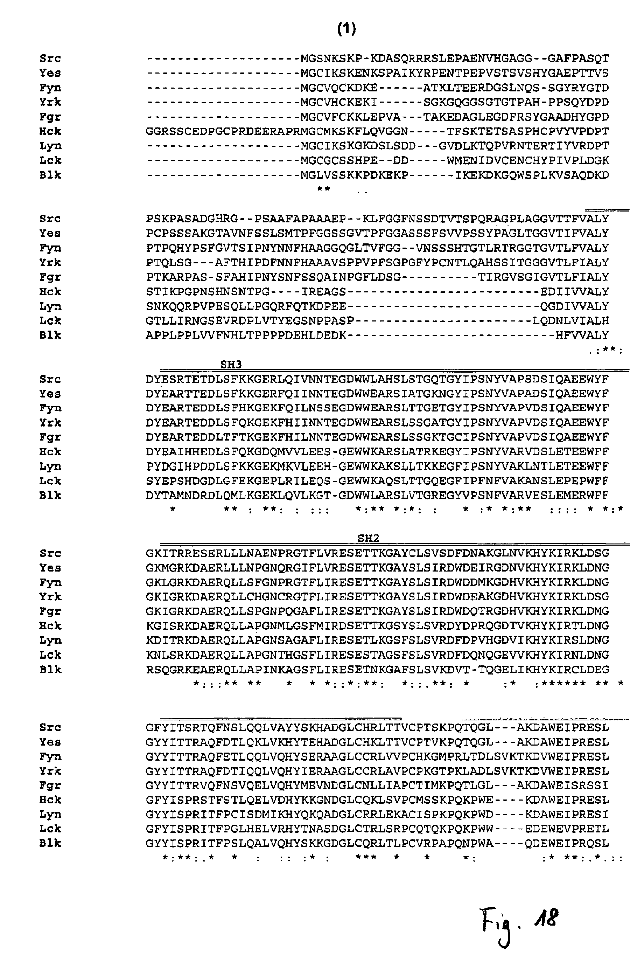

**Fig. 17** shows phenotypes of **ZM74.6 control cell line** incubated in serum-free medium for 1 day in the absence (a) or presence (b) of tet.
Fig. 18 shows an alignment of the sequences of the different members of the Src family: Src, Yes, Fyn, Yrk, Fgr, Hck, Lyn, Lck and Blk. All sequences are human, except Yrk which is from chicken. The kinase domain **SH1,** the domain **SH2** which binds phosphotyrosine-containing sequences and the domain **SH3** which binds proline-rich motifs are marked. The position of the constant amino acids K and T in the domain SH2 and Y in the domain SH1 are each identified in parenthesis for the different members of the Src family. It can be seen, that for example the Src kinase mutant Src-K298A, as described above, corresponds to the Yes kinase mutant Yes-K305A, the Fyn kinase mutant Fyn-K299A, the Yrk kinase mutant Yrk-K298A, the Fgr kinase mutant Fgr-K291A, the Hck kinase mutant Hck-K289A, the Lyn kinase mutant Lyn-K275A, the Lck kinase mutant Lck-K273A and the Blk kinase mutant Blk-K269A.

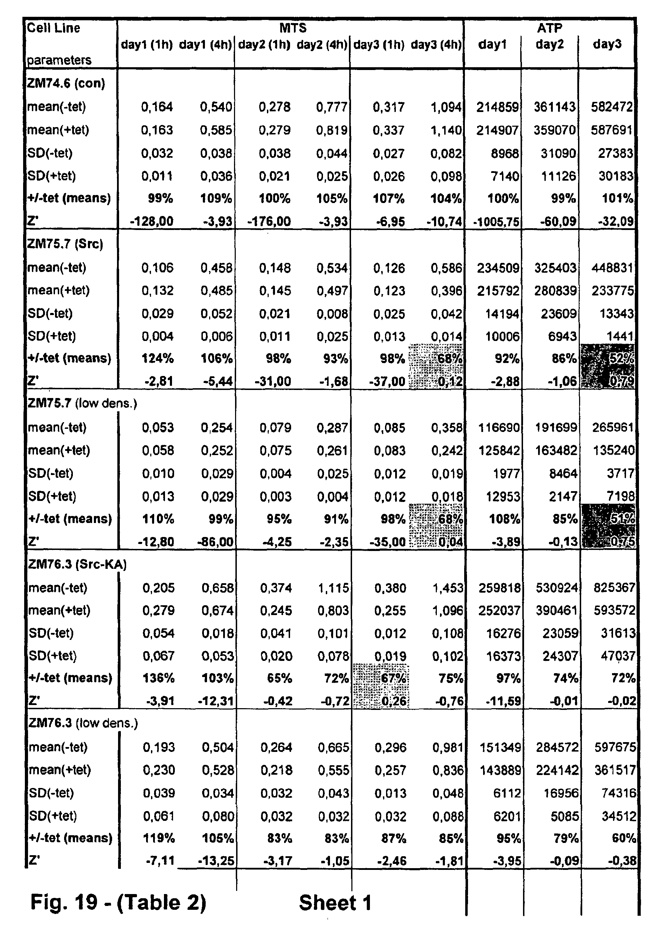

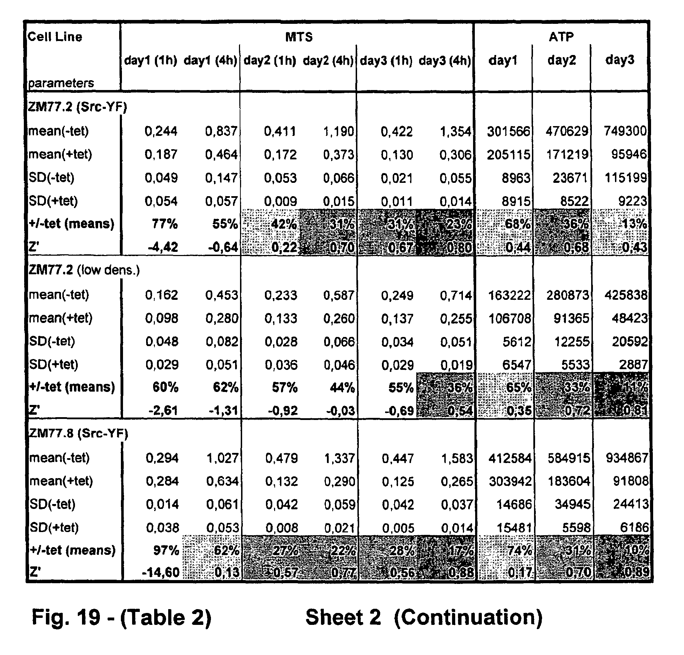

**Fig. 19** shows table 2 which summarizes results from MTS and ATP assays. Cell lines **ZM75.7 (Src), ZM76.3 (Src-KA), ZM77.2 (Src-YF)** and **ZM77.8 (Src-YF),** were tested for their reduction of cell viability/metabolic activity upon tet-induction.
   The indicated cell lines were grown in 96wells under their optimal growth conditions (see below, example 3). As a variation some cell lines were also seeded at 50% lower density (low dens.). One day after seeding cells were incubated with or without tet. Cell viability was measured 1 day, 2 days, and 3 days later using a Victor2 plate reader in the absorption mode (MTS assay) or the luminescence mode (ATP assay). In the MTS assay the cells were measured 1h and 4h after addition of the assay reagens. Ratios of cell viabilities with/without tet and the corresponding Z' values are indicated in bold type. SD (standard deviation).

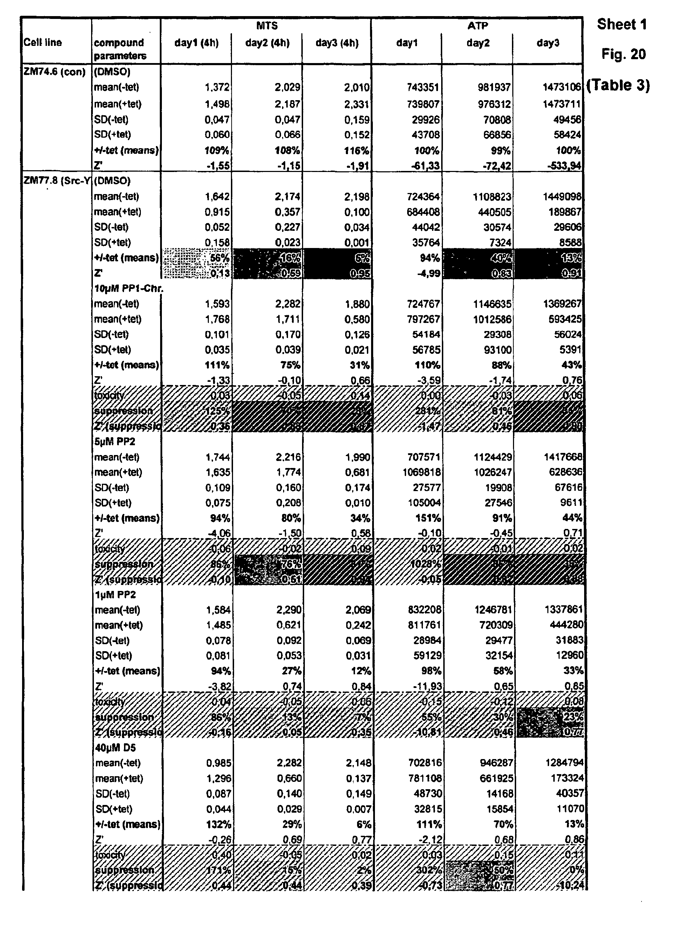

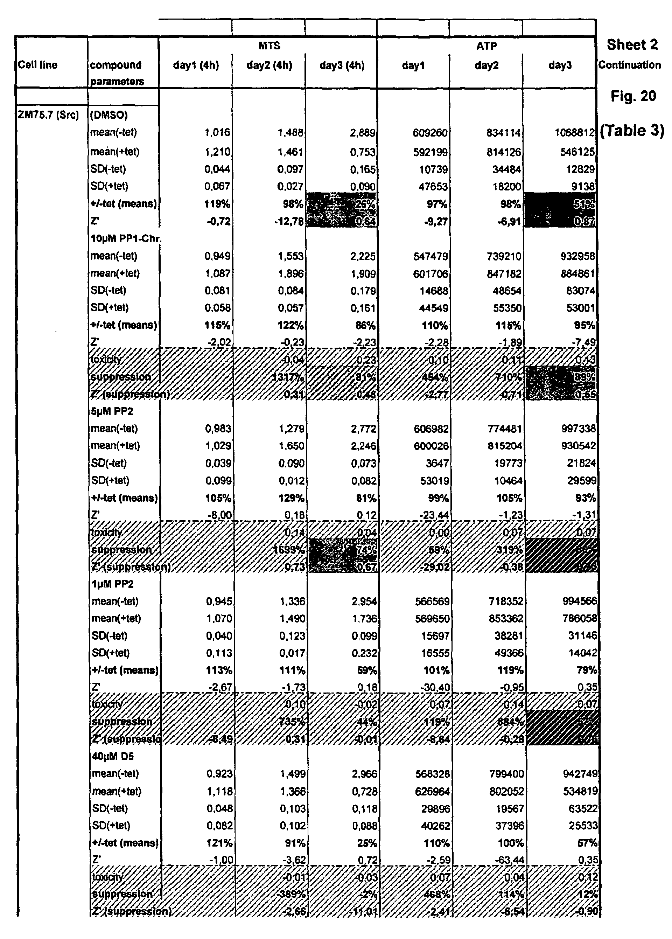

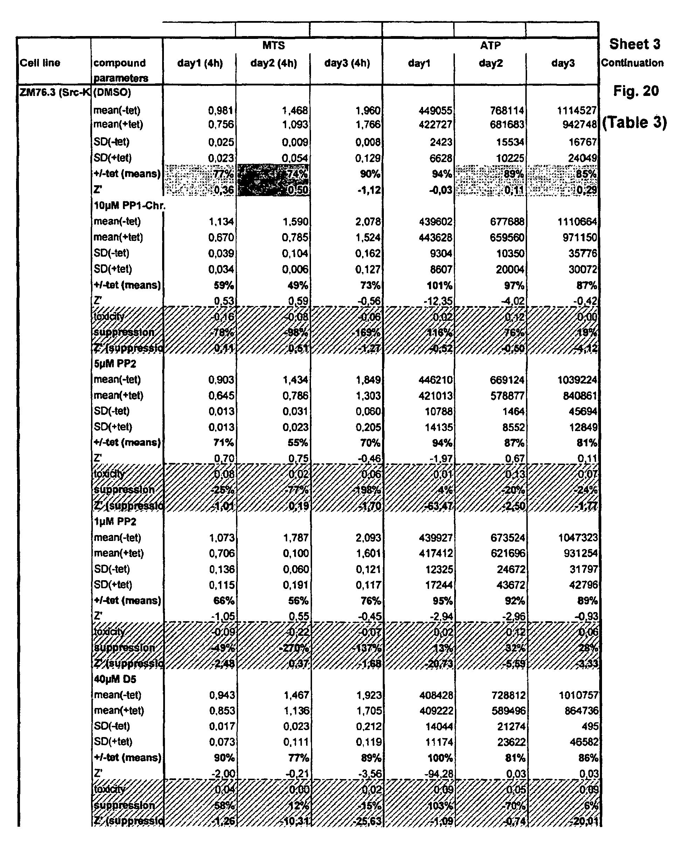

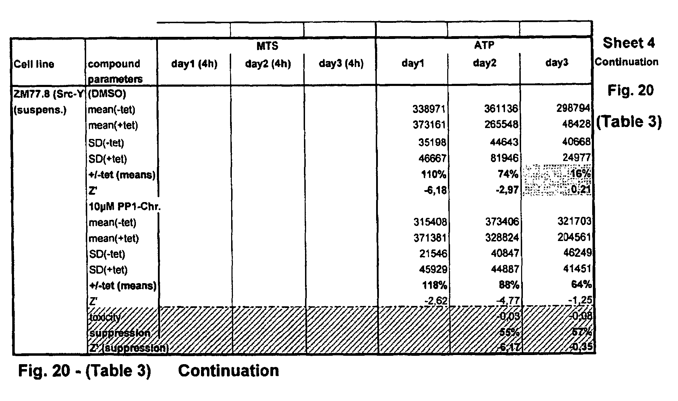

**Fig. 20** shows table 3 which summarizes results from analogous assays (MTS and ATP assays), in which the cell lines **ZM77.8 (Src-YF), ZM75.7 (Src)** and **ZM76.3 (Src-KA)** were used to evaluate the effects of test compounds PP1-Chr. and PP2 and of the newly identified compound "D5" with respect to their ability to suppress the tet-induced reduction in cell viability/metabolic activity.
   The indicated cell lines were grown in 96wells under their optimal growth conditions (see below, example 3). In one case (suspens.), cells were grown in suspension in ultralow adhesion plates. One day after seeding cells were incubated with or without tet and with the indicated compounds at the indicated concentrations or vehicle (0.2% DMSO). Cell viability was measured 1 day, 2 days, and 3 days later using a Victor2 plate reader in the absorption mode (MTS assay) or the luminescence mode (ATP assay). In the MTS assay the cells were measured 4h after addition of the assay reagens. Ratios of cell viabilities with/without tet and the corresponding Z' values are indicated in bold type. SD (standard deviation). Toxicity of the compounds (1 - mean(-tet,(DMSO))/mean(-tet,compound)) is indicated for each cell line. Suppression values reflect the capability of the compounds to suppress the tet-induced reduction of cell viability and are indicated in bold type together with the corresponding Z' (suppression) values.
**Fig. 21** shows table 4 which shows data obtained from the CTB assay and in comparison, with the ATP assay.
   The indicated cell lines were grown in 96wells under their optimal growth conditions (see below, example 3). One day after seeding cells were incubated with or without tet and with the indicated compounds at the indicated concentrations or vehicle (0.2% DMSO). Cell viability was measured 1 day, 2 days, and 3 days later using a Victor2 plate reader in the fluorescence mode (CTB assay) or the luminescence mode (ATP assay). In the CTB assay the cells were measured 1, 2, 3 or 4h after addition of the assay reagens. Ratios of cell viabilities with/without tet and the corresponding Z' values are indicated in bold type. SD (standard deviation). Toxicity of the compounds (1 - mean(-tet,(DMSO))/mean(-tet,compound)) is indicated for each cell line. Suppression values reflect the effects of the compounds to suppress the tet-induced reduction of cell viability and are indicated in bold type together with the corresponding Z' (suppression) values.

The following examples do not limit the scope of the present invention.

### Examples

Inter alia proof-of-principle experiments are provided, in which the following two classes of compounds are tested: (i) compounds, which directly inhibit the tyrosine kinase of Src (direct inhibitors), and (ii) compounds which inhibit the Hsp90 chaperone system, on which Src depends for proper folding, thereby causing Src degradation (indirect inhibitors).

As result, it follows that this assay platform can be used for the identification of compounds which directly inhibit Src activity as well as of compounds which interfere with the Hsp90 chaperone system.

### Example 1- Generating of expression constructs for Src and the Src mutants Src-KA, Src-TQ, Src-YF, Src-TQ/YF

Expression constructs have been generated to enable stable and inducible expression of Src kinase, and the Src kinase mutants Src-KA, Src-TQ, Src-YF and Src-TQ/YF (abbreviations denote single letter amino acid-code) in mammalian cells.

pcDNA4/TO (Invitrogen) was chosen as expression vector. The features of this vector are already described above.

The following mutant derivatives of the Src wildtype expression construct were generated. This has been achieved by means of site-directed mutagenesis using the "QuickChange Site-Directed Mutagenesis Kit" (cat.no. 200519, Stratagene, La Lolla, CA, USA) according to the protocol of the manufacturer and the following oligonucleotides:

Src-K298A: 5'-CCAGGGTGGCCATCGCCACCCTGAAGCCTGGCAC-3' (sense) and
5'-GTGCCAGGCTTCAGGGTGGCGATGGCCACCCTGG-3' (antisense);

Src-T341Q: 5'- CCATTTACATCGTCCAGGAGTACATGAGCAAG-3' (sense) and
5'-CTTGCTCATGTACTCCTGGACGATGTAAATGG-3' (antisense);

Src-Y530F: 5'-CCACCGAGCCCCAGTTCCAGCCCGGGGAG-3' (sense) and
5'- CTCCCCGGGCTGGAACTGGGGCTCGGTGG-3' (antisense).

Src-T(341)Q/Y(530)F: combination of Src-T341Q and Src-Y530F

### Example 2 - Generation and Biochemical Characterization of Human Cell Lines Stably Inducibly Expressing Src/ Src Mutants

The above described expression constructs were transfected into the "293/TREx" cell line (Invitrogen) using FuGENE (Roche, Basel, Switzerland) or Effectene (Qiagen) transfection reagents according to the manufacturers' protocols.

Cells are cultivated at standard incubator conditions (37°C, 5% CO₂/ humidified atmosphere) on standard cell culture plastic plates/ multiwell plates (Nunc, Roskilde, Denmark) in growth medium (DMEM (Gibco/ Invitrogen, cat.no. 61965-026), 10% FBS (Biochrom, cat.no. S0115, Lot No. 521A), 1% sodium pyruvate (Gibco, cat.no. 11360-039)) in the presence of the antibiotic blasticidin (5µg/ml) in order to keep the selection pressure on tet-repressor expression.

24 hrs after transfection of the 293/TREx cells with the inducible Src/ Src mutant expression constructs, zeo (cat.no. R-250-1, Invitrogen, Carlsbad, CA, USA) was included into the growth medium (250 µg/ml) for selection. Successfully transfected cells, which have stably integrated the transfected DNAs into the genome and which express the resistance gene, are resistant to zeo; all other cells, which have either not taken up the plasmid DNA or which have not stably integrated or lost the DNA, die in the course of ~ 1 week.

To obtain separately growing colonies (mono-clones) of stably transfected cells, cells were splitted at ratios of 1:50, 1:500 and 1:5000 onto new 6cm-culrure plates 24hrs after transfection. The majority of the cells were selected and expanded in pool (polyclonal). Monoclonal colonies were picked using cloning cylinders (e.g. Dunn Laboratories, cat.no. 2090-00608 for 6 (diameter) x 8 (hight) mm, no. 2090-00808 for 8 x 8 mm cylinders) and expanded in parallel until cell numbers were high enough for the cells to cover a 10cm-culture dish with near confluence (~10⁷ cells). The polyclonal pools were expanded as well. Aliquots (2 x 3cm-plate (2 x ~10⁶ cells) of the obtained mono- and poly-clonal cell lines were used for characterization, while the remaining bulks of cells were aliquotted, frozen in several cryovials (Nunc) and stored in liquid nitrogen.

For characterization, cells from each cell line were treated with tet (2µg/ml) (cat-no. 550205, Invitrogen) to induce the expression of the transgenes (Src / Src mutants) or left untreated (1 x 3cm-plate of cells, see above). ~16 h later cells were lysed. Src / Src mutant expression is monitored by Western blotting using Src-specific antibodies (Anti-c-Src (N-16) antibody, cat.no. sc-19, Sta. Cruz Biotechnology, Santa Cruz, CA, USA) and horseradish peroxidase-coupled secondary anti-rabbit IgG-antibody (cat.no. 25800685 Amersham Biosciences, Little Chalfont, UK). In order to qualify for further use in assay development a cell line has to have a clearly detectable expression of the desired transgene, and this expression must be tightly inducible, i.e. > 10-fold and preferably > 100-fold lower expression level without tet treatment, most preferably no detectable expression without tet (above the endogenous background, which is near the limit of detection). Therefore, the level of expression has to be high enough to technically allow the detection of an at least 10-fold weaker expression signal (see Fig. 1).

Several different cell lines were obtained and characterized for each transgene with the following results:
1 polyclonal and 14 monoclonal cell lines stably inducibly expressing Src: ZM75.poly, ZM75.1, .2, .3, .4, .5, .6, .7, .8, .9, .10, .11, .13, .14, .15.
1 polyclonal and 13 monoclonal cell lines stably inducibly expressing Src-KA: ZM76.poly, ZM76.2, .3, .4, .5, .6, .7, .8, .9, .10, .11, .12, .14, .15.
1 polyclonal and 19 monoclonal cell lines stably inducibly expressing Src-YF: ZM77.poly, ZM77.1, .2, .3, .4, .5, .6, .7, .8, .9, .11, .13, .14, .16, .17, .18, .19, .20, .21.
1 polyclonal and 21 monoclonal cell lines stably inducibly expressing Src-TQ: ZM78.poly, ZM78.2, .3, .5, .6, .7, .8, .9, .10, .11, .13, .14, .15, .16, .17, .18, .19, .20, .21, .22, .23, .28.
1 polyclonal and 30 monoclonal cell lines stably inducibly expressing Src/TQ/YF: ZM170.poly, ZM170.1, .2, .3, .4, .5, .6, .7, .8, .10, .11, .13, .15, .19, .20, .21, .22, .24, .25, .26, .27, .28, .29, .30, .31, .32, .33, .34, .35, .36, .37
display a clearly detectable and inducible Src / Src mutant expression.

1 polyclonal and 4 monoclonal control cell lines (transfected with the empty expression vector pcDNA4/TO) were obtained:
ZM74.poly, ZM74.3, .4, .5, .6. These control cell lines do not express any exogenous Src (consequently, the above requirements do not apply here).

Fig.1 exemplarily shows the inducible expression levels of some of the above mentioned cell lines.

### Example 3 - Qualitative/ Semi-quantitative Determination (by Eye/ Microscope) of a Biological Readout (Phenotype) for the Expression of Src/ Src Mutants and Identification and Characterization of Src Modulating Chemical Compounds

### a) Biological Responses in Normal Cell Culture Plates

### i) Biological Responses upon Induction of Src / Src Mutants Expression

Phenotype changes -tet → +tet were evaluated in 24-well plates (Nunc; cat.no. 143982) under the following several different growth conditions to identify the strongest possible response (Δₚₜ₍₋ₜₑₜ→+ₜₑₜ₎max) 1. full (10% serum containing) growth medium a) without any growth factor supplement (-), b) with epidermal growth factor (EGF; 100ng/ml) (cat.no. 115, Research Diagnostics Inc. (RDI), Flanders, NJ, USA) or c) platelet derived growth factor type BB (PDGF-BB; 30ng/ml) (cat.no. 114b, RDI) or d) hepatocyte growth factor (HGF; 20ng/ml) (RDI, cat.no.300-010), or 2. serum-free medium a) - or b) +EGF or c) +PDGF or d) +HGF. In all cases, cells were first seeded into standard 24-well or 96-well plates (Nunc) in full growth medium. The cell number seeded per well was also varied to identify the most suitable cell densities producing Δₚₜ₍₋ₜₑₜ→+ₜₑₜ₎max.

~24 h later the medium was changed to serum-free medium or not, growth factors and tet (2µg/ml) were added where applicable. Subsequently, the following observations were made: ZM74 (con) cell lines: As expected, no significant or consistent/ reproducible difference of the phenotype (morphology, proliferation, cell death) was apparent under any of the afore-mentioned growth conditions.

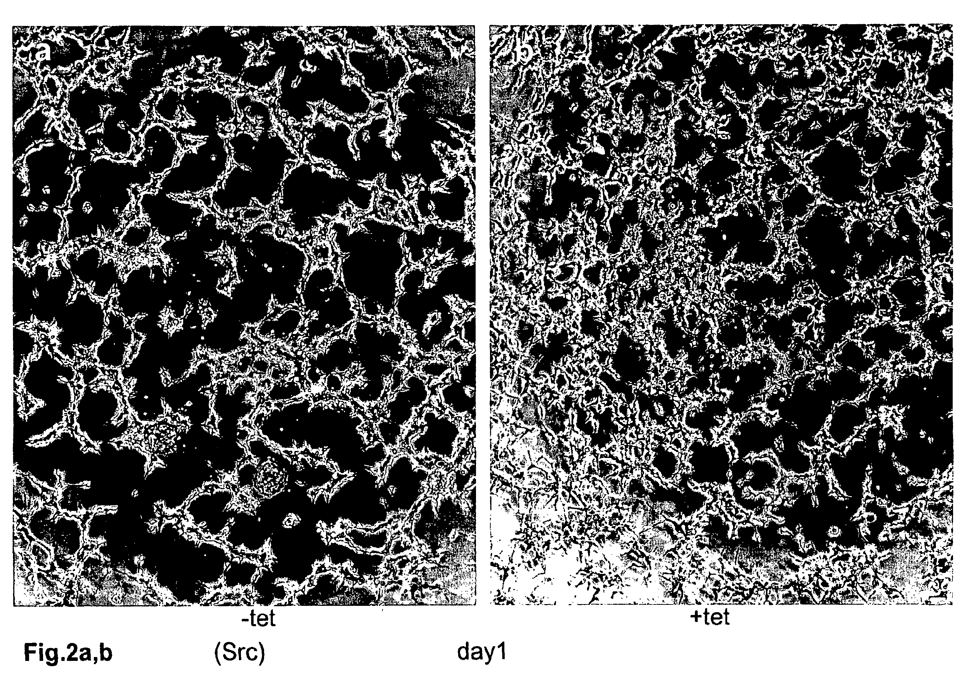

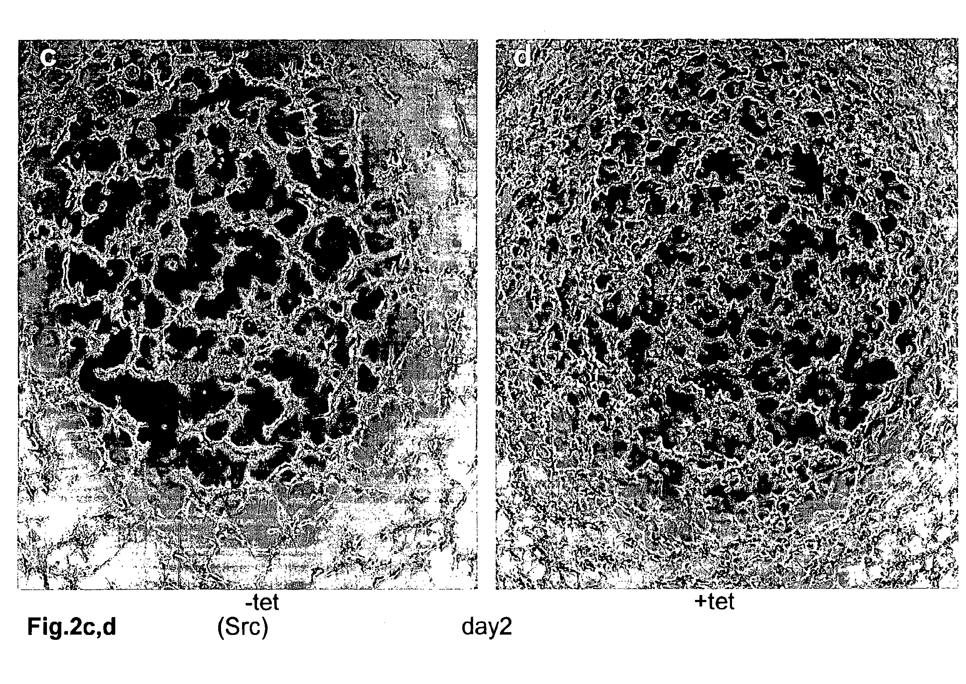

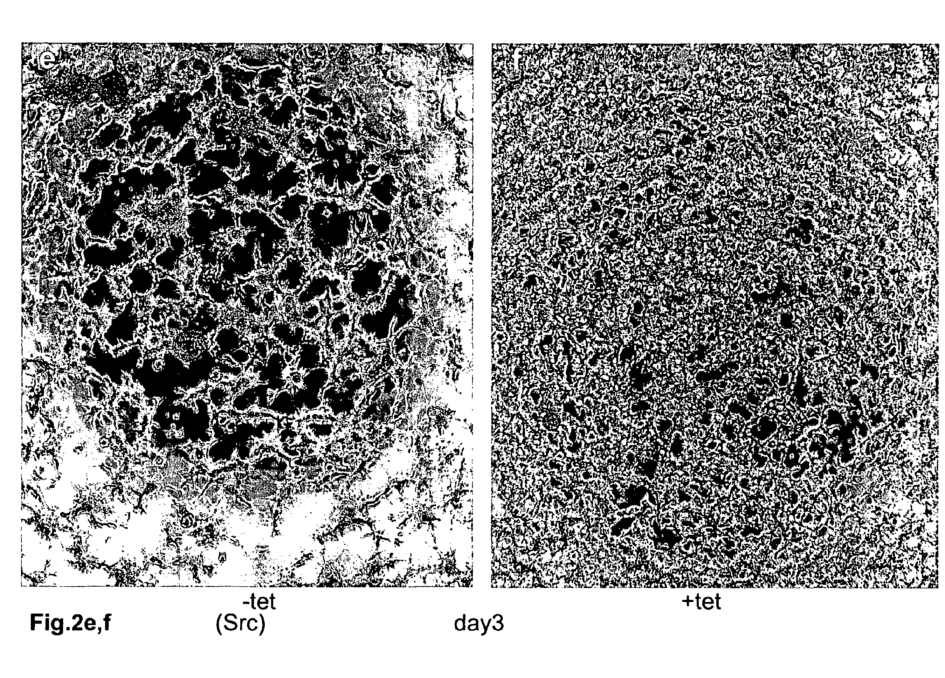

ZM75 (Src) cell lines: Induction of Src expression led to a most apparent phenotype change of the respective cells when they were grown in serum-free medium and without any growth factors (see Fig. 2).

Under such starving conditions cells did not grow well and start to move towards each other and clump together forming filamentous or round aggregates. These aggregates grew over time, sometimes into big clumps/ foci, and the cells appeared to fuse into big masses, while a large proportion of the plastic surface of the culture dish remained free of cells (see Fig. 2 a, c, e).

In the presence of tet, when Src expression was induced, the cells looked more viable, their distribution on the culture dish was more even and the cells grew more individually rather than forming large aggregates or foci, finally reaching confluence all over the dish (see Fig. 2 b, d, f). It was obvious that proliferation is increased.

In short, Src expression (in serum-free medium) allows the cells to grow more normal, like in serum-containing medium (see Fig. 2). The functional expression of wildtype Src compensates, at least in part, for the lack of serum. Therefore, the Src expression-induced phenotype changes are best revealed under serum-free conditions. A cell density/ confluence of approximately 30% at the time point of tet-induction is preferred. This gives the cells enough space to grow and spread over the plate (as they do in the presence of tet) during the following 2-3 days. A much higher cell density lead to premature full confluence (in the presence of tet), which diminish Δ_{pt(-tet→+tet)}. A much lower cell density lead to a general retardation of growth and to a reduced cell viability, which obscures the biological response and lead to a reduced Δ_{pt(-tet→+tet}). Δ_{pt(-tet→+tet)}max is usually reached 2-3 days after tet induction.

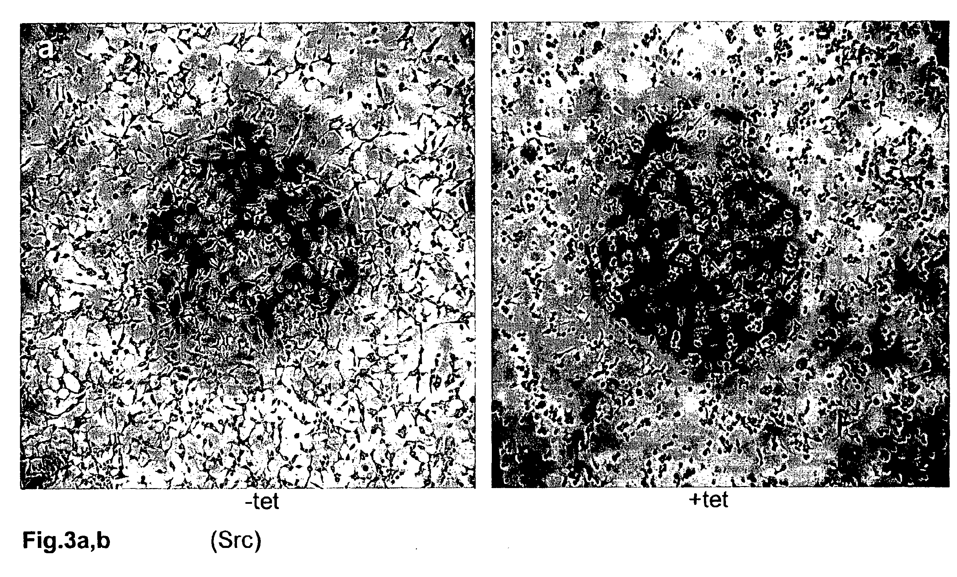

In one cell line, namely ZM75.7, expression of Src leads to rounding up of most cells and the cessation of growth, followed in some cases by cell death (see Fig.3). This phenotype change correlates with the very high inducible Src expression level in this particular cell line. Because tet-induction does not promote or allow cell growth in ZM75.7, but rather leads to cessation of growth, the optimal cell density for Δ _{pt(-tet→+tet)}max is somewhat higher for this cell line (~50%) than for the other ZM75 cell lines, and experiments can also be performed in serum-containing medium.

This suggests the existence of a threshold level of Src activity in a cell above which Src does not support and foster cellular viability anymore, but rather leads to rounding up of cells, detachment from the dish and cell death. This is seen yet more strikingly in cell lines expressing the hyperactive Src-YF (see below).

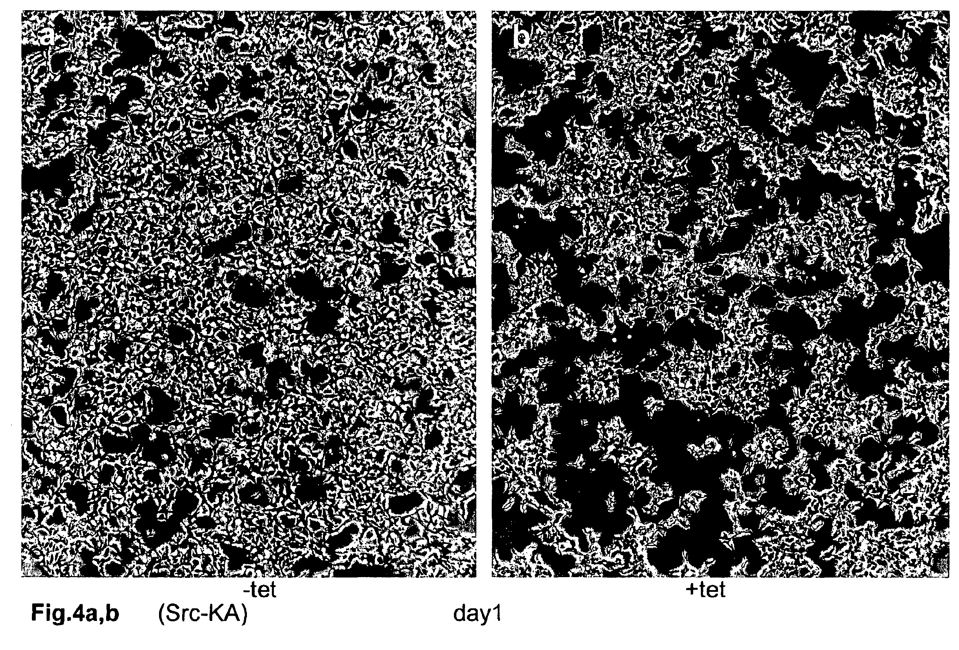

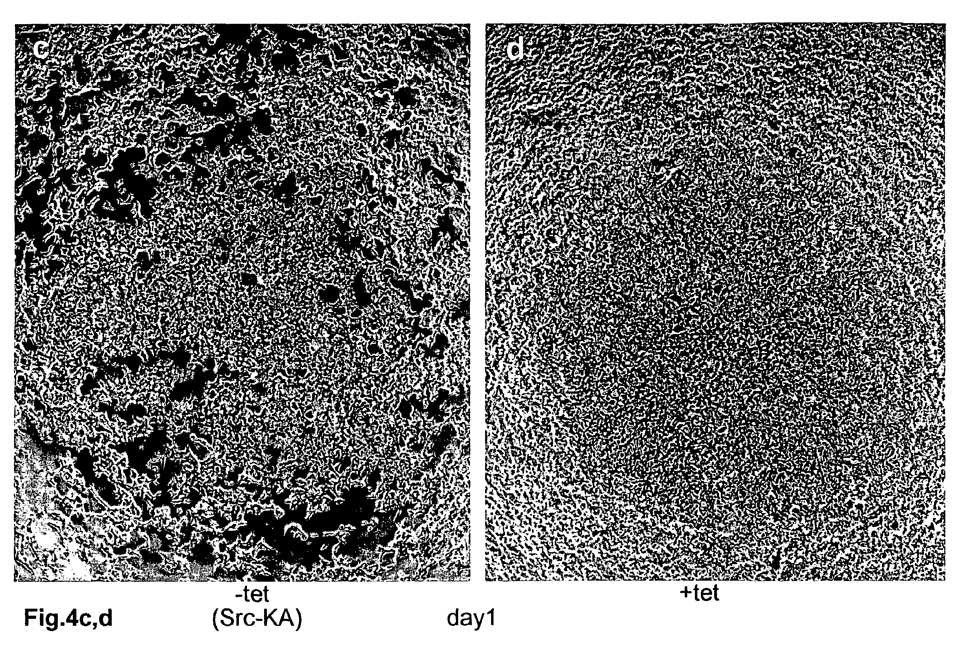

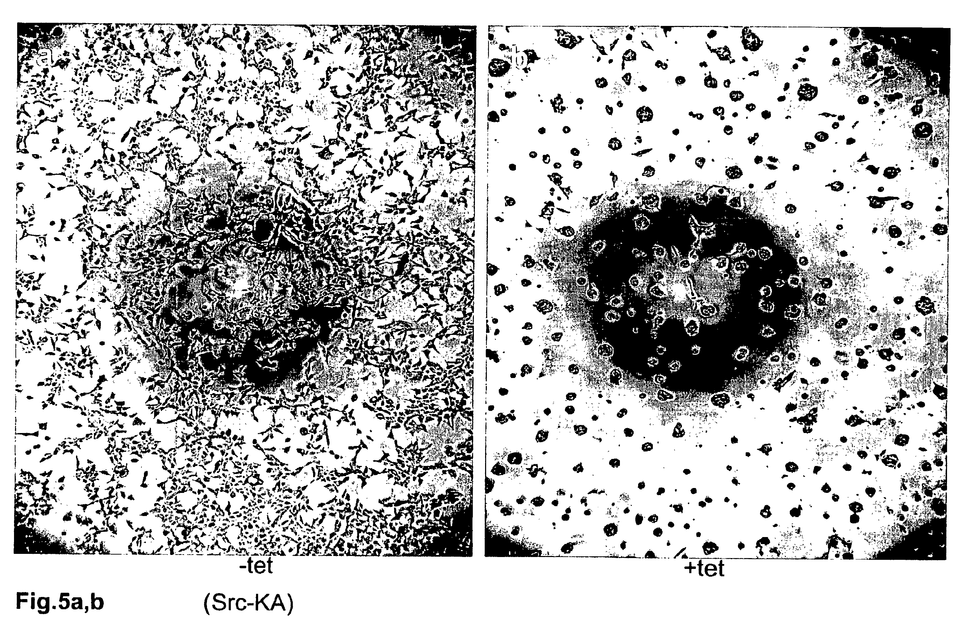

ZM76 (Src-KA) cell lines: Induced expression of Src-KA leads to a reduced proliferation rate and to uneven distribution of the cells over the culture dish surface and formation of aggregates/ clumps. (see Fig.4) This phenotype change is particularly dramatic in ZM76.3 correlating with the very high inducible expression level of Src-KA in this cell line (see Fig.5). The expression of Src-KA therefore appears to exert a negative effect on the cells, most likely due to a dominant-negative effect of this mutant as is well known in the literature from inactive mutants of other tyrosine kinases (Levin et al., 1993). As Src-KA induces a negative effect, the tet-induced phenotype-change is most obvious under optimal growth conditions, i.e. in serum-containing medium supplemented with 100ng/ml EGF. The optimal cell density at the time of tet-induction is around 30% (50% for ZM76.3) giving the cells enough space to grow and spread over the plate (as they do in the absence of tet) during the following 2-3 days. Δ_{pt(tet→+tet)}max is usually reached 2-3 days after tet induction.

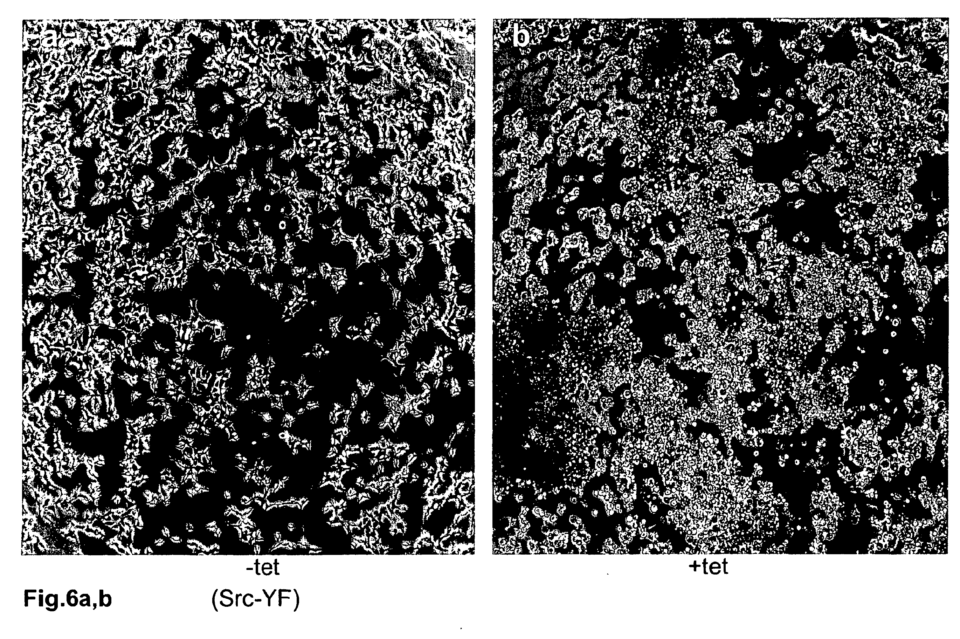

ZM77 (Src-YF) cell lines: Induction of Src-YF expression leads to rounding up of cells, followed by detachment of the cells and cell death (see Fig.6). As noticed before, the extent and the rate of this phenotype-change correlates with the level of transgene expression inducible in the different monoclonal cell lines. In most ZM77 cell lines, which inducibly express moderate to high levels of Src-YF, like for instance ZM77.2 and ZM77.8, the phenotype changes are very rapid and dramatic: Rounding up of the cells starts within a few hours and 6-10 h after tet addition cells are almost completely rounded up and detached; the phenotype is fully established overnight; i.e. Δ_{pt(-tet→+tet)} is already very high, although the continued growth of the cells in the absence of tet further increases Δ_{pt(-tet→+tet)} until the cells reach confluence. In order to reach Δ_{pt(-tet→+tet)}max as rapid as possible, cells should be seeded so that the density is 60-70% at the time of tet induction. The phenotype change is seen equally well in medium with or without serum. However, serum-containing medium was used, because cells start to starve in serum-free medium after approximately one day, and therefore serum-free medium diminish the huge Δ_{pt(-tet→+tet)}max.

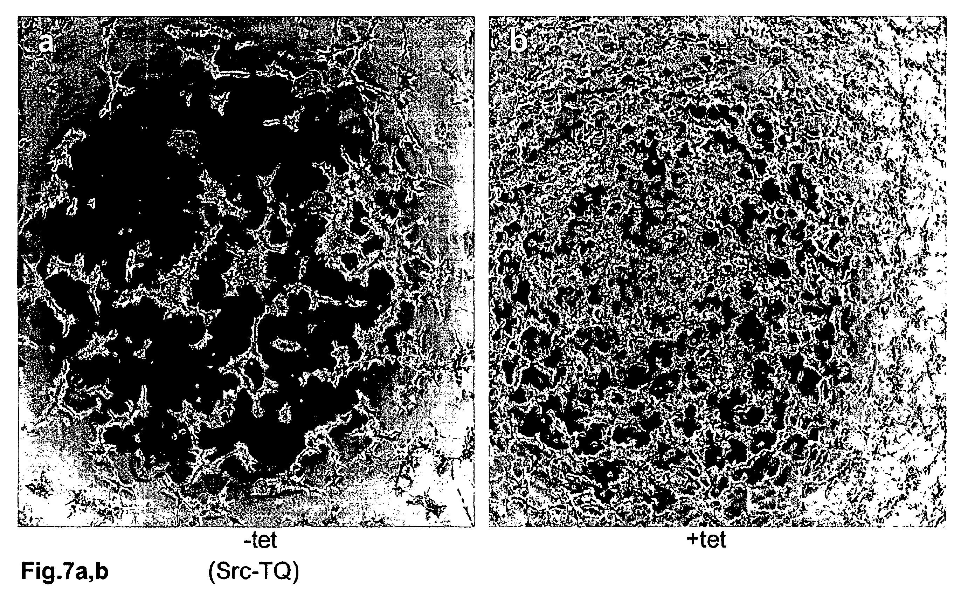

ZM78 (Src-TQ) cell lines: As expected, induction of Src-TQ expression leads to phenotype-changes very similar to those observed in ZM75 cell lines upon expression induction of wildtype Src (see above) (see Fig.7). As in the latter, tet-induced phenotypes are most overt under serum-free conditions.

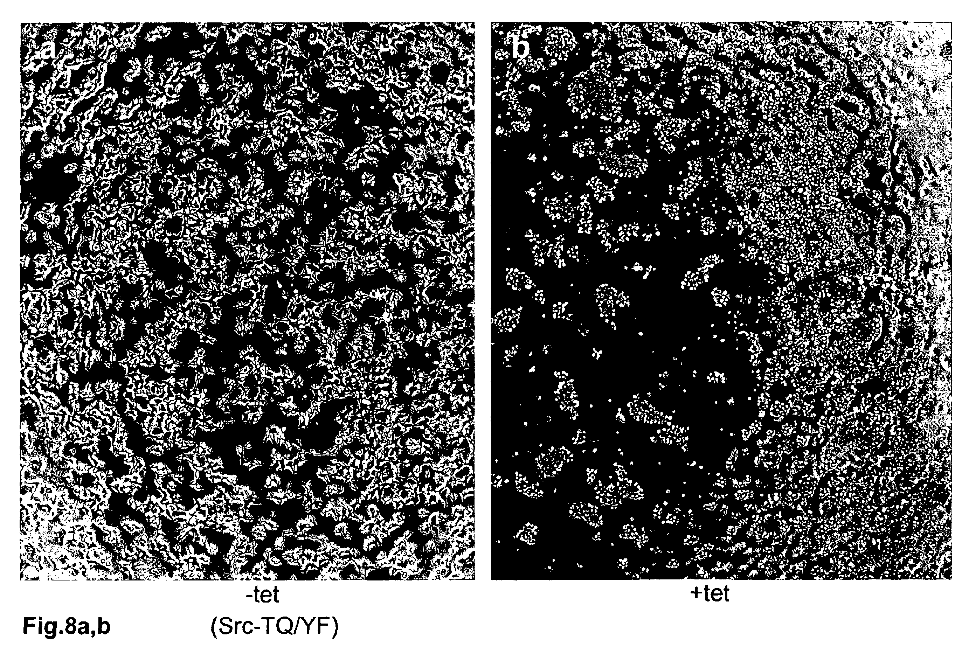

ZM170 (Src-TQ/YF) cell lines: As expected, induction of Src-TQ/YF expression leads to phenotype changes equivalent to those observed in ZM77 cell lines upon expression induction of hyperactive Src (see Fig.8).

Comparisons of the extents of the respective phenotypic changes (-tet **→** +tet) provided a rational basis for the selection of a few cell lines displaying strong biological responses upon tet-induction, which were used to further develop the assay as described below.

The following cell lines were chosen:
ZM75.13, and ZM75.poly; in addition: ZM75.7 (providing a different and more dramatic (ZM77-like) response);
ZM76.3;
ZM77.poly, ZM77.2 and ZM77.8;
ZM78.13;
ZM170.21;
ZM74.6 was routinely used as the control.

### ii) Suppression of the Biological Responses by Chemical Compounds (Proof-of-Principle)

In proof-of-principle experiments, test compounds, known to inhibit Src kinase activity were used to determine, whether a suppression of the aforementioned Src/ Src mutant-induced phenotype changes can be observed.

To that end, the test compounds PP1-Chr., PP2 (cat.no. 529573, Calbiochem) and SU6656 (cat.no. 572635, Calbiochem) (see Fig.9p for chemical structures) were individually added to the cells together with tet, and the development of the phenotype changes in the presence of these compounds was monitored subsequently in direct comparison with cells that received no compound or only vehicle (0.2% DMSO) together with tet induction. The compounds were also added to the cells without tet-induction of Src/ Src mutant expression as controls for non-specific/ toxicity effects. The experiments were initially performed with several ZM77 cell lines. As depicted in Fig.9, the phenotype changes of the cell line ZM77.poly (Src-YF) were partially suppressed by the various test compounds (see Fig.9a-k). In the context of the ZM77 cell lines, the presence of one of the test compounds reduced the tet-induced rate of cell rounding, detachment and death. The effect was most obvious at early time points (8h - 1 day), when the phenotype of the tet-treated cells, that did not receive any compound, was just fully established or established to a large extent. The block of the phenotype change by the compounds was released at later time points (depending on the compounds, typically after 8h to 2 days), in most cases due to a progressing degradation of the compounds.

Furthermore a clear correlation between different compound *concentrations* and the *degrees* of suppression of the tet-inducible biological responses was shown (e.g. Fig.9j,k and Fig.9m,n,o): Suppression was generally stronger with increasing concentrations of the test compounds.

Compounds were also tested on the Src-wt expressing cell line ZM75.7 (Fig.10). An interesting phenomenon was observed. Inhibition of Src not only reduced or prevented the tet-induced phenotype of round and detached cells, but also resulted in an increased confluence of the cells due to increased proliferation and a more even distribution of the cells on the plate (Fig.10c,e).

The presumable explanation for this is that the partial inhibition of Src in these cells by the compounds reduces the very high levels of active Src in these cells to a level, which is functionally comparable to the Src expression levels in other ZM75 cell lines, which promote confluence of the cells (see above). The partial inhibiton by the compounds brings the activity of Src in these cells down below the threshold level of Src activity above which Src does not promote cellular viability anymore, but rather leads to cessation of growth, rounding up of cells, detachment from the dish and cell death.

In a small-scale screening campaign testing novel compounds with unknown activity against Src in whole cells, several molecules were identified, which significantly and specifically suppressed the observable phenotype changes. The (concentration-dependent) suppression effect of one compound, designated "D5", which has been identified in this screen with ZM77 cell lines, is shown in Fig.9 I-o. These results demonstrate that the described assay is well suited to screen and identify (novel) compounds, which inhibit Src activity in whole cells.

For reasons of specificity some compounds were further tested on some of the other cell lines:

Importantly, the compounds were not able to suppress the tet-induced phenotype changes in Src-KA expressing ZM76 cell lines; rather it is often observed that the presence of some compounds further increases the tet-induced phenotype change (negative suppression), due to a synergism with the dominant-negative effect [this is shown examplarily for PP1-Chr./ PP2 and D5 in Fig.11a-f]. This indicates that these compounds exert their effects specifically via inhibition of Src's kinase activity. It can therefore be excluded that the observed effects are mediated by their binding to any of the non-catalytic domains of Src thereby blocking protein-protein interaction-mediated signalling nor mediated by any indirect means resulting in a general inhibition of Src folding and/ or expression.

The test compounds PP1-Chr./ PP2 were also ineffective in suppressing the tet-induced phenotype changes in Src-TQ expressing ZM78 cell lines and in SrcTQ/YF expressing ZM170 cell lines [compare Fig.12a-d (Src-TQ/YF expressing ZM170.21) with Fig.13a-d (Src-YF expressing ZM77.8)]. This indicates, as a further level of specificity, that the compounds inhibit Src kinase activity by binding into the ATP binding site and that other non-Src-related kinases, which do not have a threonine at the homologous position, would not be inhibited by these compounds.

In contrast, the novel inhibitor D5, while ineffective against the Src-KA-induced effects, did suppress both Src-YF- and Src-TQ/YF-induced effects, although suppression was somewhat weaker for the latter (compare Fig.12e,f with Fig.13, e,f]. This shows, that D5 does not seem to be very specific for the threonine at position 341, but also tolerates glutamine instead, which makes it obvious that it will to some extent also inhibit other non-Src-related tyrosine kinases.

In further analogously performed proof-of-principle experiments, the test compounds geldanamycin (cat.no. G-1047, A.G. Scientific), 17-AAG (cat.no. A-1256, A.G. Scientific Inc., San Diego, CA, USA) and radicicol (cat.no. R-1130, A.G. Scientific) were used to determine, whether a suppression of the Src/ Src mutant-induced phenotype changes can be observed.

These Hsp90 inhibitors were very effective in these assays, suppressing the phenotype of all Src and Src mutant expressing cell lines tested in a concentration-dependent manner [exemplarily shown for radicicol and 17-AAG in Fig.s11g-j, 12g-j and 13g-j]. The suppression is comparable or stronger and usually a bit longer-lasting than the suppression observed with the direct Src kinase inhibitors PP1-Chr., PP2, SU6656 and the newly identified D5. The inhibition via the folding machinery is very fundamental for Src. When direct Src kinase inhibitory compounds are degraded over time the block is released without any delay, whereas reexpression of correctly folded Src protein takes some time after derepression of the folding machinery following the degradation of Hsp90 inhibitory compounds.

It is important to emphasize that these compounds are also effectively suppressing Src-KA-and Src-TQ/YF-induced phenotype changes (Fig.s11/12g-j). This fits perfectly with their mechanism of action: Through inhibition of Hsp90, they prevent a proper folding of Src and Src mutants, leading to proteasomal degradation of the (misfolded) Src proteins. This degradation affects all Src and Src mutant proteins likewise, including Src-KA and Src-TQ/YF. Thus, any inhibitor of the chaperone system, which is responsible for Src folding and which consists of Hsp90, Hsp70 and Hop (which interacts with both Hsp70 and Hsp90 via two of its TPR domains; Scheufler et al., 2000), can be distinguished from a direct Src kinase inhibitor by its suppression of Src-KA-induced effects.

Taken together, the mutants Src-KA and Src-TQ/ Src-TQ/YF allow the classification of compounds with regard to their modes of action / specificity as follows:

**Tab.1:**

| | | **Suppression of Src-** | | |
|---|---|---|---|---|
| **Mode of action/ specificity** | **examples** | **YF (wt)** | **TQ/YF (TQ)** | **KA** |
| *1. Indirect (upstream):* | | | | |
| Inhibition of the Hsp90/Hsp70/Hop multichaperone complex | Radicicol, Geldanamycin, 17-AAG | + | + | + |
| *2. Direct or indirect (downstream)* | | | | |
| Kinase inhibitors (non-specific or non-ATP-competitive) or inhibitors of Src effector(s) | SU6656 | + | + | - |
| *3. Direct* | | | | |
| a) Kinase inhibitors (ATP-competitive) with limited specificity | D5 | + | +/- | - |
| b) Kinase inhibitors (ATP-competitive + specific for Src(-like) kinases) | PP1-Chr., PP2 | + | - | - |

It follows that the assay platform of this invention is not only suited for the identification and characterization of compounds inhibiting the activity of Src, but can equally well be used to screen, identify and evaluate compounds which inhibit the Hsp90-based chaperone system by any means: inhibitors of the Hsp90ATPase; compounds which block protein-protein-interaction between Hsp90 and other proteins like Hsp70, Hop, Cdc37 and others cooperating with Hsp90 in the folding of Hsp90 client proteins (e.g. HER2, Akt, Raf, Cdk4, Src), as well as inhibitors of these cooperating proteins and compounds blocking protein-protein interactions among these cooperating proteins, for example the interaction between the TPR domains of Hop on the one hand and the carboxy-termini of Hsp70 and Hsp90 on the other hand.

### b) Biological Responses in Ultralow Attachment Plates

### i) Biological Responses upon Induction of Src / Src Mutants Expression

Cells were seeded into 24-well or 96-well "Ultra Low Attachment" plates (cat.no.: 3473, Costar Corning Corp., Acton, MA, USA). These plates are coated with a covalently bound hydrogel layer that prevents cellular attachment to the polystyrene surface of the wells. As the cells were not attached to these wells, a medium change was impossible here. However, the phenotypes were tested in full serum-containing medium in the absence or presence of EGF, PDGF-BB or HGF as detailed above. Tet-induction was also started the day after seeding.

Largely irrespective of the cell numbers per well, the cells of all cell lines were in suspension and formed one or few very condensed/compact, opaque, rod-, disc- or kidney-shaped aggregates with a smooth surface, in which individual cells could not be discerned. Phenotype changes became apparent ~24 h after tet induction in ZM77 and ZM170 cell lines only, and Δₚₜ₍₋ₜₑₜ →+ₜₑₜ₎max was reached after ~2 days. In these cell lines Src-YF or Src-TQ/YF expression, respectively, induces scattering of the cells. Many cells are floating in the medium individually or in small clusters and the remaining large aggregate(s) are irregular-shaped and rather loose than compact; individual cells can easily be discerned and thus, the surfaces of these aggregates are rough as with bunches of grapes (Fig.14a,b). This biological response was readily observed with or without any of the above growth factors, wherefore normal growth medium without any additional growth factor was subsequently used in this assay type.

There was no phenotype change upon tet-induction in control or any of the other Src, Src-KA or Src-TQ expressing cell lines.

### ii) Suppression of the Biological Responses by Chemical Compounds (Proof-of-Principle)

In proof-of-principle experiments, test compounds known to inhibit Src kinase activity or known to inhibit the Hsp90 chaperone complex were used to determine whether a suppression of the aforementioned Src-YF-induced phenotype changes can be observed.

To that end, the test compounds PP1-Chr., SU6656 and PP2 were individually added to the cells together with tet, and the development of the phenotype changes in the presence of these compounds was monitored subsequently in direct comparison with tet-induced cells that received no compound (Fig.14c-j). The compounds were also added to the cells without tet-induction of Src/ Src mutant expression as controls for non-specific/ toxicity effects. The experiments were performed with several ZM77 cell lines. As depicted in Fig.14, the tet-induced phenotype changes in ZM77.poly were partially suppressed by the various compounds: Cell scattering and conversion of the compact aggregate into loose and irregular clusters was inhibited to various extents, depending on the nature and concentration of the compounds.

In a small-scale screening campaign testing novel compounds with unknown activity against Src in whole cells, several molecules were identified, which significantly and specifically suppressed the observable phenotype changes. The (concentration-dependent) suppression effect of one compound, designated "D5", which has been identified in this screen with ZM77 cell lines, is shown in Fig.14k-n. These results demonstrate that the described assay is well suited to screen and identify (novel) compounds which inhibit Src activity in whole cells.

Specificity tests with PP1-Chr. and PP2 using the Src-TQ/YF expressing cell line ZM170.21 demonstrated that these compounds were largely ineffective in suppressing the tet-induced phenotype (cell scattering, etc.) (see Fig. 14/15 c, d). D5 slightly suppressed the phenotype changes of ZM170.21 (see Fig. 14/15 e, f). These results are consistent with the data obtained in the normal cell culture plates facilitating adherent growth (see above for details and conclusions).

In contrast to the Src-kinase inhibitors, which only suppressed the tet-induced phenotype in the Src-YF expressing cell lines, the Hsp90 inhibitors radicicol and 17-AAG were able to suppress to a large extent the tet-induced phenotype in both, Src-YF and Src-TQ/YF expressing cell lines (see Fig. 14/15 g, h, i, j). These results are in line with the data obtained in the normal cell culture plates facilitating adherent growth (see above for details and conclusions).

These results demonstrate that the described assay with suspended cells is well suited to screen and identify compounds, which modulate Src activity or the function of the Hsp90 chaperone system (including Hsp70, Hop and other co-involved proteins) in whole cells. This assay can distinguish between direct, ATP-competitive and Src (family)-specific inhibitors (category 2b in Tab.1) and other inhibitors with different binding modes/ specificity (categories 1 and 2a) by comparison of results from Src-YF with Src-TQ/YF expressing cells/cell lines.

### c) General considerations

Most of the mentioned cell lines have been subjected to a qualitative determination of their biological responses upon tet-mediated Src/ Src mutant expression induction.

The strength of the biological responses correlated with the respective inducible transgene expression levels. A big difference of the phenotypes -tet versus +tet, i.e. a strong biological response upon induction of expression, was desired, because the effect of a drug will suppress the tet-induced phenotype to a point somewhere in between these two extremes:
(i) no expression induction (-tet), phenotype reflects no (or background) Src/ Src mutant activity and
(ii) induced expression (+tet), phenotype reflects maximal Src/ Src mutant activity in the respective cell line.

A compound, which is added to the cells at the time of tet-induction, will diminish the development of the tet-inducible phenotype dependent on and quantitatively correlating with its inhibitory effect on Src/ Src mutant activity: If there is no inhibition (0%), the induced phenotype will be maximal (ii). On the other hand, if there is a complete inhibition (100%), the tet-induced phenotype is completely suppressed and the phenotype is unchanged, i.e. equivalent to the situation without tet (i). Accordingly, a partial inhibition of Src/ Src mutant activity by a compound will result in a partial suppression of the tet-inducible phenotype (1%-99%). The wider the range between the phenotypes of i) -tet and of ii) +tet (Δₚₜ₍₋ₜₑₜ→+ₜₑₜ) the more precisely can the effect of a potentially Src inhibitory compound be measured.

### Example 4 - Quantitative Determination of the Biological Readout (Phenotype) for the Expression of Src/Src Mutants and of the Potency of Src Modulating Chemical Compounds

### a) Examples: Proof-of-principle

To proof the concept of the assay platform according to the invention in a quantitative manner with a highly parallel readout, the following cell viability / metabolic activity assays from Promega according to the manufacturer's protocols: "CellTiter 96 AQueous One Solution Cell Proliferation Assay (MTS)", CellTiter-Blue™ Cell Viability Assay (CTB), and the ATP-status assay "CellTiter-Glo Luminescent Cell Viability Assay" (ATP) were used to obtain quantitative values reflecting (aspects of) the phenotype changes.

The cells were seeded into white-walled/ clear bottom 96-well plates ["MICROTEST" by Falcon, cat.no. 353947] for MTS and ATP assays and in black-walled/ clear bottom 96-well plates ["MICROTEST" by Falcon, cat.no. 353948] for the CTB assay at the appropriate densities. Tet and/or compound addition (where applicable) was started the next day. Measurements were taken 1,2 and 3 days later using the Wallac Victor²™ multilabel plate reader (Perkin Elmer). Absorbance was measured at 490nm (MTS). Luminescence mode was used for the ATP assay. For CTB fluorescence excitation wavelength was 550nm (filter: 550(20)nm), emission wavelength was 590 nm (filter: 590(20)nm).

Characteristic results of these assays are summarized in tables 2, 3 and 4:

Indicated are the means of sixplicate determinations (from 6 independent wells) in the absence of tet (mean(-tet)) and in the presence of tet (mean(+tet)), followed by the respective corresponding standard deviations SD(-tet) and SD(+tet). The cell viability/ metabolic activity upon tet-induction in relation to the cell viability/ metabolic activity without tet ("+/tet(means") is calculated from the quotient of the means of the measured cell viability/ metabolic activity signals from i) cells incubated without tet and ii) cells incubated with tet. A +/tet(means)-value of 100% indicates that cells are equally viable/ metabolically active with and without tet; wherever tet addition (i.e. induction of Src/ Src mutant expression) changes the phenotype towards reduced viability, the percentage decreases correspondingly. Furthermore indicated are the corresponding Z' values. In the present context: Z'=1-(3(SD(+tet)+SD(-tet))/ |mean(+tet)-mean(-tet)|. Z' is a simple statistical characteristic for assay quality assessment; its theoretical maximum value is 1 (no data variability; SD=0) (Zhang et al., 1999). Z'≥0 is required for an assay useful for a screening approach; a Z'≥0.5 is considered excellent for an assay with regard to high throughput screening. In Tab.s 2, 3 and 4 all +/-tet(means)-values, which are accompanied by Z' values ≥0, ≥0.5 and ≥0.8 are highlighted by yellow, light orange and dark orange background color, respectively.

Three more parameters reflecting the effects of compounds on cell viability/metabolic activity in these assays are indicated in Tab.3 and Tab.4: Toxicity reflects the nonspecific (i.e. irrespective of tet-induced Src/ Src mutant expression) effects of a compound on the viability/ metabolic activity of the cells. It is calculated as toxicity=1-mean(-tet, compound)/mean(-tet,(DMSO)). For simplicity reasons, the quotient "mean(-tet,compound)/mean(-tet,(DMSO)" is also called Qₜₒₓ hereafter. The vehicle itself (0.2% DMSO) had no toxic effect on any cell line. If the compound does not change cell viability/metabolic activity Qₜₒₓ=1 and thus toxicity=0. If a compound completely kills all cells with no remainder of cell viabil-ity/metabolic activity Qₜₒₓ=0 and toxicity=1. A negative toxicity value indicates that cell viability / metabolic activity is higher in the presence of a compound than without any compound. Negative toxicity values are usually small and due to a minor impreciseness of the measured values. The suppression value quantifies the potency of a compound with respect to suppression of the tet-induced change in cell viability / metabolic activity. Usually, tet-induction reduces cell viability / metabolic activity due to Src activity and addition of a Src inhibitory compound mitigates this reduction. A suppression of 100% means that the compound totally suppresses the tet-induced decrease in cell viability / metabolic activity (as in the absence of tet). The following formula is employed: Suppres-sion=(mean(+tet,compound)-mean(+tet,(DMSO))×Qₜₒₓ/(mean(-tet,compound)-mean(+tet,(DMSO))×Qₜₒₓ). Finally, the corresponding Z' value is calculated as follows: Z'(suppression)=1-

3(SD(+tet,compound)+SD(+tet,(DMSO))×Qₜₒₓ/|mean(+tet,compound)-mean(+tet,(DMSO))×Qₜₒₓ). The factor Qₜₒₓ corrects the means and SD determined in the absence of a compound for the (nonspecific) toxicity effect of the respective compound, which would otherwise overlay and obscure the specific suppression, if toxicity is not neglectable.

As a general result, the cell viability/metabolic activity of the ZM74.6 control cell line (con) is not changed by tet addition, because tet does not induce any Src or Src mutant expression in these cells: +/-tet(means)≈100%.

Table 2 shows that the high-level Src expressing cell line ZM75.7 (see above for further details) shows statistically relevant reductions in cell viability/metabolic activity in MTS and ATP assays. Especially in the ATP assay let-induction for 3 days reduced cell viability/metabolic activity to ~50% as compared with no tet-induction with very good corresponding Z' values of 0.75 and 0.79. The Src-YF expressing cell lines ZM77.2 and ZM77.8 were tested in parallel for their reduction of cell viability/ metabolic activity upon tet-induction. Here the values are even lower than with ZM75.7, declining to 10% cell viability/ metabolic activity after 3 days (compared with no tet-induction) in the ATP assay; the corresponding Z' value of 0.89 is excellent. Tet-induction for 1day reduced the cell viability/ metabolic activity to 55-65% (MTS) and to ~70% (ATP) and tet treatment for 2days reduced the cell viability/ metabolic activity to 20-40% (compared with no tet-induction). Compared with the rapid phenotype change that was obvious from microscopic evaluation by eye (see above), the reductions in cell viability/ metabolic activity measured in these assays are significantly delayed. As described in detail above, the larger the extent of the phenotype change upon tet addition Δₚₜ₍₋ₜₑₜ→+ₜₑₜ₎ the more easily and reliably a compound's capability to suppress the tet-induced phenotype change can be measured. In other words, the most suitable cell line for screening and measuring of Src inhibitors is the one with the most dramatic tet-inducible drop in cell viability/ metabolic activity (smallest +/-tet(means)-value). In that regard, it was found that the cell line ZM77.8 is the most well suited cell line corroborating the findings from the evaluations by eye (microscope).

The Src-KA expressing representative ZM76.3 shows only a moderate tet-induced reduction to roughly 65-70% of the cell viability/ metabolic activity without tet after 2-3 days in these assays (best Z' value in this experiment: 0.26), although the evaluation by eye reveals a very clear phenotype change 1-2 days after tet addition.
Lower cell densities do not usually improve effects and data quality.

Table 3 summarizes results from analogous assays, in which the cell lines ZM77.8(Src-YF), ZM75.7 (Src) and ZM76.3 (Src-KA) were used to evaluate the effects of test compounds PP1-Chr. and PP2, which are known Src kinase inhibitors, and of the newly identified compound "D5", which also appeared to suppress the tet-inducible phenotype in ZM77.8 (see above), with respect to their ability to suppress the tet-induced reduction in cell viability/ metabolic activity. ZM77.8 produced progressively lower +/-tet(means)-values declining to 5% (Z'=0.95) in the MTS and to 13% (Z'=0.91) in the ATP assay 3days after tet addition. These dramatic reductions and the corresponding excellent Z' values provide a very good basis for the measurement of the suppressive effects of compounds as can be seen in Tab.3. Interestingly, the best suppression values are already reached 2days after tet and compound addition, obviously due to the progressing degradation of the compounds in the medium and/or in the cells at 37°C over the course of several days. This finding is also in line with observations described above. For example, in the MTS assay +/-tet(means) was down to 16% after 2days, whereas in the presence of 10µM PP1-Chr. +/-tet(means) was only reduced to 75%, which makes a suppression of 70% accompanied by an excellent Z'(suppression) value of 0.86. After 3 days, the suppression declined to 28% with a Z'(suppression) still being excellent. In general it can be said that suppression effects by compounds are most obvious after 2 days, sometimes even 1day, of tet/compound incubation, while Z'(suppression) values are best after 2-3 days. Tab.3 also shows that the suppression of a compound (namely PP2) correlates with its concentration (here 5µM and 1µM), in line with the observation described above. The newly identified compound D5 at 40µM concentration mediated a 50% suppression (Z'=0.77) in the ATP assay 2days after tet/compound addition.

The suppression effects mediated by the test compounds PP1-Chr. and PP2 on ZM75.7 were detected with good to excellent Z'(suppression) values 3 days after tet/compound addition. The effect of D5 was not detected by this experiment

The compounds did not suppress the Src-KA induced phenotype of the cell line ZM76.3; this is in line with the observation described above. Rather, in the MTS assay, the presence of some compounds further reduces the cell viability/ metabolic activity (negative suppression), obviously due to a synergism with the dominant-negative effect. As already put forward above this indicates that these compounds exert their effects specifically via inhibition of Src's kinase activity rather than acting through any of the non-catalytic domains of Src or, indirectly, through inhibition of Src folding and/or expression.

The ATP-assay reagent was also added to the wells of a ultralow attachment plate containing ZM77.8 cells in suspension and transferred into the appropriate white-walled 96-well plates for luminescence measurement. Although the Z' values were not in the very good range, it can be seen that the cell viability is significantly decreased (as under adherent conditions) after 3 days of tet-induction and that addition of PP1-Chr. suppresses this reduction. Thus, in principle, a highly parallel and quantitative detection and determination of Src inhibitors also works with this assay variation.

Both assay types, MTS and ATP, are well suited to screen and detect in a quantitative and highly parallel manner compounds which are able to inhibit Src activity in intact cells. Furthermore, by using a series of appropriate concentrations the 50% effective concentration of a compound (EC50) can be determined. In summary, there is no big quality difference between MTS and ATP assay. Reductions of cell viability/metabolic activity are usually a bit more rapidly monitored with MTS (4h measurements are better than 1h measurements). The ATP assay delivers somewhat better Z' values in the majority of the cases; only with ZM76.3 the MTS assay is superior.

Table 4 shows data obtained from the CTB assay and in comparison, with the ATP assay. On each day 4 measurements are taken (1, 2, 3 and 4 h after the addition of the CTB assay reagents). The Src-TQ/YF expressing cell line ZM170.21, which allows to determine the specificity of compound inhibition (see above; Tab.1) was also included. Moreover the compounds radicicol and 17-AAG, which are no direct Src kinase inhibitors, but rather inhibit Hsp90, which is required for a proper folding of Src, and thus indirectly inhibit Src activity, were included.

The tet-inducible reductions of the cell viability/ metabolic activity (+/-tet(means)-values) are almost identical between ZM77.8 and ZM170.21 and go down to ~25% on day 1, to 8-9% on day 2 and to 2-3% on day 3 in the CTB assay (2, 3 or 4 h measurement time points), which provides an ideal basis for the measurement of suppression effects by compounds.

In this experiment, PP1-Chr+PP2 were used as a representative of the direct Src kinase inhibitory and Src-family-specific compounds. D5 was used as an example for a compound, which inhibits Src as a direct Src kinase inhibitor, but which is less specific and, according to the observations by eye/microscope (see above; Tab.1), can also to some extent inhibit the Src-TQ/YF mutant. Radicicol and 17-AAG were used as representatives of a compound class, which indirectly inhibits Src (via inhibition of the chaperone system).

In the Src-YF expressing cell line ZM77.8 all compounds clearly suppress the tet-induced reductions of the cell viability/ metabolic activity (+/-tet(means)-values). In the CTB assay the suppression is already evident and statistically reliable (good to excellent Z'(suppression) values) after 1day of incubation with tet and compounds (see Fig.15a). In the ATP assay the suppression is also evident on day1, the corresponding Z'(suppression) values are in the good to excellent range from day2 on.

In contrast, in the Src-TQ/YF expressing cell line ZM170.21 the Src family specific inhibitors PP1-Chr. and PP2 did not significantly suppress the +/-tet(means)-values (Fig.15b). This again demonstrates (in line with the observations described above), that this cell line is perfectly suited to discriminate between Src(family)-specific kinase inhibitors and non-specific or less specific inhibitors. This is further exemplified by the results with compound D5 (Fig.15b): In contrast to PP1-Chr. and PP2, this compound also significantly suppresses the +/-tet(means)-values in ZM170.21. The suppression is weaker than in ZM77.8 suggesting that D5, although not as specific as PP1-Chr. or PP2, has at least a preference for Src-family kinases.

Radicicol and 17-AAG were equally effective in both cell lines (Fig.15a,b).

Moreover, radicicol was also effectively suppressing the Src-KA-induced phenotype in the cell line ZM76.3 (CTB assay), although the Z'(suppression) values are too low for this cell line to be used in a medium or high-throughput screen, which require higher Z' values (Tab.4).

Evaluating the suitability of both assay formats (CTB and ATP) it can be stated that CTB shows better (more rapid and stronger) reductions upon let-addition in the relevant cell lines (ZM77.8, 77.2, 170.21, 76.3, 75.7) than the ATP assay (Tab.4 and data not shown). Due to this, at day2 and 3, the suppression (in %) in the CTB assay is often less than in the ATP assay, because the +/-tet(mean)-values are small (due to strong tet-induced reduction of cell viability / metabolic activity). The Z' values are more often better in the CTB than in the ATP assay. In CTB, later measurements (3-4h after reagent addition) are usually better and more reliable than early measurement time points (esp. 1h). Reductions (+/-tet(mean)-values) are usually best after 3 days both in ATP and CTB assays, while suppressions are often better at day2 (or even day1). Values with the control cell line ZM74.6 indicate that the CTB assay in general is probably less accurate than the ATP assay, which however, is roughly compensated for by the stronger reductions - at least this is shown by the comparable or slightly better Z' values. It follows that the CTB assay should preferably be given priority over the ATP assay in a first assay run, while an accompanying ATP assay gives more reliability and confidence. If the number of cell lines / compounds is small and the evaluation is very important both assays might be run in parallel.

The above described proof-of-principle experiments demonstrate that the three employed assay types (MTS, ATP and CTB) are very well suitable to screen, identify and quantitatively evaluate compounds, which can inhibit Src activity in intact cells directly and indirectly. So, this also includes the possibility to identify compounds which interfere with the Hsp90 chaperone system (including cooperative proteins, like Hsp70, Hop and others) as detailed above. It is furthermore possible to determine the specificity of such compounds by means of comparing their effects on Src-YF expressing (ZM77) cell lines with their effects on Src-TQ/YF expressing cell lines. Other mutations besides the TQ mutation described and used herein, which change important specificity determinants in or near the ATP binding site, and which consequently allow to discriminate between compounds specific for Src family kinases and compounds which are also able to tightly bind and inhibit other more distantly related tyrosine kinases, could be used in this assay.

If one compares the tet-inducible phenotype changes and their suppression by various compounds in ZM77 and ZM170 cell lines observed by eye (microscope), as detailed in example 3 or 4, respectively, with the measurements based on cell viability and metabolic activity by means of the above described assays, it is striking that the assays record the phenotype changes (and suppressions) with some delay: It takes 1-2 days (depending on the assay type) of tet(/compound) incubation until the changes (reductions) are evident and statistically significant. The reductions continue at least until day3. In contrast, observation of the cells under the microscope reveals that already 8-12h after addition of tet, i.e. induction of Src-YF or Src-TQ/YF expression, the dramatic phenotype change is (almost) fully established.

It is preferred to use image analysis systems for quantification of phenotype changes upon induction.

In a preferred embodiment it is suggested to use the assay platform in a two-stage process: 1. A Src-YF expressing cell line, e.g. ZM77.8, (or its analogue of other Src kinase family members) can be employed in a medium or high throughput screen using the very well suited CTB assay. Hits should preferably be confirmed by ATP and/or MTS assays. These assays are amenable to automation. 2. a) Verified hits can then be screened against a Src-TQ/YF expressing cell line, e.g. ZM170.21, (or its analogue of other Src kinase family members) using the same assays in order to evaluate them with respect to specificity. b) A Src-KA expressing cell line, e.g. ZM76.3, (or its analogue of other Src kinase family members) can then be included to further characterize the mode of action, especially for hits, which suppressed the phenotype changes of both Src-YF and -TQ/YF expressing cell lines. If Src-KA expressing cell lines do not yield statistically relevant data in these assays, they should preferably be evaluated by eye/ microscope. This will not be a major problem, because the number of hits will already be small after the initial filtering with Src-YF (and Src-TQ/YF) expressing cell lines. Although a qualitative difference of compound inhibition between Src-YF and wildtype Src was not expected, it is advisable to also check the verified hits on Src expressing cell lines (or other Src family member expressing cell lines). This may be done by the aforementioned assays in the case of the ZM75.7 cell line or by eye/ microscope.

### b) General Considerations and Methods

Displaying the most dramatic response, the Src-YF expressing ZM77 cell lines, e.g ZM77.8, seemed ideally suited to establish a method enabling a highly parallel and quantitative readout of these readily observable phenotype changes and their suppression by potential Srcinhibitory test compounds.

Also well suited is the Src expressing cell line ZM75.7. For reasons of classification of novel inhibitors according to their mode of action and specificity (see Tab.1, p.15) the Src-TQ/YF and Src-KA expressing cell lines, e.g. ZM170.21 and ZM76.3, respectively, are employed.

## Claims

1. Method of identifying, selecting and/or characterizing a compound which modulates the activity of at least one Src family kinase, comprising the steps of:
a. cultivating at least one cell (or at least one cell line) containing at least one nucleic acid coding for a Src family kinase or a mutated Src family kinase under suitable conditions,
b. expressing the nucleic acid(s) coding for a Src family kinase or a mutated Src family kinase in the cell(s) of step (a) under suitable conditions,
c. contacting the cell(s) of step (b) with at least one test compound and
d. determining whether the phenotype of the cell(s) of step (c) is changed as compared with the phenotype of the cell(s) of step (b), whereby a difference in the phenotype indicates that said test compound modulates the activity of at least one Src family kinase.

2. Method of claim 1, wherein the nucleic acid coding for Src family kinase or a Src family kinase mutant is inducibly expressed according to step (b).

3. Method of claim 1 or claim 2, wherein at least one cell (or at least one cell line) expresses a wildtype (wt) Src family kinase.

4. Method of any of claims 1 to 3, wherein at least one cell (or at least one cell line) expresses a Src kinase family member mutant, which displays a hyperactive form of a Src family kinase.

5. Method of claim 4, wherein at least one cell (or at least one cell line) expresses a Src kinase family member mutant, which contains at the regulatory tyrosine residue (phosphorylation site) a mutation, which does not allow the tyrosine residue to be phosphorylated, preferably by deletion, substitution or modification of the regulatory tyrosine residue.

6. Method of any of claims 1 to 5, wherein at least one cell (or at least one cell line) expresses a Src kinase family member mutant, which displays a "kinase dead" form of a Src family kinase.

7. Method of claim 6, wherein at least one cell (or at least one cell line) expresses a Src kinase family member mutant, which contains a mutation at the ATP binding site of a Src family kinase, which does not allow to bind ATP properly, preferably by deletion, substitution or modification of at least one residue of the ATP binding site.

8. Method of any of claims 1 to 7, wherein at least one cell (or at least one cell line) expresses a Src kinase family member mutant, which displays a "specificity determining" form of a Src family kinase.

9. Method of claim 8, wherein at least one cell (or at least one cell line) expresses a Src kinase family member mutant, which contains a mutation of at least one residue, which is characteristic for the Src kinase family, preferably at least one residue in the ATP binding site of a Src family kinase, which allows to identify test compounds which do not bind to the Src family mutant (in contrast to wt Src).

10. Method of any of claims 1 to 9, wherein at least one cell (or at least one cell line) expresses a Src kinase family member "double" mutant, which contains (i) at least one mutation leading to a hyperactivated form of a Src family kinase and (ii) at least one mutation at a specificity determining region.

11. Method of any of claims 1 to 10, wherein the mode of action (direct or indirect), the specificity, and/or the effectiveness of a test compound on the (activity of a) Src family kinase is/are determined.

12. Method of any of claims 1 to 11, wherein at least one, preferably two, three or four, nucleic acids coding for a mutated Src family kinase are selected from the group consisting of SEQ ID NOs 1 to 4 (Src kinase mutant sequences), SEQ ID NOs 5 to 8 (Yes kinase mutant sequences), SEQ ID NOs 9 to 12 (Fgr kinase mutant sequences), SEQ ID NOs 13 to 16 (Fyn kinase mutant sequences), SEQ ID NOs 17 to 20 (Lck kinase mutant sequences), SEQ ID NOs 21 to 24 (Hck kinase mutant sequences), SEQ ID NOs 25 to 28 (Lyn kinase mutant sequences), SEQ ID NOs 29 to 32 (Blk kinase mutant sequences), and SEQ ID NOs 33 to 36 (Yrk kinase mutant sequences).

13. Method of any of claims 1 to 12, wherein the method is based on four cells (or four cell lines), each of them containing one of the four nucleic acids, each of them expressing one of the mutant sequences of one Src family member according to claim 12, e.g. SEQ ID NOs 1 to 4 for Src.

14. Method of any of claims 1 to 13, wherein the cell or cell line is transfected with a vector, in particular an expression vector, containing the nucleic acid of a Src family kinase or a Src family kinase mutant.

15. Method of claim 14, wherein the vector is selected from the group consisting of pcDNA4/TO, PcDNA3-derivatives, and pcDNA5/TO.

16. Method of any of claims 1 to 15, wherein the expression of the nucleic acid in the transfected cell (or cell line) is induced by adding an inductor, preferably an antibiotic, e.g. tetracycline.

17. Method of any of claims 1 to 16, wherein the induced expression causes an overexpression of the nucleic acid in the transfected cell or cell line.

18. Method of any of claims 1 to 17, wherein the transfected cell is an eukaryotic cell, preferably a vertebrate cell, more preferably a mammalian cell, most preferably a human cell.

19. Method of claim 18, wherein the cell is an immortalised cell, a tumor cell line or a lymphoid cell.

20. Method of any of claims 1 to 19, wherein the phenotype change of the transfected cells upon induction and addition of a test compound is determined by qualitative and/or quantitative analysis, preferably by eye, microscope or image analysis.

21. Method of any of claim 20, wherein the phenotype change is determined by assessing whether the cellular phenotype of step (c) is changed as compared with the cellular phenotype of step (b), whereby a difference in the cellular phenotype indicates that said test compound modulates the activity of a Src family kinase.

22. Compound identified, selected and/or **characterized by** a method of any of claims 1 to 21.

23. Compound of claim 22 as a medicament, particularly for the treatment of diseases, which are at least in part caused by a Src family kinase.

24. Use of a compound of claims 22 or 23 for the production of a medicament for the treatment of diseases, which are at least in part caused by a Src family kinase, particularly by a dysfunction of a Src family kinase, in particular cancer, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, transplant rejection or allergic rhinitis.

25. Pharmaceutical composition containing a compound according to claims 22 or 23, and a pharmaceutically acceptable carrier, adjuvant or vehicle.

26. Pharmaceutical composition according to claim 25 for the preparation of a medicament for the treatment of cancer, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, transplant rejection or allergic rhinitis.

27. Use of a pharmaceutical composition of any of claims 25 or 26 for the production of a medicament for the treatment of diseases, which are at least in part caused by a Src family kinase, preferably cancer, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, transplant rejection or allergic rhinitis.
